# EUROPEAN PATENT SPECIFICATION

(11) **EP 2 287 300 B1**
(45) Date of publication and mention of the grant of the patent: **27.06.2018**
(21) Application number: 09738819.3
(22) Date of filing: 28.04.2009
(51) Int. Cl.: C12N 15/09, A01H 5/00, A61K 39/106, A61K 39/108, A61P 31/04, C07K 14/245, C07K 14/25, C07K 14/28, C07K 19/00, C12N 5/10, A61K 39/112

(54) **BACTERIAL TOXIN VACCINE**
BAKTERIENTOXINIMPFSTOFF
VACCIN DE TOXINE BACTÉRIENNE

(30) Priority: 02.05.2008 JP 2008120573
(43) Date of publication of application: 23.02.2011
(60) Divisional application: 14152631.9
(73) Proprietor: Idemitsu Kosan Co., Ltd., Chiyoda-ku, Tokyo 100-8321 (JP); National University Corporation Nara Institute of Science and Technology, Ikoma-shi, Nara 630-0192 (JP)
(72) Inventor: SAWADA, Kazutoshi, Sodegaura-shi Chiba 299-0293 (JP); YOSHIDA, Kazuya, deceased (JP); MATSUI, Takeshi, Sodegaura-shi Chiba 299-0293 (JP)
(74) Representative: Vossius & Partner Patentanwälte Rechtsanwälte mbB
(86) International application number: PCT/JP2009/058345
(87) International publication number: WO 2009/133882

(56) References cited:
- WO-A1-2005/011733
- WO-A2-01/89456
- GEORGE R A ET AL: "An analysis of protein domain linkers: their classification and role in protein folding", PROTEIN ENGINEERING, OXFORD UNIVERSITY PRESS, SURREY, GB, vol. 15, no. 11, 1 November 2002 (2002-11-01), pages 871-879, XP002374925, ISSN: 0269-2139, DOI: DOI:10.1093/PROTEIN/15.11.871
- RAN ET AL: "The immunogenicity of fusion protein linking the carboxyl terminus of the B subunit of Shiga toxin 2 to the B subunit of E. coli heat-labile enterotoxin", VETERINARY MICROBIOLOGY, ELSEVIER BV, NL, vol. 127, no. 1-2, 19 December 2007 (2007-12-19), pages 209-215, XP022393837, ISSN: 0378-1135, DOI: DOI:10.1016/J.VETMIC.2007.08.021
- GAO X ET AL: "Immunogenicity of a novel Stx2B-Stx1B fusion protein in a mice model of Enterohemorrhagic Escherichia coli O157:H7 infection", VACCINE, ELSEVIER LTD, GB, vol. 27, no. 14, 23 March 2009 (2009-03-23), pages 2070-2076, XP025969109, ISSN: 0264-410X, DOI: DOI:10.1016/J.VACCINE.2009.01.115 [retrieved on 2009-02-02]
- SATOH J ET AL: "The 5'-untranslated region of the tobacco alcohol dehydrogenase gene functions as an effective translational enhancer in plant", JOURNAL OF BIOSCIENCE AND BIOENGINEERING, ELSEVIER, AMSTERDAM, NL, vol. 98, no. 1, 1 January 2004 (2004-01-01), pages 1-8, XP004544191, ISSN: 1389-1723
- MASON HUGH S ET AL: "Edible plant vaccines: Applications for prophylactic and therapeutic molecular medicine", TRENDS IN MOLECULAR MEDICINE, vol. 8, no. 7, July 2002 (2002-07), pages 324-329, XP002634139, ISSN: 1471-4914
- TSUJI T ET AL: "A nasal vaccine comprising B-subunit derivative of Shiga toxin 2 for cross-protection against Shiga toxin types 1 and 2", VACCINE, ELSEVIER LTD, GB, vol. 26, no. 17, 16 April 2008 (2008-04-16), pages 2092-2099, XP022590859, ISSN: 0264-410X, DOI: DOI:10.1016/J.VACCINE.2008.02.034 [retrieved on 2008-03-07]
- WEN SHARON X ET AL: "A plant-based oral vaccine to protect against systemic intoxication by Shiga toxin type 2", PROCEEDINGS OF THE NATIONAL ACADEMY OF SCIENCES OF THE UNITED STATES OF AMERICA, vol. 103, no. 18, May 2006 (2006-05), pages 7082-7087, XP002635191, ISSN: 0027-8424
- MATSUI T ET AL.: "Production of double repeated B subunit of Shiga toxin 2e at high levels in transgenic lettuce plants as vaccine material for porcine edema disease.", TRANSGENIC RES., 21 October 2010 (2010-10-21), XP002634124, DOI: DOI: 10.1007/s11248-010-9455-9
- MATSUI TAKESHI ET AL: "Transgenic Lettuce Producing a Candidate Protein for Vaccine against Edema Disease", BIOSCIENCE BIOTECHNOLOGY AND BIOCHEMISTRY, vol. 73, no. 7, July 2009 (2009-07), pages 1628-1634, XP002635192, ISSN: 0916-8451
- KAZUTOSHI SAWADA ET AL.: 'Kumikae Lettuce ni yoru Kachikuyo Keiko Vaccine Tanpakushitsu Seisan no Kenkyu Kaihatsu' BIOTECHNOLOGY SYMPOSIUM 06 November 2007, pages 28 - 31, XP008128434
- KAZUTOSHI SAWADA ET AL.: 'Lettuce ni yoru Vaccine Seibun Seisan Gijutsu Kaihatsu (Sono 1) Kumikae Lettuce ni yoru Kachikuyo Keiko Vaccine Tanpakushitsu Seisan no Kenkyu Kaihatsu' PREPRINTS OF BIOTECHNOLOGY SYMPOSIUM 06 November 2007, pages 107 - 108, XP008128433
- TAKESHI MATSUI ET AL.: 'Buta Fushu Vaccine Tanpakushitsu Seisan no Tameno Lettuce Shoho Yuso Kogaku' ABSTRACT OF THE 25TH MEETING AND SYMPOSIUM IN CHIBA OF THE JAPANESE ASSOCIATION FOR PLANT TISSUE CULTURE 08 August 2007, page 180, XP008144517
- YASUDA, HIROSHI. ET AL.: 'The Correlation between Expression and Localization of a Foreign Gene Product in Rice Endosperm' PLANT AND CELL PHYSIOLOGY vol. 47, no. 6, 13 April 2006, pages 756 - 763, XP008112037
- O'DOWD A M ET AL: "Novel modifications to the C-terminus of LTB that facilitate site-directed chemical coupling of antigens and the development of LTB as a carrier for mucosal vaccines", VACCINE, ELSEVIER LTD, GB, vol. 17, no. 11-12, 1 March 1999 (1999-03-01), pages 1442-1453, XP004158272, ISSN: 0264-410X, DOI: 10.1016/S0264-410X(98)00375-2
- GUSTAVSSON MALIN ET AL: "Stable linker peptides for a cellulose-binding domain-lipase fusion protein expressed in Pichia pastoris", PROTEIN ENGINEERING, OXFORD UNIVERSITY PRESS, SURREY, GB, vol. 14, no. 9, 1 September 2001 (2001-09-01), pages 711-715, XP009118009, ISSN: 0269-2139
- JIN LIANG ET AL: "A Th1-recognized peptide P277, when tandemly repeated, enhances a Th2 immune response toward effective vaccines against autoimmune diabetes in nonobese diabetic mice", JOURNAL OF IMMUNOLOGY, AMERICAN ASSOCIATION OF IMMUNOLOGISTS, US, [Online] vol. 180, no. 1, 1 January 2008 (2008-01-01), pages 58-63, XP009155180, ISSN: 0022-1767 Retrieved from the Internet: URL:http://jimmunol.org/content/180/1/58.a bstract>
- OSCHERWITZ J ET AL: "A V3 loop haptenic peptide sequence, when tandemly repeated, enhances immunogenicity by facilitating helper T-cell responses to a covalently linked carrier protein", VACCINE, ELSEVIER LTD, GB, vol. 17, no. 19, 14 May 1999 (1999-05-14), pages 2392-2399, XP004168970, ISSN: 0264-410X, DOI: 10.1016/S0264-410X(99)00030-4

## Description

### Technical Field

The present invention relates to a hybrid protein used for vaccines for diseases caused by bacterial toxins such as Shiga toxins, cholera toxins, and *Escherichia coli* heat-labile toxins, and a DNA construct for producing the hybrid protein.

### Background Art

Shiga toxins (Stxs, verotoxins) are proteinous exotoxins produced by enterohemorrhagic *Escherichia coli* of pathogenic *Escherichia coli* species. The Shiga toxins cause hemorrhagic enteritis, hemolytic uremic syndrome, encephalopathy, and the like.

The Shiga toxins are broadly classified into Stx1 and Stx2, each of which is further classified into subclasses. An example of the Stx2 includes Stx2e which causes swine edema disease. The swine edema disease is known to frequently occur in baby pigs one to two weeks after weaning. A fatality due to infection with edema disease bacteria is 50 to 90%, which is extremely high.

Further, cholera toxins (CTs) are proteinous exotoxins produced by *Vibrio cholerae.* The CTs are known to cause severe diarrhea and emesis.

Still further, *Escherichia coli* heat-labile toxins (LTs) are proteinous endotoxins produced by enterotoxigenic *Escherichia coli.* The LTs are known to cause diarrhea and emesis.

The bacterial toxins, Stxs, LTs, and CTs are all known to include a B-subunit pentamer involved in adhesion to cells and an A-subunit monomer having a toxicity. The LTs and the CTs are also known to be similar structurally and functionally.

As a method of preventing the diseases caused by those bacterial toxins, methods of administering a vaccine by an injection or a nasal spray and administering the vaccine orally are known.

For example, a technology where an attenuated Stx2e protein is produced using recombinant *Escherichia coli* and administered to pigs by an injection is known (Non-patent Document 1). However, for example, an amount of the attenuated Stx2e protein produced by the recombinant *Escherichia coli* is not sufficient and the administration of the vaccine by an injection requires human labor. This has been a problem. Further, the method of administering the vaccine orally draws increasing attention in terms of reducing the labor in a stockbreeding field. In such a context, a technology where the bacterial toxin protein is produced by plants using a transgenic technology has been being developed. For example, a transgenic plant containing DNA encoding an LT protein B subunit (LTB) and expressing the DNA has been described (Patent Documents 1 and 2). A transgenic plant expressing DNA encoding the LT protein or the CT protein has been also described (Patent Document 3). However, there has been a problem that the amount of the produced protein is not sufficient in those technologies. An example of producing the LTB in *Lactuca sativa* has been reported (Non-patent Document 2). In this study, a gene of the LT protein B subunit including modified codons is expressed in *Lactuca sativa* using both a cauliflower mosaic virus 35S RNA promoter (CaMV35S) which is a promoter expressed highly in a plant and Kozak sequence which is an enhancer. As a result, it has been reported that the LT protein B subunit is accumulated in an amount of about 2.0% by mass of a total soluble protein of *Lactuca sativa.* However, this extent of the accumulated protein is thought to be insufficient to efficiently prevent a bacterial disease by utilizing the transgenic plant. That is, it is necessary to efficiently produce and accumulate the target bacterial toxin protein in plant cells. A hybrid bacterial toxin subunit is provided by WO 2005/011733. A further hybrid comprising the A-subunit of LT-A and the B-subunit of CT-B is provided by WO 2001/89456. The immunogenicity of a hybrid comprising the B-subunit of Shiga toxin 2 and the B-subunit of E. coli heat-labile enterotoxin has been studied by Ran (2007) Veterinary Microbiology 127, no.1-2, pages 209-215.

The inventors of the present invention found that the Stx2e protein could be produced efficiently in a plant such as *Lactuca sativa* and accumulated at a high concentration in a plant body by expressing the Stx2e protein where a secretory signal peptide derived from a plant had been added to its amino terminus, using a 5'-untranslated region (ADH 5'-UTR) of an alcohol dehydrogenase gene derived from a plant, and filed the patent (Patent Document 4).

[Patent Document 1] JP 10-507916 A
[Patent Document 2] JP 2000-166411 A
[Patent Document 3] JP 2002-533068 A
[Patent Document 4] WO 2009/004842 A1
[Non-patent Document 1] Makino et al., Microbial Pathogenesis, Volume 31, Number 1, July 2001, pp. 1-8(08)
[Non-patent Document 2] Kim et al., Protein Expression and Purification, Volume 51, Number 1, Jan 2006, pp. 22-27(06)

### Summary of Invention

It is an object of the present invention to more efficiently produce a Stx protein and other bacterial toxin proteins having a conformation similar thereto using plant cells.

Through production of a hybrid protein in which two or three bacterial toxin proteins such as Stx2e and CT are tandemly linked through a peptide having a particular sequence in plant cells, the inventors of the present invention have succeeded in accumulating the bacterial toxin protein at a high concentration in the plant cells and completed the present invention.

The present invention is as follows:
(1) A hybrid protein comprising
   (a) two toxin proteins selected from the group consisting of Shiga toxin (Stx) protein, cholera toxin (CT) protein or *Escherichia coli* heat-labile toxin (LT) protein, wherein said toxin proteins are tandemly linked through a peptide having the amino acid sequence represented by SEQ ID NO: 2, 82 or 84, and
   (b) a peptide having the amino acid sequence represented by SEQ ID NO: 2, 82 or 84 linked to the C-terminus of the tandemly linked toxin proteins of (a),
   wherein the Stx proteins is a STx2eB protein, wherein the CT protein is a CT protein B subunit, and wherein the LT protein is a LT protein B subunit.
(2) The hybrid protein according to item 1, wherein said toxin proteins are tandemly linked through a peptide having the amino acid sequence represented by SEQ ID NO: 2.
(3) The hybrid protein according to item 1 or 2, comprising an amino acid sequence represented by SEQ ID NO: 14 or 16.
(4) The hybrid protein according to item 1, comprising an amino acid sequence represented by SEQ ID NO: 90, 92, 98, or 100.
(5) The hybrid protein according to any one of items 1 to 4, wherein a secretory signal peptide derived from a plant is added to is amino terminus.
(6) The hybrid protein according to anyone of items 1 to 5, wherein an endoplasmic reticulum retention signal peptide is added to its carboxyl terminus.
(7) A DNA construct comprising DNA encoding the hybrid protein according to any one of items 1 to 6.
(8) The DNA construct according to item 7, comprising DNA having a base sequence represented by SEQ ID NO: 13, 15, 89, 91, 97, or 99.
(9) The DNA construct according to item 7 or 8, wherein DNA encoding a hybrid protein is operably-linked to a 5'-untranslated region of an alcohol dehydrogenase gene derived from a plant.
(10) The DNA construct according to item 9, wherein the 5'-untranslated region of an alcohol dehydrogenase gene derived from a plant is derived from *Nicotiana tabacum.*
(11) The DNA construct according to item 10, comprising a base sequence represented by any one of SEQ ID NOS: 28, 29, 101, 102, 103, 104, 108, 109, 110, or 111.
(12) A recombinant vector comprising the DNA construct according to any one of items 7 to 11.
(13) A transformant transformed with the recombinant vector according to item 12.
(14) The transformant according to item 13, wherein the transformant is a transformed plant cell or a transformed plant.
(15) A seed, which is obtained from the transformant according to item 13 or 14.
(16) A peptide having an amino acid sequence represented by one of SEQ ID NO: 2, 82, or 84.

The disclosure relates to:
(1) a hybrid protein, in which two or three of Shiga toxin proteins, cholera toxin proteins, or *Escherichia coli* heat-labile toxin proteins are each tandemly linked through a peptide having the following characteristics (A) and (B) :
   (A) the number of amino acids is 12 to 30; and
   (B) the content of proline is 20 to 35%;
(2) the hybrid protein according to Item (1), in which the peptide further has the following characteristic (C):
   (C) proline is allocated every two or three amino acids;
(3) the hybrid protein according to Item (2), in which the peptide has an amino acid sequence represented by SEQ ID NO: 2, 82, or 84;
(4) the hybrid protein according to Item (3), in which two of the Shiga toxin proteins, cholera toxin proteins, or *Escherichia coli* heat-labile toxin proteins are tandemly linked through the peptide having the amino acid sequence represented by SEQ ID NO: 2;
(5) the hybrid protein according to any one of Items (1) to (4), in which the Shiga toxin proteins are Shiga toxin protein B subunits;
(6) the hybrid protein according to any one of Items (1) to (5), in which the Shiga toxin proteins are Stx2e proteins;
(7) the hybrid protein according to any one of Items (1) to (4), in which the cholera toxin proteins are cholera toxin protein B subunits;
(8) the hybrid protein according to Item (4), including an amino acid sequence represented by SEQ ID NO: 10, 12, 14, or 16;
(9) the hybrid protein according to Item (3), including an amino acid sequence represented by SEQ ID NO: 86, 88, 90, 92, 94, 96, 98, or 100;
(10) the hybrid protein according to any one of Items (1) to (9), in which a secretory signal peptide derived from a plant is added to its amino terminus;
(11) the hybrid protein according to Item (10), in which an endoplasmic reticulum retention signal peptide is added to its carboxyl terminus;
(12) the hybrid protein according to any one of Items (1) to (9), in which a chloroplast transit signal peptide is added to its amino terminus;
(13) a DNA construct, including DNA encoding the hybrid protein according to any one of Items (1) to (12);
(14) the DNA construct according to Item (13), including DNA having a base sequence represented by SEQ ID NO: 9, 11, 13, or 15;
(15) the DNA construct according to Item (13), including DNA having a base sequence represented by SEQ ID NO: 85, 87, 89, 91, 93, 95, 97, or 99;
(16) the DNA construct according to any one of Items (13) to (15), in which DNA encoding a hybrid protein is operably-linked to a 5'-untranslated region of an alcohol dehydrogenase gene derived from a plant;
(17) the DNA construct according to Item (16), in which the 5'-untranslated region of the alcohol dehydrogenase gene derived from the plant is derived from *Nicotiana tabacum*;
(18) a DNA construct according to Item (17), including a base sequence represented by any one of SEQ ID NOS: 24 to 29;
(19) a DNA construct according to Item (17), including a base sequence represented by any one of SEQ ID NOS: 101 to 111;
(20) a recombinant vector, including the DNA construct according to any one of Items (13) to (19);
(21) a transformant transformed with the recombinant vector according to Item (20);
(22) a transformant according to Item (21), in which the transformant is a transformed plant cell or a transformed plant;
(23) a seed, which is obtained from the transformant according to Item (21) or (22); and
(24) a peptide, having an amino acid sequence represented by SEQ ID NO: 2, 82, or 84.

### Brief Description of Drawings

In the accompanying drawings:
FIG. 1 is a view illustrating a design of Stx2eB expression vectors, in which an arrow denotes a translation initiation site, and an inverted triangle denotes a site to be cleaved after the translation;
FIG. 2 is a photograph illustrating levels of accumulated Stx2eB obtained in a transient expression experiment using *Lactuca sativa* protoplasts;
FIG. 3 is a photograph illustrating levels of accumulated CTB obtained in a transient expression experiment using the *Lactuca sativa* protoplasts;
FIG. 4 is a view illustrating a design of DNA constructs encoding an Stx2eB-YFP fusion protein, in which an arrow denotes a translation initiation site, and an inverted triangle denotes a site to be cleaved after the translation;
FIG. 5 is a photograph illustrating localization of the Stx2eB-YFP fusion protein obtained in a transient expression experiment using cultured tobacco cell protoplasts;
FIG. 6 is a photograph illustrating localization of the Stx2eB-YFP fusion protein obtained in a transient expression experiment using the cultured tobacco cell protoplasts;
FIG. 7 is a photograph illustrating localization of vacuole-type GFP and the Stx2eB-YFP fusion protein in co-expression of ARF1pWT and ARF1pDN obtained in a transient expression experiment using the cultured tobacco cell protoplasts;
FIG. 8 is a photograph illustrating the levels of accumulated Stx2eB obtained in a transformation experiment using cultured tobacco cells (BY2), in which a numeral in each lane denotes a clone number;
FIG. 9 is a photograph illustrating the levels of accumulated Stx2eB obtained in a transformation experiment using the cultured tobacco cells (BY2), in which the numeral in each lane denotes the clone number;
FIG. 10 is photographs illustrating the levels of accumulated Stx2eB obtained in a transformation experiment using the cultured Tobacco cells (BY2), in which the numeral in each lane denotes the clone number;
FIG. 11 is a graph illustrating the levels of accumulated Stx2eB obtained in a transformation experiment using the cultured tobacco cells (BY2), in which each numeral denotes the clone number;
FIG. 12 is a graph illustrating a relationship between mRNA levels of Stx2eB and the levels of accumulated Stx2eB;
FIG. 13 is a view illustrating the design of DNA constructs encoding Stx2eB, in which A, B, and C denote the designs of an endoplasmic reticulum type, a cytoplasm type, and a chloroplast type of the DNA constructs, respectively;
FIG. 14 is a view illustrating the design of DNA constructs encoding CTB, in which A, B, and C denote the designs of an endoplasmic reticulum type, a cytoplasm type and a chloroplast type of the DNA constructs, respectively;
FIG. 15 is a photograph illustrating the levels of accumulated Stx2eB obtained in a transient expression experiment using the *Lactuca sativa* protoplasts;
FIG. 16 is a photograph illustrating the levels of accumulated CTB obtained in a transient expression experiment using the *Lactuca sativa* protoplasts;
FIG. 17 is photographs illustrating the levels of accumulated Stx2eB obtained in a transformation experiment using the cultured tobacco cells (BY2), in which the numeral in each lane denotes the clone number;
FIG. 18 is photographs illustrating the levels of accumulated Stx2eB obtained in a transformation experiment using a tobacco plant body, in which the numeral in each lane denotes the clone number;
FIG. 19 is a photograph illustrating the levels of accumulated Stx2eB obtained in a transformation experiment using the tobacco plant body, in which the numeral in each lane denotes the clone number;
FIG. 20 is a view illustrating the design of DNA constructs encoding Stx2eB;
FIG. 21 is a view illustrating the design of DNA constructs encoding CTB;
FIG. 22 is photographs illustrating the levels of accumulated Stx2eB obtained in a transformation experiment using the cultured tobacco cells (BY2); and
FIG. 23 is a photograph illustrating the levels of accumulated CTB obtained in a transformation experiment using the cultured tobacco cells (BY2).

### Description of Embodiments

In a hybrid protein of the present invention, two or three of Shiga toxin (Stx) proteins, cholera toxin (CT) proteins, or *Escherichia coli* heat-labile toxin (LT) proteins are each tandemly linked through a peptide having the following characteristics (A) and (B):
(A) the number of amino acids is 12 to 30; and
(B) the content of proline is 20 to 35%.

Shiga toxins (Stxs) are classified into type 1 (Stx1) and type 2 (Stx2). The Stx1 is further classified into subclasses a to d, and the Stx2 is further classified into subclasses a to g, respectively. The Shiga toxin includes one A subunit which is a toxin main body and five B subunits involved in invasion into intestinal mucosa.

Of those, for example, Stx2e is known as a swine edema disease toxin, and its A subunit (Stx2eA) is represented by an amino acid sequence of SEQ ID NO: 4 and its B subunit (Stx2eB) is represented by an amino acid sequence of SEQ ID NO: 6.

In Stx2eA and Stx2eB, one or several amino acids may be substituted, deleted, inserted, or added in the amino acid sequences represented by SEQ ID NO: 4 or 6 as long as they can cause an immune response by administering to pigs. For example, the term "several" means the number of preferably 2 to 30, more preferably 2 to 20, and still more preferably 2 to 10, in Stx2eA, and means the number of preferably 2 to 10, more preferably 2 to 5, and still more preferably 2 to 3, in Stx2eB.

Further, Stx2eA and Stx2eB may be those having an identity of preferably 85% or more, more preferably 90% or more, and still more preferably 95% or more to the amino acid sequences represented by SEQ ID NOS: 4 and 6, and being capable of causing the immune response by administering to the pig.

The cholera toxin (CT) includes one A subunit (CTA) which is the toxin main body and five B subunits (CTB) represented by SEQ ID NO: 8 and involved in the invasion into intestinal mucosa.

In CTB, one or several amino acids may be substituted, deleted, inserted, or added in the amino acid sequence represented by SEQ ID NO: 8 as long as CTB can cause the immune response by administering to animals. The term "several" means preferably 2 to 10, more preferably 2 to 5, and still more preferably 2 to 3.

Further, CTB may be those having an identity of preferably 85% or more, more preferably 90% or more, and still more preferably 95% or more to the amino acid sequences represented by SEQ ID NO: 8, and being capable of causing the immune response by administering to the animals.

The *Escherichia coli* heat-labile toxin (LT) protein includes one A subunit which is the toxin main body and five B subunits involved in the invasion into intestinal mucosa.

The Shiga toxin, the cholera toxin, and the *Escherichia coli* heat-labile toxin are also collectively referred to as "bacterial toxins" herein.

The number of the amino acids in the peptide is preferably 12 to 25 and more preferably 12 to 22. The content of proline in the peptide is preferably 20 to 27% and more preferably 20 to 25%.

Further, proline is allocated preferably every two or three residues in the peptide. But, in this case, the amino acids other than proline may be consecutive within 5 residues and preferably 4 residues in the terminus of the peptide.

In addition, the total content of serine, glycine, arginine, lysine, threonine, glutamine, asparagine, histidine, and aspartic acid in the amino acids other than proline is preferably 70% or more, more preferably 80% or more, and still more preferably 90% or more in the peptide. Further, the total content of serine, glycine, and asparagine in the amino acids other than proline is preferably 70% or more, more preferably 80% or more, and still more preferably 90% or more in the peptide. Still further, the total content of serine and glycine in the amino acids other than proline is preferably 70% or more, more preferably 80% or more, and still more preferably 90% or more in the peptide. This is because the peptide containing those amino acids abundantly is hard to form a secondary structure (β-sheet structure and helix structure).

Meanwhile, the total content of alanine, methionine, and glutamic acid in the amino acids other than proline is preferably 30% or less, more preferably 20% or less, and still more preferably 10% or less in the peptide. This is because the peptide containing those amino acids abundantly easily forms the helix structure. The total content of tryptophan, leucine, isoleucine, tyrosine, phenylalanine, and valine in the amino acids other than proline is preferably 20% or less, more preferably 10% or less, and still more preferably 5% or less in the peptide. This is because the peptide containing those amino acids abundantly easily forms the β-sheet structure and the helix structure.

The peptide is preferably selected from the peptide (PG12) having the amino acid sequence represented by SEQ ID NO: 2, the peptide (PG17) having the amino acid sequence represented by SEQ ID NO: 82, and the peptide (PG22) having the amino acid sequence represented by SEQ ID NO: 84.

In the hybrid protein of the present invention, two or three hybrid proteins of the A subunit and the B subunit may be tandemly linked through the above peptide, or two or three A subunits may be tandemly linked through the peptide, or two or three B subunits may be tandemly linked through the peptide. When the hybrid protein contains the A subunit, the A subunit is preferably attenuated. In the hybrid protein of the present invention, preferably two or three B subunits are tandemly linked through the peptide. In the hybrid protein of the present invention, preferably two B subunits are tandemly linked through PG12.

Further, in the hybrid protein of the present invention, the peptide is preferably added to its C terminus. PG12 is particularly preferably added to its C terminus in the hybrid protein of the present invention.

The hybrid protein of the present invention has the amino acid sequence represented by SEQ ID NO: 10, 12, 14, 16, 86, 88, 90, 92, 94, 96, 98, or 100, for example. In the hybrid protein having the amino acid sequence represented by SEQ ID NO: 10, two Stx2eBs are tandemly linked through PG12. In the hybrid protein having the amino acid sequence represented by SEQ ID NO: 12, two CTBs are tandemly linked through PG12. In the hybrid protein having the amino acid sequence represented by SEQ ID NO: 14, two Stx2eBs are tandemly linked through PG12 and further PG12 is linked to the C terminus thereof. In the hybrid protein having the amino acid sequence represented by SEQ ID NO: 16, two CTBs are tandemly linked through PG12 and further PG12 is linked to the C terminus thereof. In the hybrid protein having the amino acid sequence represented by SEQ ID NO: 86, three Stx2eBs are each tandemly linked through PG12. In the hybrid protein having the amino acid sequence represented by SEQ ID NO: 88, three Stx2eBs are each tandemly linked through PG12 and further PG12 is linked to the C terminus thereof. In the hybrid protein having the amino acid sequence represented by SEQ ID NO: 90, two Stx2eBs are tandemly linked through PG17 and further PG12 is linked to the C terminus thereof. In the hybrid protein having the amino acid sequence represented by SEQ ID NO: 92, two Stx2eBs are tandemly linked through PG22 and further PG12 is linked to the C terminus thereof. In the hybrid protein having the amino acid sequence represented by SEQ ID NO: 94, three CTBs are each tandemly linked through PG12. In the hybrid protein having the amino acid sequence represented by SEQ ID NO: 96, three CTBs are each tandemly linked through PG12 and further PG12 is linked to the C terminus thereof. In the hybrid protein having the amino acid sequence represented by SEQ ID NO: 98, two CTBs are tandemly linked through PG17 and further PG12 is linked to the C terminus thereof. In the hybrid protein having the amino acid sequence represented by SEQ ID NO: 100, two CTBs are tandemly linked through PG22 and further PG12 is linked to the C terminus thereof.

By using the peptide such as PG12, PG17, or PG22 as a linker for linking the bacterial toxin proteins, the level of the bacterial toxin protein accumulated in the plant cell is increased.

In the hybrid protein of the present invention, a secretory signal peptide derived from a plant or a chloroplast transit signal peptide is preferably added to its amino terminus. Here, the term "addition" is a concept including both the case where the secretory signal peptide is directly bound to the amino terminus of the two or three bacterial toxin proteins linked through the peptide and the case where the secretory signal peptide is bound thereto through another peptide.

The secretory signal peptide is derived from preferably a plant belonging to the family *Solanaceae, Brassicaceae,* or *Asteraceae,* further preferably a plant belonging to the genus *Nicotiana, Arabidopsis, Lactuca,* etc., and more preferably *Nicotiana tabacum* or *Arabidopsis thaliana, Lactuca sativa,* etc.

Moreover, it is preferably derived from a β-D-glucan exohydrolase of *Nicotiana tabacum* or a 38-kDa peroxidase of *Nicotiana tabacum* (GenBank ACCESSION D42064).

An example of the secretory signal peptide includes a peptide that is derived from a β-D-glucan exohydrolase of *Nicotiana tabacum* and has the amino acid sequence represented by SEQ ID NO: 18.

An example of the chloroplast transit signal peptide includes a chloroplast transit signal peptide (transit peptide, T.P., SEQ ID NO: 79) derived from *Lactuca sativa* Rbcs (Rubisco small subunit) (GenBank ACCESSION D14001). A base sequence of DNA which encodes the chloroplast transit signal peptide derived from *Lactuca sativa* Rbcs is represented by SEQ ID NO: 80. Herein, the hybrid protein including the chloroplast transit signal peptide added to its amino terminus is also referred to as a chloroplast-type (Chl) hybrid protein, and a DNA construct encoding the chloroplast-type (Chl) hybrid protein is also referred to as a chloroplast-type DNA construct. The chloroplast-type hybrid protein is efficiently accumulated particularly in a plant whose chloroplast is developed well such as *Nicotiana tabacum.*

The hybrid protein in which neither the secretory signal peptide nor the chloroplast transit signal protein is added to its amino terminus is referred to as a cytoplasm-type (Cyt) hybrid protein, and the DNA construct encoding the cytoplasm-type hybrid protein is referred to as a cytoplasmic-type DNA construct. In the cytoplasm-type hybrid protein, particularly preferably three bacterial toxin protein B subunits are tandemly linked through the peptide.

In the hybrid protein of the present invention, the signal peptide such as an endoplasmic reticulum retention signal peptide and a vacuolar transport signal peptide may be added to its carboxyl terminus. Here, the term "addition" is the concept including both the case where the signal peptide is directly bound to the carboxyl terminus of the hybrid protein and the case where the signal peptide is bound thereto through another peptide. Herein, the hybrid protein in which the secretory signal peptide is added to its amino terminus and the endoplasmic reticulum retention signal peptide is added to the carboxyl terminus is also referred to as an endoplasmic reticulum-type (ER) hybrid protein, and the DNA construct encoding the endoplasmic reticulum-type hybrid protein is also referred to as an endoplasmic reticulum-type DNA construct. The endoplasmic reticulum-type hybrid protein is efficiently accumulated particularly in a plant such as *Lactuca sativa.*

In the hybrid protein of the present invention, the endoplasmic reticulum retention signal peptide is preferably added to its carboxyl terminus. Examples of the endoplasmic reticulum retention signal peptide include an endoplasmic reticulum retention signal peptide including KDEL sequence (SEQ ID NO: 19), HDEL sequence (SEQ ID NO: 20), KDEF sequence (SEQ ID NO: 21), or HDEF sequence (SEQ ID NO: 22).

The vacuolar transport signal peptide is derived from preferably a plant belonging to the family *Solanaceae, Brassicaceae,* or *Asteraceae,* further preferably a plant belonging to the genus *Nicotiana, Arabidopsis, Armoracia,* etc., and more preferably *Nicotiana tabacum, Arabidopsis thaliana, Armoracia rusticana,* etc. In addition, the peptide is preferably derived from a chitinase. The amino acid sequence of a vacuolar transport signal peptide derived from a tobacco chitinase is represented by SEQ ID NO: 76. Meanwhile, the base sequence of DNA encoding a vacuolar transport signal peptide derived from a tobacco chitinase is represented by SEQ ID NO: 75, for example.

Moreover, the peptide is preferably derived from a horseradish peroxidase C1a isozyme. The amino acid sequence of a vacuolar transport signal peptide derived from a horseradish peroxidase C1a isozyme is represented by SEQ ID NO: 78. Meanwhile, the base sequence of DNA encoding a vacuolar transport signal peptide derived from a horseradish peroxidase C1a isozyme is represented by SEQ ID NO: 77, for example. Herein, the hybrid protein in which the secretory signal peptide is added to its amino terminus, and the vacuolar transport signal peptide is added to its carboxyl terminus is also referred to as a vacuole-type (Vac) hybrid protein, and a DNA construct encoding the vacuole-type hybrid protein is also referred to as a vacuole-type DNA construct.

The hybrid protein of the present invention may be synthesized chemically, or may be produced by genetic engineering. A method of producing by the genetic engineering is described later.

The DNA construct of the present invention is characterized by containing DNA encoding the hybrid protein of the present invention.

That is, the DNA construct of the present invention includes DNA in which DNAs encoding the two or three bacterial toxin proteins are tandemly linked through DNA encoding the peptide. The DNA encoding the peptide is represented by, for example, SEQ ID NO: 1 (PG12), SEQ ID NO: 81 (PG17), and SEQ ID NO: 83 (PG22). Examples of the DNA encoding the bacterial toxin protein include DNA (SEQ ID NO: 3) encoding Stx2eA, DNA (SEQ ID NO: 5) encoding Stx2eB, and DNA (SEQ ID NO: 7) encoding CTB. The DNA encoding the peptide and the DNA encoding the bacterial toxin protein are linked by matching their reading frames except stop codons.

The DNA encoding the bacterial toxin protein can be obtained by a common genetic engineering technique based on the base sequence of SEQ ID NO: 3, 5, or 7, for example. Specifically, a cDNA library is prepared from a bacterium which produces each bacterial toxin according to a conventional method, and a desired clone is selected using a probe prepared from the library based on the base sequence. Alternatively, the DNA can also be synthesized chemically based on the base sequence, or synthesized by PCR with genomic DNA as a template using a 5'- and 3'- terminal base sequence of the base sequence as primers, for example.

The DNA encoding the hybrid protein of the present invention is represented by SEQ ID NO: 9, 11, 13, 15, 85, 87, 89, 91, 93, 95, 97, or 99.

In the DNA encoding the hybrid protein, preferably a codon corresponding to an amino acid which composes the hybrid protein is appropriately modified so that the amount of the translated hybrid protein is increased depending on a host cell in which the hybrid protein is produced.

As the method of modifying the codon, a method of Kang et al. (2004) may serve as a reference, for example. And, the method of selecting the codon frequently used in the host cell, the method of selecting the codon in which the content of GC is high, or the method of selecting the codon frequently used in house keeping genes in the host cell is exemplified.

Further, the DNA encoding the hybrid protein may be DNA which hybridizes with DNA having the base sequence of SEQ ID NO: 9, 11, 13, 15, 85, 87, 89, 91, 93, 95, 97, or 99 under a stringent condition. The term "stringent condition" refers to the condition where a so-called specific hybrid is formed whereas no non-specific hybrid is formed. There is exemplified the condition where two DNAs with high identity, e.g., two DNAs having the identity of preferably 80% or more, more preferably 90% or more, and particularly preferably 95% or more are hybridized with each other whereas two DNAs with lower identity than that are not hybridized. Further, there is exemplified the condition of 2×SSC (330 mM NaCl, 30 mM citric acid) at 42°C and preferably 0.1×SSC (330 mM NaCl, 30 mM citric acid) at 60°C.

In the DNA construct of the present invention, preferably the DNA encoding the hybrid protein is operably-linked to an enhancer. The term "operably" refers to the fact that the hybrid protein is produced in host cells when a vector obtained by inserting the DNA construct of the present invention into a vector including a suitable promoter is introduced into suitable host cells. In addition, the term "linked" refers to both a case where two DNAs are directly linked and a case where two DNAs are linked via another base sequence.

Examples of the enhancer include Kozak sequence and a 5'-untranslated region of an alcohol dehydrogenase gene derived from a plant. Particularly preferably, the DNA encoding the hybrid protein is operably-linked to the 5'-untranslated region of an alcohol dehydrogenase gene derived from a plant.

The 5'-untranslated region of an alcohol dehydrogenase gene refers to a region including a sequence between the base at the transcription initiation site of a gene encoding an alcohol dehydrogenase and the base before the translation initiation site (ATG, methionine). The region has a function to increase a translation level. The phrase "function to increase a translation level" refers to a function to increase an amount of a protein produced by translation when the information encoded in a structural gene is transcribed and then translated to produce a protein. The region may be derived from a plant, and it is preferably derived from a plant belonging to the family *Solanaceae, Brassicaceae,* or *Asteraceae,* further preferably derived from a plant belonging to the genus *Nicotiana, Arabidopsis, Lactuca,* etc., and more preferably derived from *Nicotiana tabacum, Arabidopsis thaliana, Lactuca sativa,* etc.

The 5'-untranslated region of an alcohol dehydrogenase gene is particularly preferably a region including the base sequence represented by SEQ ID NO: 23, which is the 5'-untranslated region of an alcohol dehydrogenase gene (NtADH 5'-UTR) derived from *Nicotiana tabacum,* for example.

The 5'-untranslated region of an alcohol dehydrogenase gene derived from a plant can be isolated from an alcohol dehydrogenase gene of a plant cultured cell where an alcohol dehydrogenase is highly expressed, for example (see JP 2003-79372 A). Meanwhile, in the case of a region having a determined base sequence, such as the 5'-untranslated region of an alcohol dehydrogenase gene derived from *Nicotiana tabacum,* the region can also be synthesized by chemical synthesis, PCR using a genomic DNA as a template and using the base sequences of the 5'- and 3'-termini of the region as primers, or the like. In addition, if a part of the region having a determined base sequence is used as a probe, the 5'-untranslated region of an alcohol dehydrogenase gene derived from another plant can be searched and isolated.

The 5'-untranslated region of an alcohol dehydrogenase gene represented by the base sequence of SEQ ID NO: 23 may have substitution, deletion, insertion, or addition of one or several bases as long as the region has a function to increase a translation level. The term "several" means the number of preferably 2 to 10, further preferably 2 to 5, and particularly preferably 2 to 3.

In addition, DNA having an identity of preferably 85% or more and particularly preferably 90% or more to the 5'-untranslated region of an alcohol dehydrogenase gene and having an ability to increase a translation level may be used.

Whether the region has an intended function to increase a translation level or not can be confirmed by, for example, a transient assay using a GUS (β-glucuronidase) gene or a luciferase gene as a reporter gene in tobacco cultured cells, or an assay in transformed cells engineered to carry those genes in a chromosome.

The DNA construct of the present invention has the base sequence represented by any one of SEQ ID NOS: 24 to 29 and SEQ ID NOS: 101 to 111, for example.

The DNA construct having the base sequence represented by SEQ ID NO: 24 is the DNA construct in which DNA (SEQ ID NO: 9) encoding the hybrid protein in which two Stx2eB proteins are tandemly linked through PG12 is linked to the 5'-untranslated region (NtADH 5'-UTR, SEQ ID NO: 23) of an alcohol dehydrogenase gene derived from *Nicotiana tabacum.* Further, the DNA construct having the base sequence represented by SEQ ID NO: 25 is the DNA construct in which DNA (SEQ ID NO: 11) encoding the hybrid protein in which two CTB proteins are tandemly linked through PG12 is linked to NtADH 5'-UTR.

The DNA construct having the base sequence represented by SEQ ID NO: 26 is the DNA construct in which DNA encoding the hybrid protein in which two Stx2eB proteins are tandemly linked through PG12, the secretory signal peptide is added to its amino terminus, and the endoplasmic reticulum retention signal peptide is added to its carboxyl terminus is linked to NtADH 5'-UTR. Further, the DNA construct having the base sequence represented by SEQ ID NO: 27 is the DNA construct in which DNA encoding the hybrid protein in which two CTB proteins are tandemly linked through PG12, the secretory signal peptide is added to its amino terminus, and the endoplasmic reticulum retention signal peptide is added to its carboxyl terminus is linked to NtADH 5'-UTR.

The DNA construct having the base sequence represented by SEQ ID NO: 28 is the DNA construct in which DNA encoding the hybrid protein in which two Stx2eB proteins are tandemly linked through PG12, the secretory signal peptide is added to its amino terminus, PG12 is linked to its carboxyl terminus, and further the endoplasmic reticulum retention signal peptide is added to its carboxyl terminus is linked to NtADH 5'-UTR. Further, the DNA construct having the base sequence represented by SEQ ID NO: 29 is the DNA construct in which DNA encoding the hybrid protein in which two CTB proteins are tandemly linked through PG12, the secretory signal peptide is added to its amino terminus, PG12 is linked to its carboxyl terminus, and further the endoplasmic reticulum retention signal peptide is added to its carboxyl terminus is linked to NtADH 5'-UTR.

The DNA construct having the base sequence represented by SEQ ID NO: 101 is the DNA construct in which DNA encoding the hybrid protein in which two Stx2eB proteins are tandemly linked through PG12 and PG12 is linked to its carboxyl terminus is linked to NtADH 5'-UTR.

The DNA construct having the base sequence represented by SEQ ID NO: 102 is the DNA construct in which DNA encoding the hybrid protein in which two Stx2eB proteins are tandemly linked through PG17 to NtADH 5'-UTR, the secretory signal peptide is added to its amino terminus, PG12 is linked to its carboxyl terminus, and further the endoplasmic reticulum retention signal peptide is added to its carboxyl terminus is linked to NtADH 5'-UTR. Further, the DNA construct having the base sequence represented by SEQ ID NO: 103 is the DNA construct in which DNA encoding the hybrid protein in which two Stx2eB proteins are tandemly linked through PG22, the secretory signal peptide is added to its amino terminus, PG12 is linked to its carboxyl terminus, and further the endoplasmic reticulum retention signal peptide is added to its carboxyl terminus is linked to NtADH 5'-UTR.

The DNA construct having the base sequence represented by SEQ ID NO: 104 is the DNA construct in which DNA encoding the hybrid protein in which two Stx2eB proteins are tandemly linked through PG12, the chloroplast transit signal peptide is added to its amino terminus, and PG12 is linked to its carboxyl terminus is linked to NtADH 5'-UTR.

The DNA construct having the base sequence represented by SEQ ID NO: 105 is the DNA construct in which DNA encoding the hybrid protein in which three Stx2eB proteins are each tandemly linked through PG12 and PG12 is linked to its carboxyl terminus is linked to NtADH 5'-UTR.

The DNA construct having the base sequence represented by SEQ ID NO: 106 is the DNA construct in which DNA encoding the hybrid protein in which three Stx2eB proteins are each tandemly linked through PG12, the secretory signal peptide is added to its amino terminus, and PG12 is linked to its carboxyl terminus, and further the endoplasmic reticulum retention signal peptide is added to its carboxyl terminus is linked to NtADH 5'-UTR.

The DNA construct having the base sequence represented by SEQ ID NO: 107 is the DNA construct in which DNA encoding the hybrid protein in which three Stx2eB proteins are each tandemly linked through PG12, the chloroplast transit signal peptide is added to its amino terminus, and PG12 is linked to its carboxyl terminus is linked to NtADH 5'-UTR.

The DNA construct having the base sequence represented by SEQ ID NO: 108 is the DNA construct in which DNA encoding the hybrid protein in which two CTB proteins are tandemly linked through PG12 and PG12 is linked to its carboxyl terminus is linked to NtADH 5'-UTR.

The DNA construct having the base sequence represented by SEQ ID NO: 109 is the DNA construct in which DNA encoding the hybrid protein in which two CTB proteins are tandemly linked through PG17, the signal peptide is added to its amino terminus, PG12 is linked to its carboxyl terminus, and further the endoplasmic reticulum retention signal peptide is added to the its carboxyl terminus is linked to NtADH 5'-UTR. Further, the DNA construct having the base sequence represented by SEQ ID NO: 110 is the DNA construct in which DNA encoding the hybrid protein in which two CTB proteins are tandemly linked through PG22, the signal peptide is added to its amino terminus, PG12 is linked to its carboxyl terminus, and further the endoplasmic reticulum retention signal peptide is added to the its carboxyl terminus is linked to NtADH 5'-UTR.

The DNA construct having the base sequence represented by SEQ ID NO: 111 is the DNA construct in which DNA encoding the hybrid protein in which two CTB proteins are tandemly linked through PG12, the chloroplast transit signal peptide is added to its amino terminus, and PG12 is linked to its carboxyl terminus is linked to NtADH 5'-UTR.

The DNA construct of the present invention can be prepared by a general genetic engineering technique, which includes the following procedures: digesting DNAs including the 5'-untranslated region of an alcohol dehydrogenase gene derived from a plant, a DNA encoding a secretory signal peptide derived from a plant, a DNA encoding a chloroplast transit signal peptide, a DNA encoding a bacterial toxin protein, and a DNA encoding an endoplasmic reticulum retention signal peptide with suitable restriction enzymes; and ligating the resultant fragments with a suitable ligase.

The recombinant vector of the present invention is characterized by including the DNA construct of the present invention. The recombinant vector of the present invention may be a vector obtained by inserting a DNA encoding a hybrid protein of the present invention into a vector so that the DNA can be expressed in host cells to be introduced with the vector. The vector is not particularly limited as long as it can replicate in host cells, and examples thereof include a plasmid DNA and a viral DNA. In addition, the vector preferably includes a selective marker such as a drug resistance gene. The plasmid DNA can be prepared from *Escherichia coli* or Agrobacterium by the alkaline extraction method (Birnboim, H. C. & Doly, J. (1979) Nucleic acid Res 7: 1513) or a modified method thereof. Commercially available plasmids such as pBI221, pBI121, pBI101, and pIG121Hm may be used. The viral DNA may be, for example, pTB2 (Donson et al., 1991) (see Donson J., Kerney CM., Hilf ME., Dawson WO. Systemic expression of a bacterial gene by a tabacco mosaic virus-based vector. Proc. Natl. Acad. Sci.(1991) 88: 7204-7208).

Promoters to be used in vectors may be appropriately selected depending on host cells to be introduced with vectors. The promoters are preferably a cauliflower mosaic virus 35S promoter (Odell et al., 1985, Nature 313:810), a rice actin promoter (Zhang et al.1991 Plant Cell 3:1155), a corn ubiquitin promoter (Cornejo et al., 1993, Plant Mol. Biol., 23:567), etc. for example. Meanwhile, terminators to be used in vectors may be appropriately selected depending on host cells to be introduced with vectors. The terminators are preferably a nopaline synthase gene transcription terminator, a cauliflower mosaic virus 35S terminator, etc.

The recombinant vector of the present invention may be prepared as follows.

First, a DNA construct of the present invention is digested with a suitable restriction enzyme, or a restriction enzyme site is added to the DNA construct by PCR. Subsequently, the DNA construct is inserted into the restriction enzyme site or multicloning site of a vector.

The transformant of the present invention is characterized by being transformed with the recombinant vector of the present invention. The host cells to be used for transformation may be eukaryotic cells or prokaryotic cells.

The eukaryotic cells are preferably plant cells, particularly preferably cells of plants belonging to the family *Asteraceae, Solanaceae, Brassicaceae,* and *Chenopodiaceae.* Moreover, the eukaryotic cells are preferably cells of plants belonging to the genus *Lactuca,* particularly preferably *Lactuca sativa* cells. In the case of using *Lactuca sativa* cells as host cells, a cauliflower mosaic virus 35S RNA promoter or the like may be used in the vector.

The prokaryotic cells may be cells of *Escherichia coli, Agrobacterium tumefaciens,* etc.

The transformant of the present invention can be prepared by a general genetic engineering technique by introducing a vector of the present invention into host cells. For example, the transformant can be prepared by the introduction method using Agrobacterium (Hood, et al., 1993, Transgenic, Res. 2:218,Hiei, et al.,1994 Plant J. 6:271), an electroporation method (Tada, et al., 1990, Theor.Appl.Genet, 80:475), a polyethylene glycol method (Lazzeri, et al., 1991, Theor. Appl. Genet. 81:437), a particle gun method (Sanford, et al., 1987, J. Part. Sci. tech. 5:27), a polycation method (Ohtsuki), etc.

After introduction of the vector of the present invention into host cells, a transformant of the present invention can be selected based on the phenotype of a selective marker. If the selected transformant is cultured, the bacterial toxin protein can be produced. The culture medium and conditions for culture may be suitably selected depending on the type of a transformant.

In addition, in the case of using a plant cell as a host cell, culture of selected plant cell in accordance with a conventional method can regenerate a plant and accumulate the bacterial toxin protein in the plant cells or outside the cell membranes of the plant cells. The method depends on the type of the plant cell, and examples thereof include the method of Visser et al. (Theor.Appl.Genet, 78:594(1989)) for potato and the method of Nagata and Takebe (Planta, 99:12(1971)) for *Nicotiana tabacum.*

In the case of *Lactuca sativa,* a shoot can be regenerated in MS medium containing 0.1 mg/l NAA (naphthalene acetic acid), 0.05 mg/l BA (benzyladenine), and 0.5 g/l polyvinylpyrrolidone, and culture of the regenerated shoot in a 1/2 MS medium containing 0.5 g/l polyvinylpyrrolidone may cause rooting.

The seed of the present invention can be obtained by collecting a seed from a plant regenerated as above. If the seed of the present invention are sown and cultivated by a suitable method, a plant capable of producing a bacterial toxin protein can be obtained and is included in the transformant of the present invention.

### EXAMPLES

### <1> Transient expression experiment

### (1) Construction of Stx2eB transient expression vector

A vector containing a DNA construct in which DNA (SEQ ID NO: 5) encoding an Stx2e protein B subunit (Stx2eB) was linked to a 5'-untranslated region (NtADH 5'-UTR) of a tobacco alcohol dehydrogenase gene was prepared as follows.

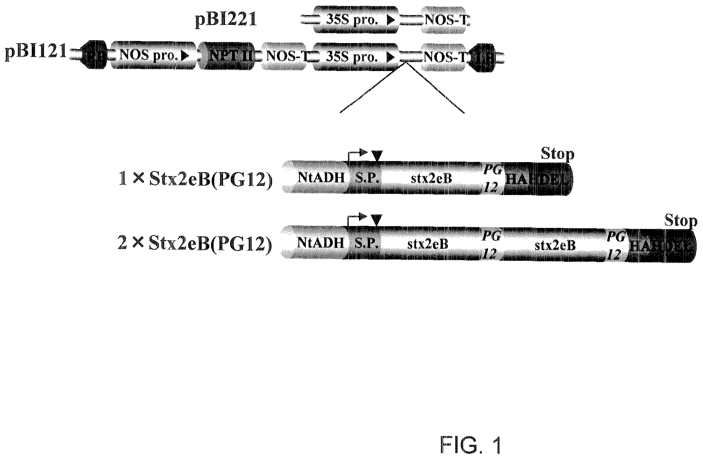

A design of the vector is shown in FIG. 1.

1×Stx2eB (PG12) denotes a DNA construct containing DNA in which DNA encoding PG12 is linked to DNA encoding Stx2eB. 2×Stx2eB (PG12) denotes a DNA construct containing DNA in which two DNAs encoding Stx2eB are linked using DNA encoding PG12 as a spacer.

In addition, a DNA construct, 3×Stx2eB (PG12) in which three DNAs encoding Stx2eB are linked using DNA encoding PG12 as a spacer, and a DNA construct, 4×Stx2eB (PG12) in which four DNAs encoding Stx2eB are linked using DNA encoding PG12 as a spacer were prepared as well.

Specific techniques are shown below.

PCR using a Kozak-stx2eb-F primer (SEQ ID NO: 30) and an stx2eb-R primer (SEQ ID NO: 31) was performed to amplify a DNA fragment encoding a mature region (except for a secretory signal peptide to periplasm, Ala 19 to Asn 87) of Stx2eB. The resulting DNA fragment was cloned into an EcoRV gap in pBluescript II SK. The resulting plasmid was cleaved with HindIII, and treated with T4 DNA polymerase, followed by self-ligation to convert a HindIII site to a NheI site (plasmid 1).

Stx2eB was inserted as follows into the multicloning site (MCS) of a transient expression vector in plant cells, pBI221 (Clontech).

In order to introduce SalI, KpnI, and SmaI sites into the MCS, SalKpnSma-F (SEQ ID NO: 32) and SalKpnSma-R (SEQ ID NO: 33) were annealed and phosphorylated with T4 polynucleotide kinase (T4 PNK) (TaKaRa) and inserted into the SacI gap of pBI221 (plasmid 2). Stx2eB was cleaved out from plasmid 1 using XbaI and KpnI to insert into plasmid 2, and the resultant product was arranged between a cauliflower mosaic virus 35S RNA promoter (35S pro.) and a nopaline synthase gene transcription terminator (NOS-T) (plasmid 3).

The 5'-untranslated region (NtADH 5'-UTR, SEQ ID NO: 23) of a tobacco alcohol dehydrogenase gene was amplified by PCR with ADH-221 (Sato et al., 2004, (see below)) as a template using ADH XbaI-F primer (SEQ ID NO: 34) and ADH NsiI-R primer (SEQ ID NO: 35). A DNA region (SEQ ID NO: 17) encoding a secretory signal peptide (SEQ ID NO: 18) of β-D glucan exohydrolase (GenBank ACCESSION AB017502) was amplified with a tobacco genomic DNA as a template using βD NsiI-F primer (SEQ ID NO: 36) and βD BamHI-R primer (SEQ ID NO: 37). The obtained respective DNA fragments of NtADH 5'-UTR and the secretory signal peptide were treated with NsiI (manufactured by Toyobo Co., Ltd.), ligated using Ligation High (manufactured by Toyobo Co., Ltd.), followed by being blunted, and cloned into the EcoRV gap in pBluescript II SK (manufactured by Stratagene) (plasmid 4).

Satoh et al., The 5'-untranslated region of the tobacco alcohol dehydrogenase gene functions as an effective translational enhancer in plant. J. Biosci. Bioeng. (2004) 98,1-8

Plasmid 4 was treated with NsiI, and blunted with T4 DNA polymerase (manufactured by Toyobo Co., Ltd.), followed by performing self-ligation to be ligated so that the initiation codon (atg) of NtADH was matched to the initiation codon of the secretory signal peptide (plasmid 5).

A DNA obtained by ligating an NtADH 5'-UTR fragment and a secretory signal peptide was amplified using plasmid 5 as a template and using ADH XbaI-F primer (SEQ ID NO: 34) and βD BamHI-R primer (SEQ ID NO: 35). The resultant DNA fragment was treated with XbaI and BamHI and inserted into the XbaI-BamHI gap of plasmid 3 (plasmid 6).

In order to add an endoplasmic reticulum retention signal (SEQ ID NO: 38), an HDEL-F primer (SEQ ID NO: 39) and an HDEL-R primer (SEQ ID NO: 40) were annealed and phosphorylated with T4 PNK, and the resultant product was inserted into the BglII gap of plasmid 6, which had been dephosphorylated with alkaline phosphatase (AP) (TakaRa) (plasmid 7).

An HA tag was added as a peptide tag for detecting Stx2eB. In order to add the HA tag, an HA-F primer (SEQ ID NO: 41) and an HA-R primer (SEQ ID NO: 42) were annealed and phosphorylated with T4 PNK. The resultant phosphorylated HA fragment was inserted into the BglII gap of plasmid 7 (plasmid 8) .

A PG12 spacer (SEQ ID NO: 2) was inserted between Stx2eB and the HA tag. A PG12-F primer (SEQ ID NO: 43) and a PG12-R primer (SEQ ID NO: 44) were annealed and phosphorylated with T4 PNK. The resulting phosphorylated DNA fragment was inserted into the BglII gap of plasmid 8 (1×Stx2eB (PG12)).

2×Stx2eB (PG12) was obtained by cleaving a 2eB-PG12 fragment out from 1×Stx2eB (PG12) with BamHI and BglII and then inserting the fragment into the BamHI gap of the 1×Stx2eB (PG12). 3×Stx2eB (PG12) was obtained by cleaving an Stx2eB-PG12 fragment out from 1×Stx2eB (PG12) with BamHI and BglII and then inserting the fragment into the BamHI gap of 2×Stx2eB (PG12). Further, 4×Stx2eB (PG12) was obtained by cleaving a 2x (Stx2eB-PG12) fragment out from 2×Stx2eB (PG12) with BamHI and BglII and then inserting the fragment into the BamHI gap of the 2×Stx2eB (PG12).

### (2) Construction of CTB transient expression vector

A vector containing a DNA construct (2×CTB (PG12)) in which DNA encoding a CT protein B subunit (CTB) had been linked to the 5'-untranslated region of a tobacco alcohol dehydrogenase gene was prepared as follows.

A DNA fragment (SEQ ID NO: 7) encoding the mature region (except for the secretory signal to the periplasm, Thr 22 to Asn 124) (SEQ ID NO: 8) of CTB was prepared. First, the following ten primers were prepared.
CTB1: SEQ ID NO: 45
CTB2: SEQ ID NO: 46
CTB3: SEQ ID NO: 47
CTB4: SEQ ID NO: 48
CTB5: SEQ ID NO: 49
CTB6: SEQ ID NO: 50
CTB7: SEQ ID NO: 51
CTB8: SEQ ID NO: 52
CTB9: SEQ ID NO: 53
CTB10: SEQ ID NO: 54

PCR using the primers synthesized above was performed under the condition described in Kang et al. (2004). That is, PCR was performed in combination of CTB1 and CTB2, CTB3 and CTB4, CTB5 and CTB6, CTB7 and CTB8, and CTB9 and CTB10, and DNA fragments of 72 bp (1+2), 74 bp (3+4), 67 bp (5+6), 82 bp (7+8) and 68 bp (9+10) were synthesized, respectively. Subsequently, the second PCR was performed in combination of CTB1+2 and CTB3+4, CTB3+4 and CTB5+6, CTB5+6 and CTB7+8, and CTB7+8 and CTB9+10, and DNA fragments of 135 bp (1+2+3+4), 132 bp (3+4+5+6), 138 bp (5+6+7+8), and 141 bp (7+8+9+10) were synthesized, respectively. Then, the third PCR was performed in combination of CTB1+2+3+4 and CTB3+4+5+6, and CTB5+6+7+8 and CTB7+8+9+10, and DNA fragments of 194 bp (1+2+3+4+5+6) and 198 bp (5+6+7+8+9+10) were synthesized, respectively. Finally, PCR was performed in combination of CTB1+2+3+4+5+6 and CTB5+6+7+8+9+10, and a DNA fragment of 315 bp in which a BamHI site and a BglII site were added to a CTB coding region was synthesized.

The DNA fragment prepared above was treated with BamHI and BglII, and inserted into a BamHI-BglII gap in plasmid 8 (plasmid 9).

The PG12 spacer (SEQ ID NO: 2) was inserted between CTB and the HA tag. A PG12-F primer (SEQ ID NO: 43) and a PG12-R primer (SEQ ID NO: 44) were annealed and phosphorylated with T4 PNK. The resulting phosphorylated DNA fragment was inserted into the BglII gap of plasmid 9 (1×CTB (PG12)).

A CTB-PG12 fragment was cut out from 1×CTB (PG12) using BamHI and BglII, and inserted into the BamHI gap of 1×CTB (PG12) (2×CTB (PG12)).

### (3) Transient expression test using Lactuca sativa protoplast

A leaf of potted *Lactuca sativa* (green wave) (about 1 g) was cut into 0.5-cm square pieces using a surgical knife, to thereby prepare leaf discs. The leaf discs were immersed in 500 mM mannitol, and shaken for 1 hour. The leaf discs were immersed in 50 ml of a protoplastization enzyme solution (1.0% cellulose RS (Yakult Honsha Co., Ltd.), 0.25% macerozyme R-10 (Yakult Honsha Co., Ltd.), 400 mM mannitol, 8 mM CaCl₂, and 5 mM Mes-KOH, pH 5.6), and the whole was shaken at room temperature for 2 hours. The protoplast suspension was passed through meshes of 100 um and 40 µm to remove the leaf discs. The protoplast suspension was centrifuged at 60 g for 5 minutes to precipitate the protoplast. The protoplast was resuspended in an aqueous solution containing 167 mM mannitol and 133 mM CaCl₂, and the suspension was centrifuged at 40 *g* for 5 minutes. The protoplast was resuspended in an aqueous solution containing 333 mM mannitol and 66.7 mM CaCl₂, and the suspension was centrifuged at 40 g for 5 minutes. The protoplast was suspended in W5 solution (154 mM NaCl, 125 mM CaCl₂, 5 mM KCl, 2 mM Mes-KOH, pH 5.6), and the suspension was allowed to stand on ice for 1 hour. The protoplast suspension was centrifuged at 40 g for 5 minutes, and the protoplast was suspended in an MaMg solution (400 mM mannitol, 15 mM MgCl₂, and 4 mM Mes-KOH, pH 5.6) to have a protoplast concentration of 2×10⁶ cells/ml.

Each of the Stx2eB transient expression vector and the CTB transient expression vector prepared above was mixed with 120 µL of a protoplast suspension, subsequently 140 µL of PEG solution (400 mM mannitol, 100 mM Ca(NO₃)₂ and 40% PEG) was added thereto, and the resulting mixture was blended gently and incubated for 7 minutes. Then, 1 mL of W5 solution was added to the protoplast suspension over about 20 minutes. A solution (1 mL) obtained by mixing 400 mM mannitol and the W5 solution at a ratio of 4:1 was added to the protoplast precipitated by centrifugation. LS medium (1 mL) containing 1% sucrose, 400 mM mannitol, and 0.3 mM carbenicillin was added to the protoplast precipitated by centrifugation, and the mixture was then cultured in a dark place at 25°C for 24 hours.

### (4) Western analysis

To the protoplast collected by centrifugation were added 30 µl of SDS-sample buffer (4% (w/v) SDS, 20% (w/v) glycerol, 0.05% (w/v) bromophenol blue, 300 mM β-mercaptoethanol, 125 mM Tris-HCl, pH 6.8), followed by thermal denaturation at 95°C for 2 minutes, to thereby prepare samples. Proteins were separated using a 15% acrylamide gel and blotted on a PVDF membrane (Hybond-P; Amersham) using an electro transfer system. An anti-HA antibody (No. 11 867 423 001, Roche) was used to detect Stx2eB and CTB.

### (a) Effects of linking number of Stx2eB

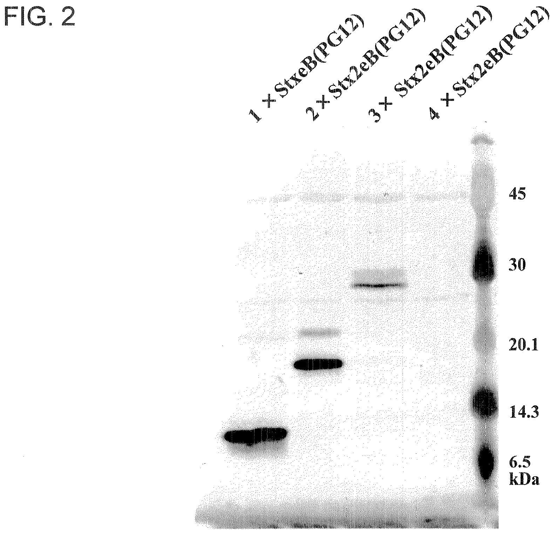

A result is shown in FIG. 2. When 1×Stx2eB (PG12) was expressed, a signal was detected at a position of about 8.5 kDa. When 2×Stx2eB (PG12) was expressed, a signal was detected at a position of about 17 kDa at the same level as when 1×Stx2eB (PG12) was expressed. When 3×Stx2eB (PG12) was expressed, a signal was detected at a position of about 26 kDa, the signal being smaller than that when 1×Stx2eB (PG12) was expressed. These corresponded to the molecular weights estimated from the design of the DNA constructs. When 4×Stx2eB (PG12) was expressed, the specific signal was below a detection limit.

From the above result, it was demonstrated that when 2×Stx2eB (PG12) and 3×Stx2eB (PG12) were expressed, hybrid proteins in which multiple Stx2eB proteins were linked could be produced.

Since each of the above DNA constructs contains one molecule of the HA tag (see FIG. 1), it is conceivable that a protein amount corresponding to about 2 times an Stx2eB protein amount when 1×Stx2eB (PG12) is expressed is accumulated when 2×Stx2eB (PG12) is expressed. That is, it has been found that when two DNAs encoding the Stx2eB protein are linked through DNA encoding PG12, the Stx2eB protein can be produced with very high efficiency.

Meanwhile, it has been also found that when three DNAs encoding the Stx2eB protein or four DNAs encoding the Stx2eB protein are linked through DNA encoding PG12, the amount of the produced Stx2eB protein is equal to or less than the amount when one Stx2eB protein is linked to PG12.

It has been found that the level of accumulated proteins tends to be higher in the Stx2eB protein (1×Stx2eB (PG12)) having PG12 added at its carboxyl terminus prepared in this experiment, compared with the Stx2eB protein having no PG12. This speculates that it is a favorable form that PG12 is added to the carboxyl terminus in the hybrid protein of the present invention.

### (b) Effects of linking number of CTB

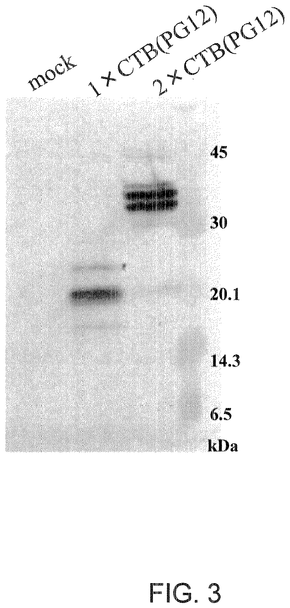

The results are shown in FIG. 3.

When 1×CTB (PG12) was expressed, a signal was detected at the position of about 20kDa. When 2×CTB (PG12) was expressed, a larger signal than that when 1×CTB (PG12) was expressed was detected at the positions of about 33 kDa and about 35 kDa.

From the above results, it was demonstrated that the hybrid protein in which two CTB proteins were linked could be produced when 2×CTB (PG12) was expressed. These corresponded to the molecular weights estimated from the design of the DNA constructs.

Since each of the above DNA constructs contains one molecule of the HA tag, it is conceivable that the protein amount corresponding to the CTB protein amount which is larger than two times the protein amount when 1×CTB (PG12) is expressed is accumulated when 2×CTB (PG12) is expressed. That is, it has been found that when two DNAs encoding the CTB protein are linked through DNA encoding PG12, the CTB protein can be produced with very high efficiency.

### (5) Analysis of localization of Stx2eB

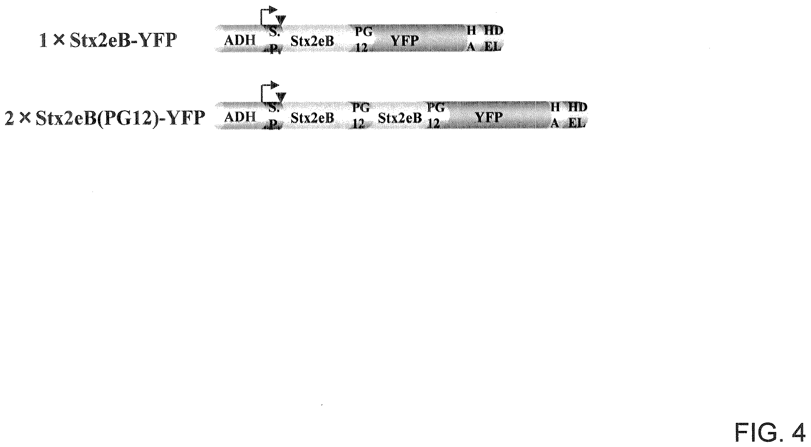

In order to analyze the localization of Stx2eB in the cell, a transient expression vector for the hybrid protein of Stx2eB and a yellow fluorescent protein YFP was prepared. The design for the vector is shown in FIG. 4. 1×Stx2eB (PG12)-YFP denotes a DNA construct in which DNA encoding the Stx2eB protein is linked to DNA encoding YFP. 2×Stx2eB (PG12)-YFP denotes a DNA construct in which DNA encoding the hybrid protein in which two Stx2eB proteins are linked through PG12 is linked to DNA encoding YFP. 2×Stx2eB (RS)-YFP using RS (Arg Ser) in place of PG12 as a spacer was also prepared.

A specific technique is shown below.

First, a DNA fragment of YFP was amplified by PCR with pEYFP (Clontech) as a template using a YFP-F primer (SEQ ID NO: 55) and a YEP-R primer (SEQ ID NO: 56). The resulting DNA fragment was treated with BamHI and BglII, and inserted into the BamHI-BglII gap of plasmid 8 (ER-YFP).

An Stx2eB fragment was cleaved out from plasmid 1 with BamHI and BglII and then inserted into the BamHI gap of the 1×Stx2eB (PG12) (2×Stx2eB (RS)).

An Stx2eB-PG12 fragment, a 2×(Stx2eB-RS) fragment, and a 2×(Stx2eB-PG12) fragment were cleaved out from 1×Stx2eB (PG12), 2×Stx2eB (RS), and 2xStx2eB (PG12) with BamHI-BglII, respectively, and each fragment was inserted into the BamHI gap of ER-YFP (1×Stx2eB (PG12)-YFP, 2×Stx2eB (RS)-YFP, and 2×Stx2eB (PG12)-YFP).

Meanwhile, an expression vector for a red fluorescent protein (mRFP, Campbell R. E. et al., 2002, (see below)) localized in the endoplasmic reticulum was prepared as a vector for visualizing the endoplasmic reticulum. PCR was performed using an mRFP-F primer (SEQ ID NO: 57) and an mRFP-R primer (SEQ ID NO: 58). The resulting DNA fragment was treated with BamHI and BglII, and inserted into the BamHI-BglII gap of plasmid 8 (ER-mRFP).
Campbell R. E. et al., A monomeric red fluorescent protein (2002), Proc. Nat. Acad. Sci., 99: 7877-7882.

The Stx2eB expression vector and the mRFP expression vector were introduced into the protoplasts of cultured tobacco cells (BY2) in the same way as the above methods, and observed using a confocal microscope observation system (LSM510, Zeiss).

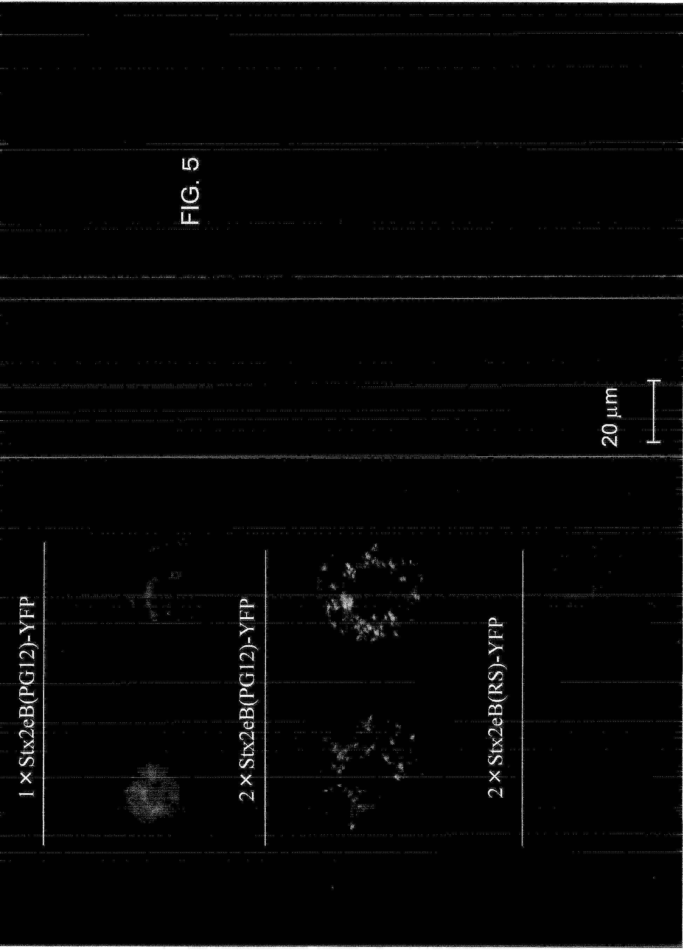

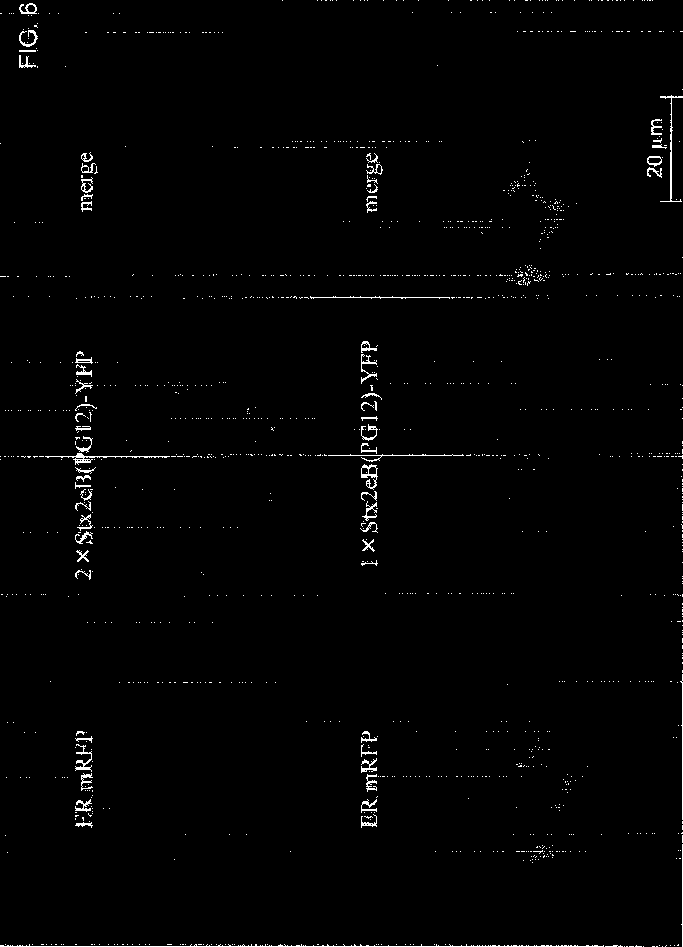

The results are shown in FIGS. 5 and 6.

FIG. 5 shows the localization of the hybrid protein of Stx2eB-YFP. When 2×Stx2eB (PG12)-YFB was expressed, it was observed that the hybrid protein of Stx2eB-YFP was localized granularly at about 100 granules/cell. When 1×Stx2eB (PG12)-YFP and 2×Stx2eB (RS)-YFP were expressed, no granule was observed.

In FIG. 6, an image A in a leftmost column shows the localization of mRFP in a certain protoplast. The localization of mRFP reflects the location of the endoplasmic reticulum. An image B in a middle column shows the localization of the hybrid protein of Stx2eB-YFP in the identical protoplast. An image in a rightmost column is a composite image of the image A and the image B. It is found from this composite image that the hybrid protein of Stx2eB-YFP is localized granularly in the endoplasmic reticula.

### (6) Effect of vesicular transport function

It was examined in which process after the protein translation the accumulation and aggregation of 2×Stx2eB (PG12) occur.

2×Stx2eB (PG12)-YFP was co-expressed with an *Arabidopsis* vesicular transport regulation protein ARF1 or a dominant negative mutant thereof ARF1 (Q71L) (ARF1DN). As a reporter for inhibition of protein transport to Golgi apparatus by the co-expression with ARF1DN, vacuole-GFP was co-expressed with each ARF1. An expression vector for each ARF1 was constructed as follows. An expression vector for the vacuole-GFP can be prepared with reference to the following document.

Di Sansebastiano et. al., Specific accumulation of GFP in a non-acidic vacuolar compartment via a C-terminal propeptide-mediated sorting pathway. Plant J. (1998) 15, 449-457

As the vesicular transport regulation protein, expression vectors for *Arabidopsis* ARF1 (GenBank ACCESSION No. M95166) and for the dominant negative mutant thereof ARF1 (Q71L) (Misaki Takeuchi et al., 2002, (see below)) were constructed. PCR using cDNA prepared from *Arabidopsis* embryo plant as a template was performed using an ARF1-F primer (SEQ ID NO: 59) and an ARF1-R primer (SEQ ID NO: 60). The resulting DNA fragment was subcloned into the EcoRV gap in pBluescript (Stratagene). Another PCR was performed using an ARFQL-F primer (SEQ ID NO: 61) and an ARFQL-R primer (SEQ ID NO: 62) to substitute a glutamine residue at position 71 with a leucine residue. Each resulting ARF1 fragment was subcloned into a transient expression vector pBI221,

Each of the vectors prepared was introduced into the protoplast of the cultured tobacco cell in the same manner as that described above, and the respective proteins were co-expressed to examine the localization of 2×Stx2eB (PG12).

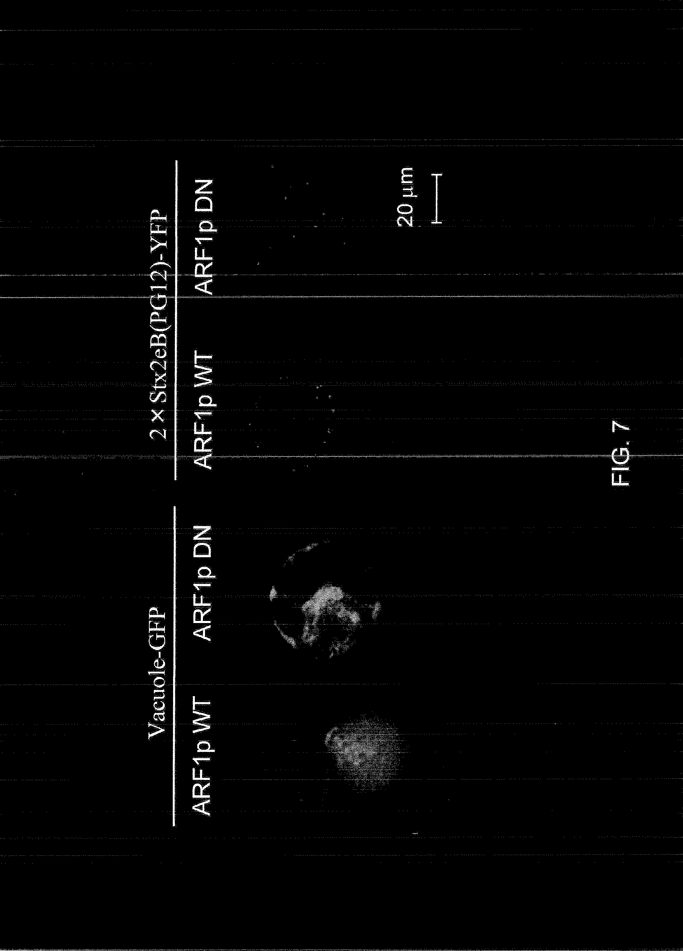

The result is shown in FIG. 7.

Both when 2×Stx2eB (PG12)-YFP was co-expressed with ARF1 and co-expressed with ARF1 (Q71L), granules were formed as observed in an expression of 2xStx2eB (PG12)-YFP alone. On the other hand, co-expression of ARF1 (Q71L) inhibited the exit of vacuole-GFP from ER. It was speculated from these that the granule formation is not dependent on vesicular transport process from the ER to Golgi apparatus.

### <2> Transformation experiments using cultured tobacco cells

### (1) Construction of vectors for transformation

1×Stx2eB (PG12), 2×Stx2eB (PG12), 3×Stx2eB (PG12), and 4×Stx2eB (PG12) were prepared in the same way as above.

Further, by using RS (Arg, Ser), PG7 (SEQ ID NO: 63), or SG12 (SEQ ID NO: 64) as a spacer instead of PG12, DNA constructs, 2×Stx2eB (RS), 2×Stx2eB (PG7), and 2×Stx2eB (SG12) were prepared by the following methods.

A PG7 spacer (SEQ ID NO: 63) was inserted between Stx2eB and the HA tag in the plasmid 8. A PG7-F primer (SEQ ID NO: 65) and a PG7-R primer (SEQ ID NO: 66) were annealed and phosphorylated with T4 PNK. The obtained phosphorylated DNA fragment was inserted into the BglII gap of plasmid 8 (plasmid 10) .

An SG12 spacer (SEQ ID NO: 64) was inserted between Stx2eB and the HA tag. An SG12-F primer (SEQ ID NO: 67) and an SG12-R primer (SEQ ID NO: 68) were annealed and phosphorylated with T4 PNK. The obtained phosphorylated DNA fragment was inserted into the BglII gap of plasmid 8 (plasmid 11).

An Stx2eB fragment was cleaved out from plasmid 1 with BamHI and BglII and then inserted into the BamHI gap of 1×Stx2eB (PG12) (2×Stx2eB (RS)). An Stx2eB-PG7 fragment was cleaved out from plasmid 10 with BamHI and BglII and then inserted into the BamHI gap of 1×Stx2eB (PG12) (2×Stx2eB (PG7)). An Stx2eB-SG12 fragment was cleaved out from plasmid 11 with BamHI and BglII and then inserted into the BamHI gap of 1×Stx2eB (PG12) (2×Stx2eB (SG12)).

In order to produce Stx2eB using a stable transformant of the plant, each of the DNA constructs of the above Stx2eB was subcloned into a vector for transformation (for the design of the vectors, see FIG. 1). Each of 1×Stx2eB (PG12), 2×Stx2eB (PG12), 3×Stx2eB (PG12), 4×Stx2eB (PG12), 2×Stx2eB (RS), 2×Stx2eB (PG7), and 2×Stx2eB (SG12) was inserted into pBI121 (Clontech) using XbaI and SacI, and allocated between a cauliflower mosaic virus 35S RNA promoter (35S pro.) and a nopaline synthetase gene transcription terminator (NOS-T).

### (2) Transformation of cultured tobacco cells

The produced vector for transformation was introduced into *Agrobacterium tumefacience* EHA105 using an electroporation method. An *Agrobacterium* medium (100 µL) cultured in 5 mL of LB medium containing 100 mg/L of kanamycin at 28°C for 2 nights was mixed with 5 to 10 mL of a suspension of cultured tobacco cells (*Nicotiana tabacum,* cv BY2) on the fourth day of the culture in a petri-dish, and the mixture was co-cultured by being left to stand in a dark place at 25°C for 2 nights. In order to remove *Agrobacterium,* the medium in the petri-dish was transferred into a 15-mL centrifuging tube, which was then centrifuged (1,000 rpm, 5 minutes, 4°C), and a supernatant was removed. A modified LS medium was charged, and the tube was centrifuged (1,000 rpm, 5 minutes, 4°C) to wash the cells. This washing operation was repeated four times to remove *Agrobacterium.* The resultant BY2 cells were then placed in a modified LS agar medium containing kanamycin (100 mg/L), and cultured by being left to stand in the dark place at 25°C. After about 2 to 3 weeks, cells which has formed a callus were transferred to a new plate, and a growing clone was selected.

### (3) Semi-quantification of Stx2eB protein using western analysis

Cultured tobacco cells cultured in a plate medium were collected in a centrifuging tube, and 1 µL of SDS sample buffer per mg of cell weight was added. The cells were denatured at 95°C for 2 minutes to make a sample for electrophoresis. Proteins were separated using 15% acrylamide gel, and then blotted onto a PVDF membrane (Hybond-P; Amersham) using an electrotransfer apparatus. An Stx2eB protein was detected using an anti-HA antibody (No. 11 867 423 001, Roche). Serial dilution of Stx2eB having the HA tag at known concentrations was prepared, and loaded on the gel. A calibration curve was prepared based on their signal intensities, and the amount of Stx2eB proteins in each sample was calculated.

The results are shown below.

### (a) Effects of linking number of Stx2eB

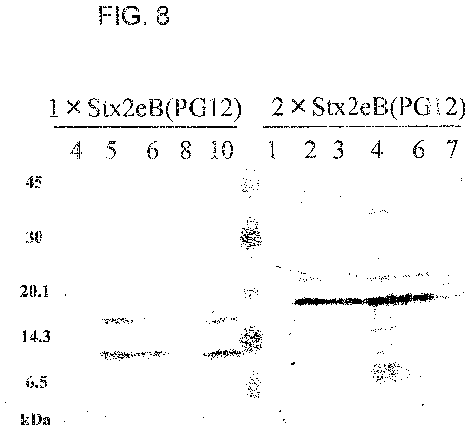

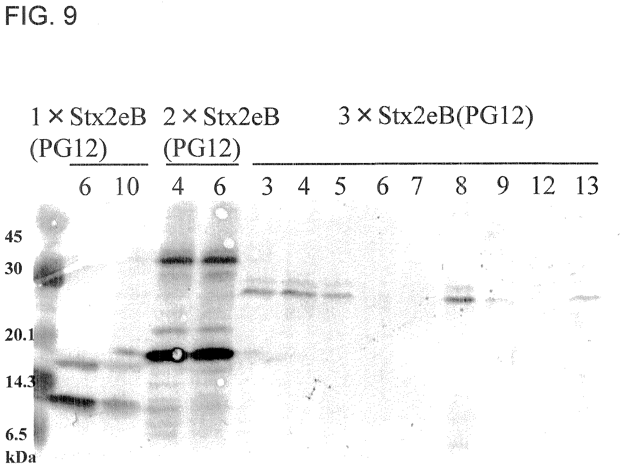

The results are shown in FIGS. 8 and 9.

When 1×Stx2eB (PG12) was expressed, signals were detected at the positions of about 10 kDa and about 17 kDa. They are presumed to be Stx2eB in which the signal peptide was cleaved and Stx2eB in which the signal peptide is not cleaved, respectively. When 2×Stx2eB (PG12) was expressed, a signal was detected at the position of about 19 kDa at the similar level to that when 1×Stx2eB (PG12) was expressed. When 3×Stx2eB (PG12) was expressed, a signal was detected at the position of about 26 kDa, the signal being smaller than that when 1×Stx2eB (PG12) was expressed. These corresponded to the molecular weights estimated from the design of the DNA constructs. When 4×Stx2eB (PG12) was expressed, a specific signal was below the detection limit (data not shown).

It has been found from the above that 2×Stx2eB (PG12) accumulates the larger amount of Stx2eB than 1×Stx2eB (PG12) and 3×Stx2eB (PG12).

### (b) Effects of spacer

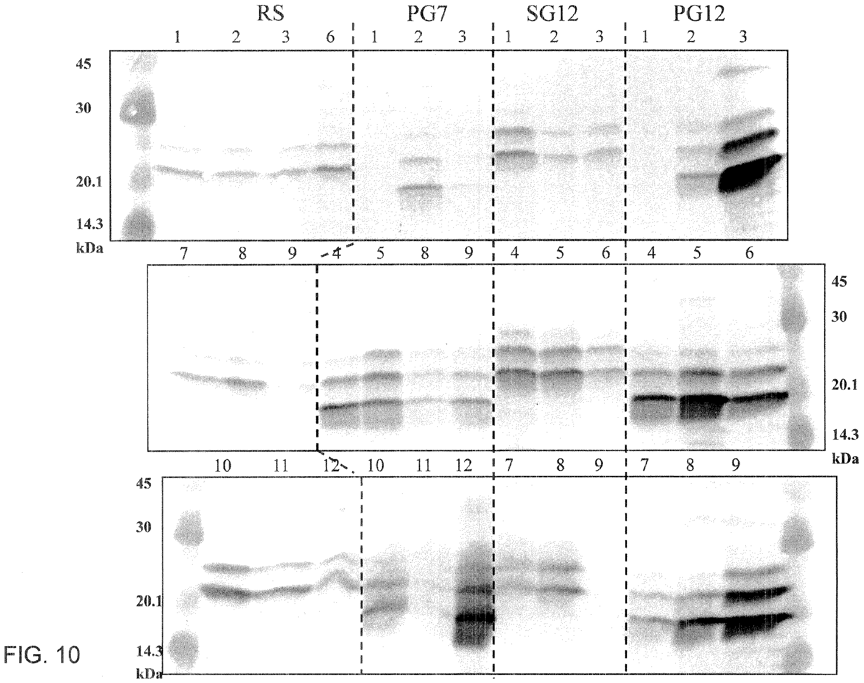

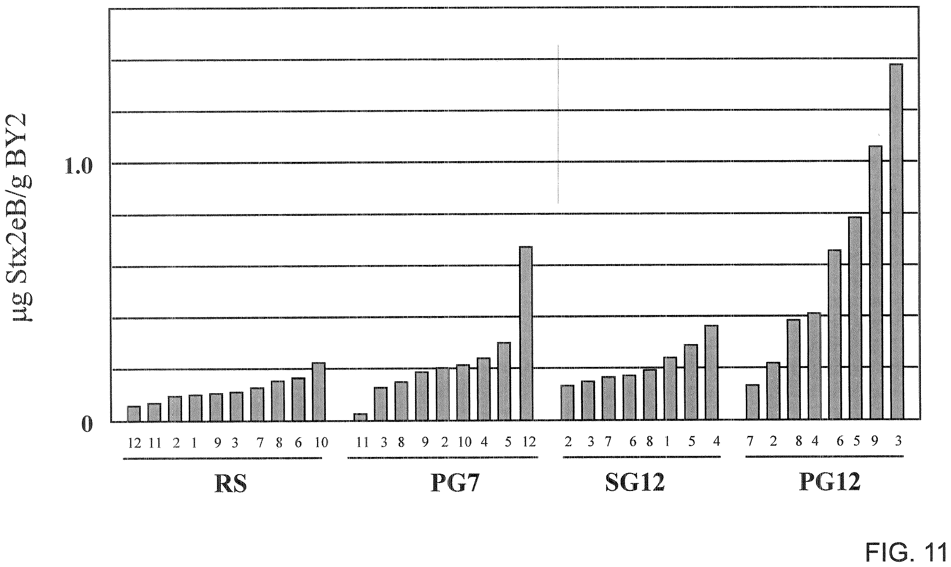

The results are shown in FIGS. 10 and 11.

Based on the intensity of the signal corresponding to Stx2eB, the level of accumulated Stx2eB was the highest when PG12 was used as a spacer. The levels were secondly high when PG7 and SG12 were used, which were in the similar degree. The level was lowest when RS was used. This indicates that the length and the amino acid sequence of the spacer between two Stx2eBs affect the level of the accumulated 2×Stx2eB protein.

### (4) Quantification of mRNA by real-time PCR

It was examined whether the level of the accumulated protein was influenced by a transcription level or not.

RNA was prepared using RNeasy Mini Kit (Qiagen) from each of the transformed BY2 cells obtained above. The resulting RNA was treated with DNase, and then reversely transcribed using Transcriptor Reverse Transcriptase (Roche). Real-time PCR was performed using SYBR Green PCR Master Mix (Applied Biosystems). A primer set (SEQ ID NO: 69 and SEQ ID NO: 70) which amplified the region containing NtADH 5'-UTR and the signal peptide which were common in the respective constructs was used for the quantification of Stx2eB mRNA. The amount of an expressed BY2 ubiquitin gene was quantified using a UBQ-F primer (SEQ ID NO: 71) and a UBQ-R primer (SEQ ID NO: 72) to compensate the mRNA level of an Stx2eB gene. Note that the mRNA level of 1×Stx2eB (PG12) was calculated by multiplying a quantified value by 1/2.

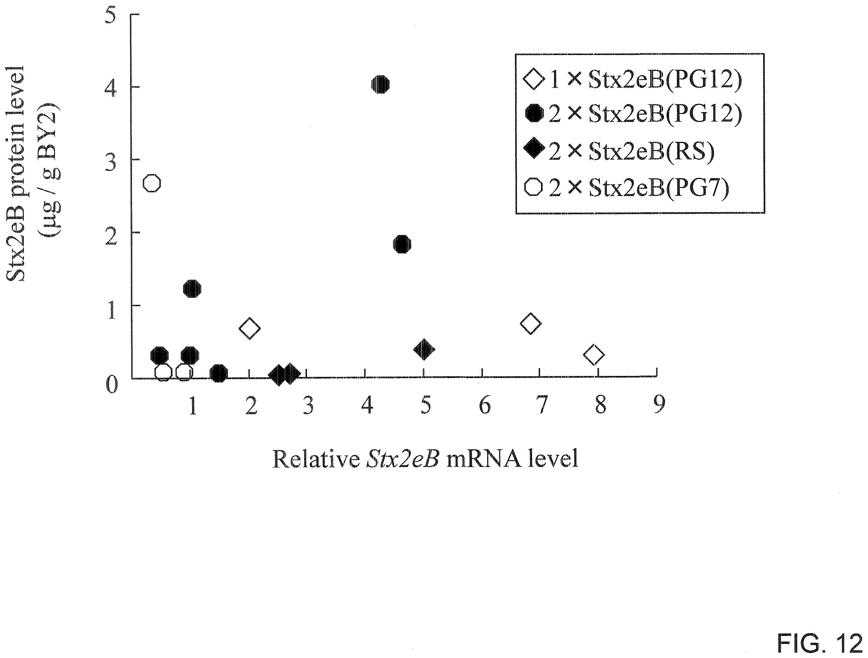

The results are shown in FIG. 12.

The accumulation levels of Stx2eB protein per mRNA tend to be higher in cells expressing 2×Stx2eB (PG12) than those in cells expressing 2×Stx2eB (RS) or 1×Stx2eB (PG12). This indicates that the difference of the spacer does not influence on the transcription level but influences on a translation level or stability of the protein after the translation. Considering together the result that the 2×Stx2eB protein was localized granularly, it is conceivable that the spacer influences on the stability of the protein after the translation.

### <3> Transient expression experiments

### (1) Construction of Stx2eB transient expression vectors

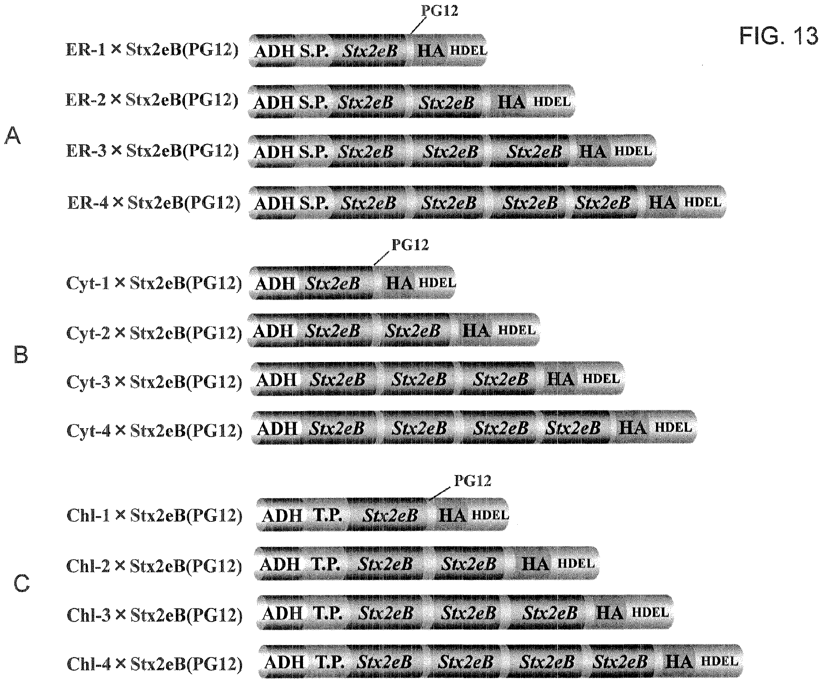

Transient expression vectors for 1×Stx2eB (PG12), 2×Stx2eB (PG12), 3×Stx2eB (PG12), and 4×Stx2eB (PG12) were constructed by the method in above <1> (1) (FIG. 13-A). These vectors are referred to as ER-1×Stx2eB (PG12), ER-2×Stx2eB (PG12), ER-3×Stx2eB (PG12), and ER-4×Stx2eB (PG12), respectively. Note that "ER" means the endoplasmic reticulum type.

Further, transient expression vectors containing the cytoplasm (Cyt) type of DNA construct (FIG. 13-B) and expression vectors containing the chloroplast (Chl) type of DNA construct (FIG. 13-C) were constructed by the following method. These DNA constructs were designed to contain DNA encoding the endoplasmic reticulum retention signal peptide, for the purpose of expressing the hybrid protein having as close a structure as possible to that of the endoplasmic reticulum type of hybrid protein. But, since these DNA constructs do not contain DNA encoding the secretory signal peptide, the endoplasmic reticulum retention signal peptide does not exert its function (retention of the protein in the endoplasmic reticulum) in the produced hybrid protein.

An NtADH 5'-UTR fragment was amplified by PCR using an ADH XbaI-F primer (SEQ ID NO: 34) and an ADH BamHI-R primer (SEQ ID NO: 112), and the resulting DNA fragment was treated with XbaI and BamHI. The XbaI-BamHI fragment of NtADH 5'-UTR was inserted into the XbaI-BamHI gap of each of ER-1×Stx2eB (PG12), ER-2×Stx2eB (PG12), ER-3×Stx2eB (PG12), and ER-4×Stx2eB (PG12) to prepare Cyt-1×Stx2eB (PG12), Cyt-2×Stx2eB (PG12), Cyt-3×Stx2eB (PG12), and Cyt-4×Stx2eB (PG12) which were cytoplasm type Stx2eB vectors.

The NtADH 5'-UTR fragment was amplified by PCR using an ADH XbaI-F primer (SEQ ID NO: 34) and an ADH NsiI-R primer (SEQ ID NO: 35). A DNA fragment (SEQ ID NO: 80) encoding the transit signal peptide, the chloroplast being derived from *Lactuca sativa* Rbcs (Rubisco small subunit) (GenBank ACCESSION D14001) (transit peptide, T.P.), was amplified by PCR with cDNA of a *Lactuca sativa* leaf as a template using a TP NsiI-F primer (SEQ ID NO: 113) and a TP BamHI-R primer (SEQ ID NO: 114). Each resulting DNA fragment of NtADH 5'-UTR and each DNA fragment of the secretory signal peptide was treated with NsiI (manufactured by Toyobo Co., Ltd.), ligated using Ligation High (Toyobo Co., Ltd.) followed by being blunted, and cloned into the EcoRV gap of pBluescript II SK (manufactured by Stratagene) (plasmid 12). Plasmid 12 was treated with NsiI, blunted with T4 DNA polymerase (Toyobo Co., Ltd.), and then self-ligated to be fused so that the initiation codon of NtADH and the initiation codon of Rbcs were matched (plasmid 13). An NtADH 5'-UTR-T.P. fusion fragment was cut out from plasmid 13 using XbaI and BamHI, and inserted into the XbaI-BamHI gap of each of ER-1×Stx2eB (PG12), ER-2×Stx2eB (PG12), ER-3×Stx2eB (PG12), and ER-4×Stx2eB (PG12) to prepare Chl-1×Stx2eB (PG12), Chl-2×Stx2eB (PG12), Chl-3×Stx2eB (PG12), and Chl-4×Stx2eB (PG12), which were chloroplast type Stx2eB vectors.

### (2) Production of CTB transient expression vectors

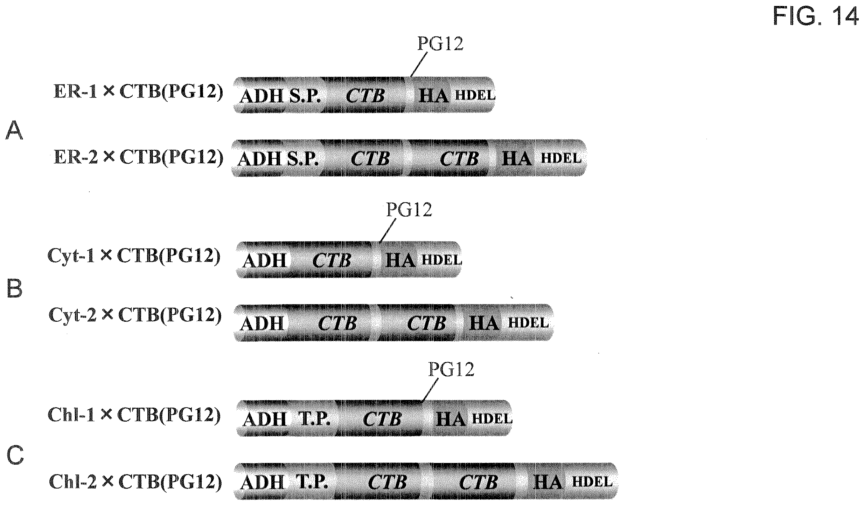

Transient expression vectors for 1×CTB (PG12) and 2×CTB (PG12) were constructed by the method in above <1> (2) (FIG. 14-A). Hereinafter, these vectors are referred to as ER-1×CTB (PG12) and ER-2×CTB (PG12).

Transient expression vectors containing the cytoplasm (Cyt) type of DNA construct (FIG. 14-B) and expression vectors containing the chloroplast (Chl) type of DNA construct (FIG. 14-C) were constructed by the following methods.

A CTB-PG12 fragment was cut out from ER-1×CTB (PG12) using BamHI and BglII, and inserted into the BamHI-BglII gap in Cyt-1×Stx2eB (PG12) and the BamHI-BglII gap in Chl-1×Stx2eB (PG12) produced in above <3> (1) to prepare cytoplasm type of 1×CTB (PG12) and chloroplast type of 1×CTB (PG12) (Cyt-1×CTB (PG12), Chl-1×CTB (PG12)).

Subsequently, a 2×(CTB-PG12) fragment was cut out from ER-2×CTB (PG12) using BamHI and BglII, and inserted into the BamHI-BglII gap of Cyt-1×Stx2eB (PG12) and the BamHI-BglII gap of Chl-1×Stx2eB (PG12) to prepare cytoplasm type of 2×CTB (PG12) and chloroplast type of 2×CTB (PG12) (Cyt-2×CTB (PG12), Chl-2×CTB (PG12)).

### (3) Transient expression experiments and western analysis

Transient expression experiments were carried out using *Lactuca sativa* protoplasts in the same way as in above <1> (3). Subsequently, Stx2eB and CTB were detected in the same way as in <1> (4).

### (a) Effects of linking number of Stx2eB

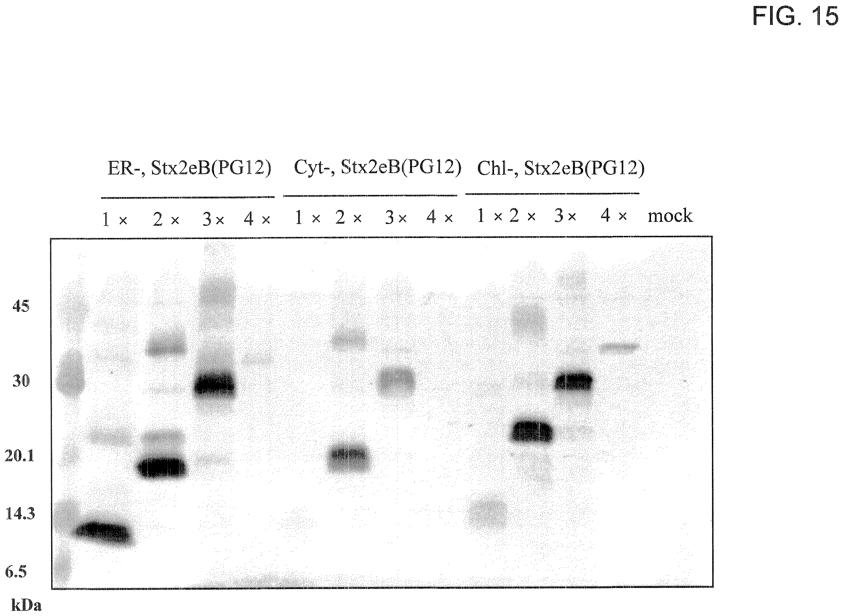

The results are shown in FIG. 15.

When ER-1×Stx2eB (PG12) was expressed, a signal was detected at the position of about 10 kDa. When ER-2×Stx2eB (PG12) was expressed, a signal was detected at the position of about 19 kDa, the signal being larger than that when ER-1×Stx2eB (PG12) was expressed. When ER-3×Stx2eB (PG12) was expressed, a signal was detected at the position of about 27 kDa, the signal being larger than that when ER-1×Stx2eB (PG12) was expressed. These corresponded to the molecular weights estimated from the design of the DNA constructs. When ER-4×Stx2eB (PG12) was expressed, a specific signal was below the detection limit.

When Cyt-1×Stx2eB (PG12) was expressed, a specific signal was below the detection limit. When Cyt-2×Stx2eB (PG12) was expressed, a signal was detected at the position of about 20 kDa. When Cyt-3×Stx2eB (PG12) was expressed, a signal was detected at the position of about 30 kDa. These corresponded to the molecular weights estimated from the design of the DNA constructs. Further, when Cyt-4×Stx2eB (PG12) was expressed, a specific signal was below the detection limit.

When Chl-1×Stx2eB (PG12) was expressed, a signal was detected faintly at the position of about 14 kDa. When Chl-2×Stx2eB (PG12) was expressed, a signal was detected at the position of about 22 kDa, the signal being at the similar level to that when ER-3×Stx2eB (PG12) was expressed. When Chl-3×Stx2eB (PG12) was expressed, a signal was detected at the position of about 30 kDa, the signal being at the similar level to that when Chl-2×Stx2eB (PG12) was expressed. These corresponded to the molecular weights estimated from the design of the DNA constructs. When Chl-4×Stx2eB (PG12) was expressed, a signal was detected faintly at the position of about 34 kDa.

Since each of the above DNA constructs contains one molecule of the HA tag (see FIG. 13), when DNA encoding two Stx2eBs is expressed and when DNA encoding three Stx2eBs is expressed, the amounts of the accumulated proteins are thought to correspond to about two times and about three times, respectively, the amount when DNA containing one Stx2eB is expressed.

Therefore, it has been found that when any of the endoplasmic reticulum type (ER), cytoplasm type (Cyt), and chloroplast type (Chl) of DNA constructs is expressed, the Stx2eB protein can be more efficiently accumulated when the protein in which two or three Stx2eBs are tandemly linked through the spacer is expressed than when one Stx2eB protein is expressed.

### (b) Effects of linking number of CTB

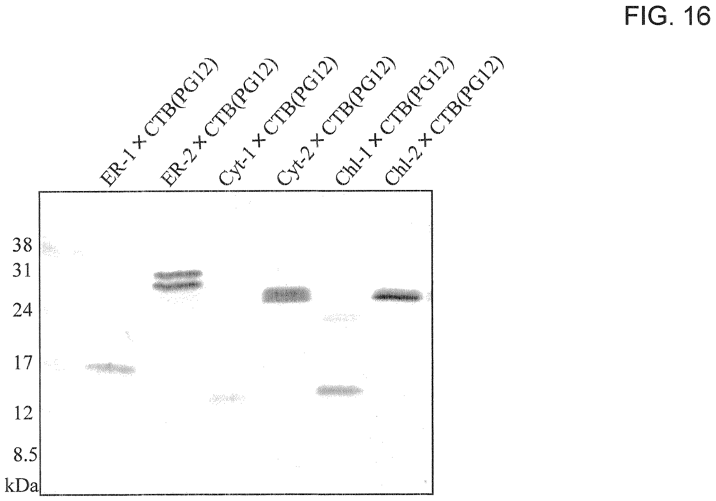

The results are shown in FIG. 16.

When ER-1×CTB (PG12) was expressed, a signal was detected at the position of about 17 kDa. When ER-2×CTB (PG12) was expressed, a larger signal than that when ER-1×Stx2eB (PG12) was expressed was detected at the positions of about 28 kDa and about 30 kDa. These corresponded to the molecular weights estimated from the design of the DNA constructs.

When Cyt-1×CTB (PG12) was expressed, a signal was faintly detected at the position of about 14 kDa. When Cyt-2×CTB (PG12) was expressed, a signal was detected at the position of about 26 kDa, the signal being at the similar level to that when ER-2×CTB (PG12) was expressed. These corresponded to the molecular weights estimated from the design of the DNA constructs.

When Chl-1×CTB (PG12) was expressed, a signal was detected at the position of about 14 kDa. When Chl-2×CTB (PG12) was expressed, a signal was detected at the position of about 26 kDa, the signal being at the similar level to that when ER-2×CTB (PG12) was expressed. These corresponded to the molecular weights estimated from the design of the DNA constructs.

From the above, it has been found that when any of the endoplasmic reticulum type (ER), cytoplasm type (Cyt), and chloroplast type (Chl) of DNA constructs is expressed, CTB proteins can be more efficiently accumulated when the protein in which two CTBs are tandemly linked through the spacer is expressed than when one CTB protein is expressed.

### <4> Transformation experiments using cultured tobacco cells

### (1) Construction of vectors for transformation

Transformation experiments were performed using ER-2×Stx2eB (PG12), Cyt-1×Stx2eB (PG12), Cyt-2xStx2eB (PG12), and Cyt-3×Stx2eB (PG12) prepared above.

Vectors for transformation were prepared in the same way as the method in above <2> (1).

### (2) Transformation and western analysis of cultured tobacco cells

Transformation experiments and the western analysis were carried out in the same ways as the methods in above <2> (2) and (3).

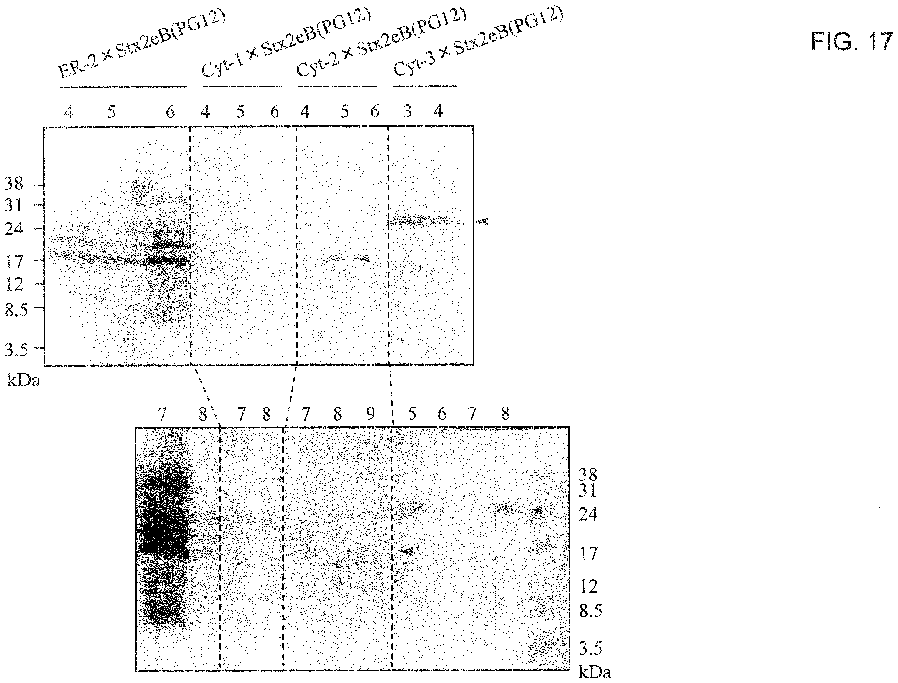

The results are shown in FIG. 17.

When ER-2×Stx2eB (PG12) was expressed, a signal was detected at the positions of about 19, 21, and 23 kDa. When Cyt-1×Stx2eB (PG12) was expressed, a specific signal was below the detection limit. When Cyt-2xStx2eB (PG12) was expressed, a signal was detected at the position of about 19 kDa. When Cyt-3×Stx2eB (PG12) was expressed, a signal was detected at the position of about 27 kDa, the signal being larger than that when Cyt-2×Stx2eB (PG12) was expressed.

From the above, it has been found that also in the transformant of the cultured tobacco cell, Stx2eB proteins can be more efficiently accumulated when the protein in which two or three Stx2eBs are tandemly linked through the spacer is expressed than when one Stx2eB protein is expressed. It has been also found that when the cytoplasm type of DNA construct is expressed in the transformant of the cultured tobacco cell, in particular when the protein in which three Stx2eBs are tandemly linked through the spacer is expressed, the Stx2eB proteins can be efficiently accumulated.

It has been also found that in the transformant of the cultured tobacco cell, the Stx2eB proteins can be more efficiently accumulated when the endoplasmic reticulum type of DNA construct is expressed than when the cytoplasm type of DNA construct is expressed.

### <5> Transformation experiments using tobacco plant body

### (1) Construction of vectors for transformation

Transformation experiments were performed using ER-2×Stx2eB (PG12), Chl-1×Stx2eB (PG12), Chl-2×Stx2eB (PG12), and Chl-3×Stx2eB (PG12) prepared above.

Vectors for transformation were prepared in the same way as the method in above <2> (1).

### (2) Transformation of tobacco plant body

Tobacco plant bodies were transformed by the following method using the vectors prepared above.

Seeds of the tobacco plant body (*Nicotiana tabacum* L. cv. Petit habana SR1) were sterilized and seeded on an MS medium. A leaf portion of the sterilized *Nicotiana tabacum* was cut into pieces each having a size of about 1×1 cm without including leaf veins, and placed to face up the backside of the leaf in a petri-dish containing sterile water. An *Agrobacterium* suspension cultured for two nights in the LB medium containing 100 mg/L of kanamycin and obtained in above <2> (2) was poured in the petri-dish, and the leaf piece was immersed therein for 3 to 5 minutes. The leaf piece was picked up, and an extra bacterial medium on the leaf piece was wiped with sterile kim-towel. The leaf piece was placed on a callus formation medium and cultured at 25°C. After 2 to 3 days, when *Agrobacterium* became visible on the medium, the leaf piece was transferred into a 50-mL tube, washed five times with sterile water, placed on a callus formation medium (containing 100 mg/L of kanamycin and 250 mg/L of carbenicillin), and cultured at 25°C for 1 to 2 weeks. When the leaf piece curled up compared with the original and showed a concavoconvex surface, the leaf piece was transferred into a shoot formation medium (containing 100 mg/L of kanamycin and 250 mg/L of carbenicillin). After additional 4 to 6 weeks, a shoot having a developed stem and leaf portion was cut off, transferred to a root formation medium (containing 100 mg/L of kanamycin and 250 mg/L of carbenicillin), and cultured at 25°C until rhizogenesis was observed. A plant body grown to a certain size was grown as a pot plant.

### (3) Western analysis

The leaf of the genetically engineered tobacco plant body produced above was sampled, and an SDS sample buffer was added thereto in the proportion of 1µL of SDS to 1 mg of leaf. The sample was thermally denatured at 95°C for 2 minutes to serve as the sample for electrophoresis. Proteins were separated using 15% acrylamide gel, and then blotted onto a PVDF membrane (Hybond-P; Amersham) using an electrotransfer apparatus. The Stx2eB protein was detected using an anti-HA antibody (No. 11 867 423 001, Roche).

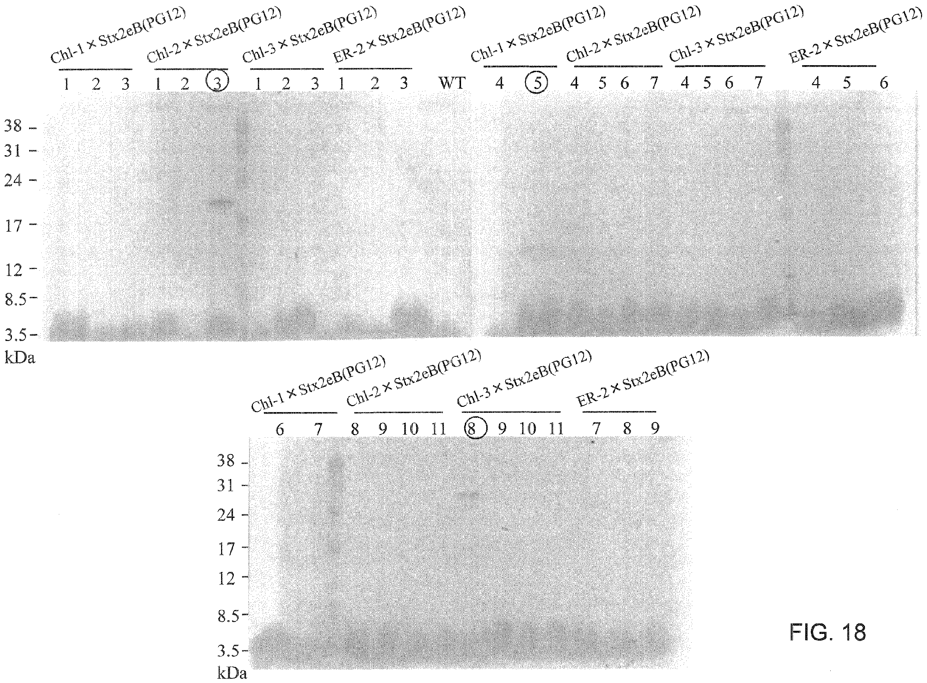

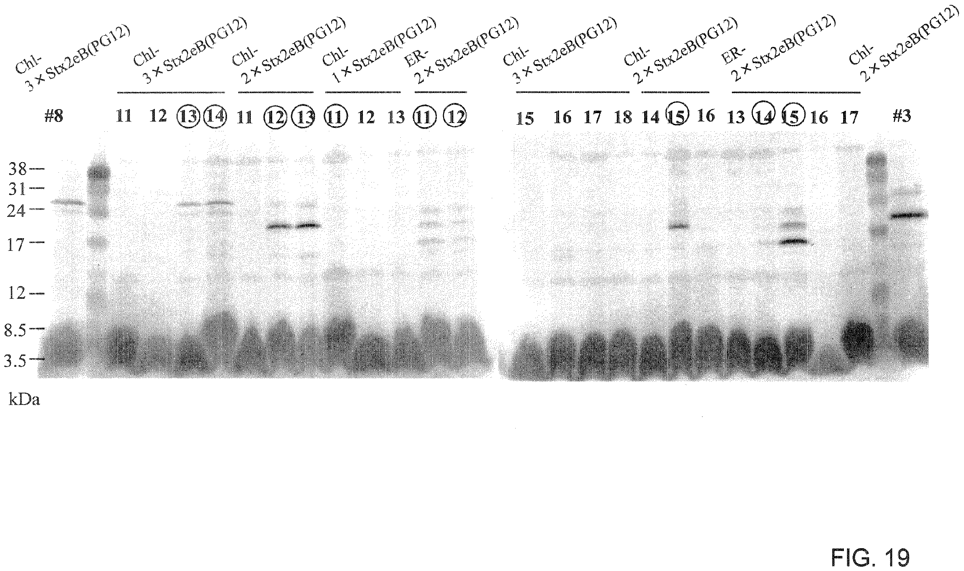

The results are shown in FIGS. 18 and 19.

Clones in which Stx2eB was accumulated with high efficiency were obtained in the plant body transformed with one of ER-2×Stx2eB (PG12), Chl-1×Stx2eB (PG12), Chl-2×Stx2eB (PG12), and Chl-3×Stx2eB (PG12). Also in the chloroplast type of DNA constructs, it has been found that the clone accumulating Stx2eB efficiently is obtained with a higher probability when the protein in which two or three Stx2eBs are tandemly linked through the spacer is expressed than when one Stx2eB protein is expressed.

When ER-2×Stx2eB (PG12) was expressed, signals were detected at the positions of about 15 kDa, about 19 kDa, and about 22 kDa. When Chl-1×Stx2eB (PG12) was expressed, a signal was detected at the position of about 12 kDa. When Chl-2×Stx2eB (PG12) was expressed, a signal was detected at the position of about 19 kDa. When Chl-3×Stx2eB (PG12) was expressed, a signal was detected at the position of about 27 kDa. These corresponded to the molecular weights estimated from the design of the DNA constructs.

### <6> Transformation experiments using cultured tobacco cells

### (1) Construction of vectors for Stx2eB transformation

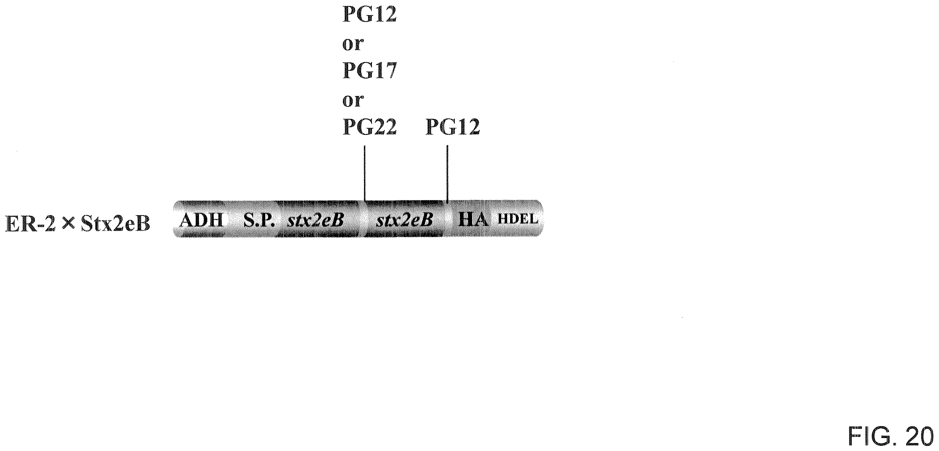

ER-2×Stx2eB (PG12) was prepared by the method in above <1> (1). ER-2×Stx2eB (PG17) and ER-2×Stx2eB (PG22) were prepared by the following method. The design of the DNA constructs is shown in FIG. 20.

A PG7-F primer (SEQ ID NO: 65) and a PG7-R primer (SEQ ID NO: 66) were annealed and phosphorylated with T4 PNK. The resulting phosphorylated DNA fragment was inserted into the BglII gap of ER-1×Stx2eB (PG12) obtained in <1> (1) (plasmid 14) .

A PG12-F primer (SEQ ID NO: 43) and a PG12-R primer (SEQ ID NO: 44) were annealed and phosphorylated with T4 PNK. The resulting phosphorylated DNA fragment was inserted into the BglII gap of ER-1×Stx2eB (PG12) (plasmid 15).

An Stx2eB-PG17 fragment was cut out from plasmid 14 using BamHI and BglII, and inserted into the BamHI gap of ER-1×Stx2eB (PG12) (2×Stx2eB (PG17)). An Stx2eB-PG22 fragment was cut out from plasmid 15 using BamHI and BglII, and inserted into the BamHI gap of ER-1×Stx2eB (PG12) (ER-2×Stx2eB (PG22)).

In order to produce Stx2eB using the stable transformant of the plant, each of the above DNA constructs for Stx2eB was subcloned into a vector for transformation. That is, each of ER-2×Stx2eB (PG12), ER-2×Stx2eB (PG17), and ER-2×Stx2eB (PG22) was inserted into pBI121 (Clontech) using XbaI and SacI, and allocated between the cauliflower mosaic virus 35S RNA promoter (35S pro.) and the nopaline synthetase gene transcription terminator (NOS-T).

### (2) Construction of vectors for CTB transformation

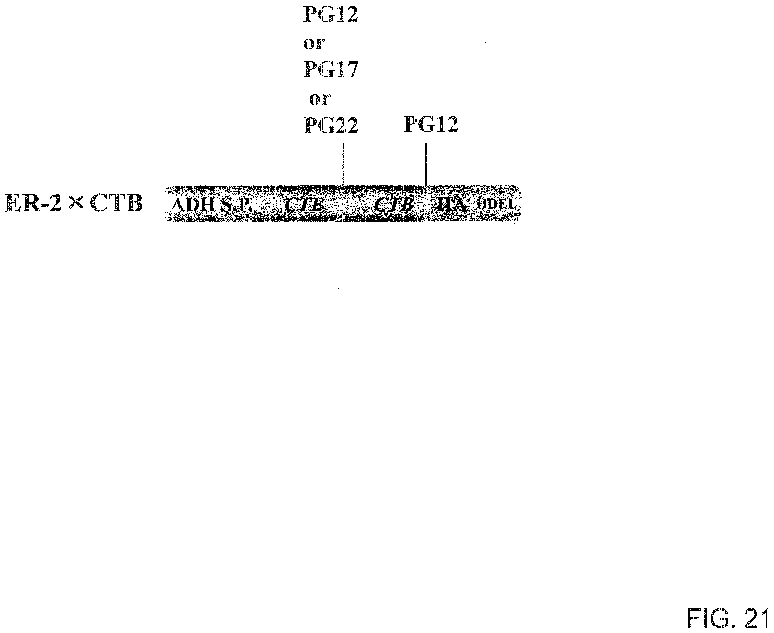

ER-2×CTB (PG12) was prepared by the method in above <1> (2). ER-2×CTB (PG17) and ER-2×CTB (PG22) were prepared by the following method. The design of the DNA constructs is shown in FIG. 21.

A PG7-F primer (SEQ ID NO: 65) and a PG7-R primer (SEQ ID NO: 66) were annealed and phosphorylated with T4 PNK. The resulting phosphorylated DNA fragment was inserted into the BglII gap of ER-1×CTB (PG12) obtained in <1> (2) (plasmid 16). A PG12-F primer (SEQ ID NO: 43) and a PG12-R primer (SEQ ID NO: 44) were annealed and phosphorylated with T4 PNK. The resulting phosphorylated DNA fragment was inserted into the BglII gap of ER-1×CTB (PG12) (plasmid 17).

A CTB-PG17 fragment was cut out from plasmid 16 using BamHI and BglII, and inserted into the BamHI gap of ER-1×CTB (PG12) (ER-2×CTB (PG17)). A CTB-PG22 fragment was cut out from plasmid 17 using BamHI and BglII, and inserted into the BamHI gap of ER-1×CTB (PG12) (ER-2×CTB (PG22)).

In order to produce CTB using the stable transformant of the plant, each of the above DNA constructs for CTB was subcloned into a vector for transformation. That is, each of ER-2×CTB (PG12), ER-2×CTB (PG17), and ER-2×CTB (PG22) was inserted into pBI121 (Clontech) using XbaI and SacI, and allocated between the cauliflower mosaic virus 35S RNA promoter (35S pro.) and the nopaline synthetase gene transcription terminator (NOS-T).

### (3) Transformation experiments and western analysis

Transformation experiments and western analysis were carried out by the methods in above <2> (2) and (3).

### (a) Effects of length of spacer on tandem linking of Stx2eB

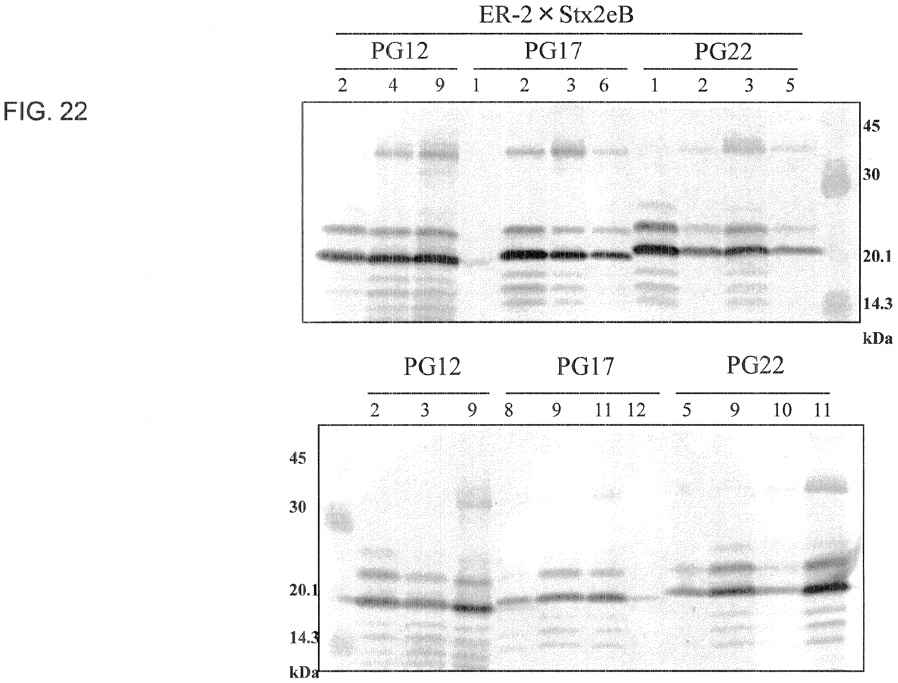

The results are shown in FIG. 22.

When one of ER-2×Stx2eB (PG17) and ER-2×Stx2eB (PG22) was expressed, a signal was detected at the similar level to that when ER-2×Stx2eB (PG12) was expressed. This indicates that any of PG17 and PG22 exhibits the same effect as PG12.

When ER-2×Stx2eB (PG12) was expressed, signals were detected at the positions of about 19 kDa and about 22 kDa. When ER-2×Stx2eB (PG17) was expressed, signals were detected at the positions of about 19 kDa and about 22 kDa. When ER-2×Stx2eB (PG22) was expressed, signals were detected at the positions of about 20 kDa and about 23 kDa. These corresponded to the molecular weights estimated from the design of the DNA constructs.

### (b) Effects of length of spacer on tandem linking of CTB

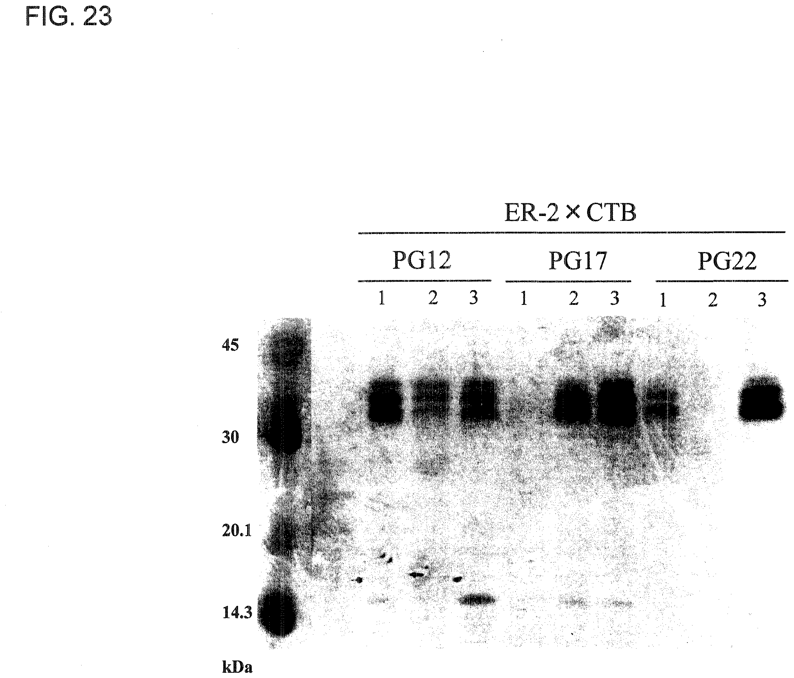

The results are shown in FIG. 23.

When one of ER-2×CTB (PG17) and ER-2×CTB (PG22) was expressed, a signal was detected at the similar level to that when ER-2×CTB (PG12) was expressed. This indicates that any of PG17 and PG22 exhibits the same effect as PG12.

When ER-2×CTB (PG12) was expressed, signals were detected at the positions of about 32 kDa, about 34 kDa, and about 36 kDa. When ER-2×CTB (PG17) was expressed, signals were detected at the positions of about 32 kDa, about 34 kDa, and about 36 kDa. When ER-2×CTB (PG22) was expressed, signals were detected at the positions of about 32 kDa, about 34 kDa, and about 36 kDa. These corresponded to the molecular weights estimated from the design of the DNA constructs.

### INDUSTRIAL APPLICABILITY

The hybrid protein of the present invention is highly stable and accumulated at a high level in plant cells. Besides, by producing the hybrid protein of the present invention in the plant using the DNA construct of the present invention, it is possible to efficiently produce oral vaccines for Shiga toxin, cholera toxin, and *Escherichia coli* heat-labile toxin.

The present invention enables to express a bacterial antigen in the plant at the level which is enough to induce immunity. The present invention enables to give the immunity against the bacterial antigen to the animal at low cost by giving a transgenic plant as food to the animal. For example, the present invention is useful for developing swine edema disease vaccine and cholera vaccine.

## Claims

1. A hybrid protein comprising
(a) two toxin proteins selected from the group consisting of Shiga toxin (Stx) protein, cholera toxin (CT) protein or *Escherichia coli* heat-labile toxin (LT) protein, wherein said toxin proteins are tandemly linked through a peptide having the amino acid sequence represented by SEQ ID NO: 2, 82 or 84, and
(b) a peptide having the amino acid sequence represented by SEQ ID NO: 2, 82 or 84 linked to the C-terminus of the tandemly linked toxin proteins of (a),
wherein the Stx protein is a STx2eB protein, wherein the CT protein is a CT protein B subunit, and wherein the LT protein is a LT protein B subunit.

2. The hybrid protein according to Claim 1, wherein said toxin proteins are tandemly linked through a peptide having the amino acid sequence represented by SEQ ID NO:2.

3. The hybrid protein according to Claim 1 or 2, comprising an amino acid sequence represented by SEQ ID NO: 14 or 16.

4. The hybrid protein according to Claim 1, comprising an amino acid sequence represented by SEQ ID NO: 90, 92, 98, or 100.

5. The hybrid protein according to any one of claims 1 to 4, wherein a secretory signal peptide derived from a plant is added to its amino terminus.

6. The hybrid protein according to anyone of claims 1 to 5, wherein an endoplasmic reticulum retention signal peptide is added to its carboxyl terminus.

7. A DNA construct comprising DNA encoding the hybrid protein according to any one of Claims 1 to 6.

8. The DNA construct according to Claim 7, comprising DNA having a base sequence represented by SEQ ID NO: 13, 15, 89, 91, 97, or 99.

9. The DNA construct according to Claim 7 or 8, wherein DNA encoding a hybrid protein is operably-linked to a 5'-untranslated region of an alcohol dehydrogenase gene derived from a plant.

10. The DNA construct according to Claim 9, wherein the 5'-untranslated region of an alcohol dehydrogenase gene derived from a plant is derived from *Nicotiana tabacum.*

11. The DNA construct according to Claim 10, comprising a base sequence represented by any one of SEQ ID NOS: 28, 29, 101, 102, 103, 104, 108, 109, 110, or 111.

12. A recombinant vector comprising the DNA construct according to any one of Claims 7 to 11.

13. A host cell transformed with the recombinant vector according to Claim 12.

14. The host cell according to Claim 13, wherein the transformant is a transformed plant cell or a transformed plant.

15. A seed, which is obtained from the transformant according to Claim 13 or 14.

16. A peptide having an amino acid sequence represented by one of SEQ ID NO: 2, 82, or 84.

## Patentansprüche

1. Hybridprotein umfassend
(a) zwei Toxin-Proteine, die ausgewählt sind aus der Gruppe bestehend aus Shiga-Toxin (Stx)-Protein, Cholera-Toxin (CT)-Protein oder hitzelabilem Toxin (LT)-Protein von *Escherichia coli,* wobei die Toxin-Proteine durch ein Peptid, das die durch SEQ ID NO:2, 82 oder 84 dargestellte Aminosäuresequenz aufweist, hintereinander verknüpft sind; und
(b) ein Peptid, das die durch SEQ ID NO:2, 82 oder 84 dargestellte Aminosäuresequenz aufweist, das mit dem C-Terminus der hintereinander verknüpften Toxin-Proteine aus (a) verknüpft ist,
wobei das Stx-Protein ein STx2eB-Protein ist, wobei das CT-Protein eine CT-Protein B-Untereinheit ist und wobei das LT-Protein eine LT-Protein B-Untereinheit ist.

2. Hybrid-Protein nach Anspruch 1, wobei die Toxin-Proteine durch ein Peptid hintereinander verknüpft sind, das die durch SEQ ID NO:2 dargestellte Aminosäuresequenz aufweist.

3. Hybrid-Protein nach Anspruch 1 oder 2, umfassend eine durch SEQ ID NO:14 oder 16 dargestellte Aminosäuresequenz.

4. Hybrid-Protein nach Anspruch 1, umfassend eine durch SEQ ID NO:90, 92, 98 oder 100 dargestellte Aminosäuresequenz.

5. Hybrid-Protein nach einem der Ansprüche 1 bis 4, wobei ein von einer Pflanze stammendes sekretorisches Signalpeptid an dessen Amino-Terminus angefügt ist.

6. Hybrid-Protein nach einem der Ansprüche 1 bis 5, wobei ein Signalpeptid zur Zurückhaltung im Endoplasmatischen Retikulum (endoplasmatic reticulum retention signal peptide) an dessen Carboxy-Terminus angefügt ist.

7. DNA-Konstrukt, umfassend DNA, die das Hybrid-Protein nach einem der Ansprüche 1 bis 6 codiert.

8. DNA-Konstrukt nach Anspruch 7, umfassend DNA, die eine Basensequenz aufweist, die durch die SEQ ID NO:13, 15, 89, 91, 97 oder 99 dargestellt ist.

9. DNA-Konstrukt nach Anspruch 7 oder 8, wobei die DNA, die ein Hybrid-Protein codiert, funktionell mit einer von einer Pflanze stammenden 5'-untranslatierten Region eines Alkoholdehydrogenase-Gens verknüpft ist.

10. DNA-Konstrukt nach Anspruch 9, wobei die von einer Pflanze stammende 5'-untranslatierte Region eines Alkoholdehydrogenase-Gens von *Nicotiana tabacum* stammt.

11. DNA-Konstrukt nach Anspruch 10, umfassend eine Basensequenz, die durch eine der SEQ ID NOs:28, 29, 101, 102, 103, 104, 108, 109, 110 oder 111 dargestellt ist.

12. Rekombinanter Vektor, umfassend das DNA-Konstrukt nach einem der Ansprüche 7 bis 11.

13. Transformante, die mit dem rekombinanten Vektor nach Anspruch 12 transformiert ist, wobei die Transformante eine eukaryotische oder prokaryotische Zelle ist.

14. Transformante nach Anspruch 13, wobei die Transformante eine transformierte Pflanzenzelle oder eine transformierte Pflanze ist.

15. Samen, der von der Transformanten nach Anspruch 13 oder 14 erhalten ist.

16. Peptid, das eine durch eine der SEQ ID NO:2, 82 oder 84 dargestellte Aminosäuresequenz aufweist.

## Revendications

1. Protéine hybride comprenant
(a) deux protéines de toxine sélectionnées dans le groupe consistant en une protéine de shigatoxine (Stx), une protéine de toxine cholérique (CT) ou une protéine de toxine thermolabile (LT) d*'Escherichia coli,* où lesdites protéines de toxine sont liées en tandem par un peptide possédant la séquence d'acides aminés représentée par SEQ ID NO: 2, 82 ou 84, et
(b) un peptide possédant la séquence d'acides aminés représentée par SEQ ID NO:2, 82 ou 84 lié à l'extrémité C-terminale des protéines de toxine liées en tandem de (a),
où la protéine Stx est une protéine STx2eB, où la protéine CT est la sous-unité B d'une protéine CT, et où la protéine LT est la sous-unité B d'une protéine LT.

2. Protéine hybride selon la revendication 1, dans laquelle lesdites protéines de toxine sont liées en tandem par un peptide possédant la séquence d'acides aminés représentée par SEQ ID NO:2.

3. Protéine hybride selon la revendication 1 ou 2, comprenant une séquence d'acides aminés représentée par SEQ ID NO:14 ou 16.

4. Protéine hybride selon la revendication 1, comprenant une séquence d'acides aminés représentée par SEQ ID NO:90, 92, 98, ou 100.

5. Protéine hybride selon l'une quelconque des revendications 1 à 4, dans laquelle un peptide signal de sécrétion dérivé d'une plante est ajouté à son extrémité amino-terminale.

6. Protéine hybride selon l'une quelconque des revendications 1 à 5, dans laquelle un peptide signal de rétention dans le réticulum endoplasmique est ajouté à son extrémité carboxy-terminale.

7. Produit de recombinaison d'ADN comprenant un ADN codant pour la protéine hybride selon l'une quelconque des revendications 1 à 6.

8. Produit de recombinaison d'ADN selon la revendication 7, comprenant un ADN possédant une séquence de bases représentée par SEQ ID NO:13, 15, 89, 91, 97, ou 99.

9. Produit de recombinaison d'ADN selon la revendication 7 ou 8, dans lequel un ADN codant pour une protéine hybride est en liaison fonctionnelle avec une région non traduite 5' d'un gène d'alcool déshydrogénase dérivé d'une plante.

10. Produit de recombinaison d'ADN selon la revendication 9, dans lequel la région non traduite 5' d'un gène d'alcool déshydrogénase dérivé d'une plante est dérivée de *Nicotiana tabacum.*

11. Produit de recombinaison d'ADN selon la revendication 10, comprenant une séquence de bases représentée par l'une quelconque de SEQ ID NO: 28, 29, 101, 102, 103, 104, 108, 109, 110, ou 111.

12. Vecteur recombinant comprenant le produit de recombinaison d'ADN selon l'une quelconque des revendications 7 à 11.

13. Transformant transformé avec le vecteur recombinant selon la revendication 12, où le transformant est une cellule eucaryote ou procaryote.

14. Transformant selon la revendication 13, où le transformant est une cellule végétale transformée ou une plante transformée.

15. Graine, qui est obtenue à partir du transformant selon la revendication 13 ou 14.

16. Peptide possédant une séquence d'acides aminés représentée par l'une de SEQ ID NO:2, 82, ou 84.


### SEQUENCE LISTING

<110> IDEMITSU KOSAN Co., Ltd.
<120> BACTERIAL TOXIN VACCINE
<130> S3062 EP S3
<150> JP 08/120573
   <151> 2008-05-02
<160> 114
<170> Patent In version 3.5
<210> 1
   <211> 36
   <212> DNA
   <213> Artificial
<220>
   <223> an artificially synthesized DNA
<400> 1
   agatcccctg gttctggtcc tggttctcct agatcc 36
<210> 2
   <211> 12
   <212> PRT
   <213> Artificial
<220>
   <223> an artificially synthesized peptide
<400> 2
   Arg Ser Pro Gly Ser Gly Pro Gly Ser Pro Arg Ser
   1               5                   10
<210> 3
   <211> 959
   <212> DNA
   <213> Escherichia coli
<400> 3
   atgaagtgta tattgttaaa gtggatactg tgtctgttac tgggtttttc ttcggtatcc      60
   tattcccagg agtttacgat agacttttcg actcaacaaa gttatgtatc ttcgttaaat     120
   agtatacgga cagcgatatc gacccctctt gaacatatat ctcagggagc tacatcggta     180
   tccgttatta atcatacacc accaggaagt tatatttccg taggtatacg agggcttgat     240
   gtttatcagg agcgttttga ccatcttcgt ctgattattg aacgaaataa tttatatgtg     300
   gctggatttg ttaatacgac aacaaatact ttctacagat tttcagattt gcacatatat     360
   cattgcccgg tgtgacaact atttccatga caacggacag cagttatacc actctgcaac     420
   gtgtcgcagc gctggaacgt tccggaatgc aaatcagtcg tcactcactg gtttcatcat     480
   atctggcgtt aatggagttc agtggtaata caatgaccag agatgcatca agagcagttc     540
   tgcgttttgt cactgtcaca gcagaagcct tacggttcag gcaaatacag agagaatttc     600
   gtctggcact gtctgaaact gctcctgttt atacgatgac gccggaagac gtggacctca     660
   ctctgaactg ggggagaatc agcaatgtgc ttccggagta tcggggagag gctggtgtca     720
   gagtggggag aatatccttt aataatatat cagcgatact tggtactgtg gccgttatac     780
   tgaattgcca tcatcagggc gcacgttctg ttcgcgccgt gaatgaagag agtcaaccag     840
   aatgtcagat aactggcgac aggcccgtta taaaaataaa caatacatta tgggaaagta     900
   atacagcagc agcgtttctg aacagaaagt cacagccttt atatacaact ggtgaatga      959
<210> 4
   <211> 304
   <212> PRT
   <213> Escherichia coli
<400> 4
   Met Lys Cys Ile Leu Leu Lys Trp Ile Leu Cys Leu Leu Leu Gly Phe
   1               5                   10                  15
   Ser Ser Val Ser Tyr Ser Gln Glu Phe Thr Ile Asp Phe Ser Thr Gln
               20                  25                  30
   Gln Ser Tyr Val Ser Ser Leu Asn Ser Ala Ile Ser Thr Pro Leu Glu
           35                  40                  45
   His Ile Ser Gln Gly Ala Thr Ser Val Ser Val Ile Asn His Thr Pro
       50                  55                  60
   Pro Gly Ser Tyr Ile Ser Val Gly Ile Arg Gly Leu Asp Val Tyr Gln
   65                  70                  75                  80
   Glu Arg Phe Asp His Leu Arg Leu Ile Ile Glu Arg Asn Asn Leu Tyr
                   85                  90                  95
   Phe Val Asn Thr Thr Thr Asn Thr Phe Tyr Arg Phe Ser Asp Phe Ala
               100                 105                 110
   His Ile Ser Leu Pro Gly Val Thr Thr Ile Ser Met Thr Thr Asp Ser
           115                 120                 125
   Ser Tyr Thr Thr Leu Gln Arg Val Ala Ala Leu Glu Arg Ser Gly Met
       130                 135                 140
   Gln Ile Ser Arg His Ser Leu Tyr Leu Ala Leu Met Glu Phe Ser Gly
   145                 150                 155                 160
   Asn Thr Met Thr Arg Asp Ala Ser Arg Ala Val Leu Arg Phe Val Thr
                   165                 170                 175
   Val Thr Ala Glu Ala Leu Arg Phe Arg Gln Ile Gln Arg Glu Phe Arg
               180                 185                 190
   Leu Ala Leu Ser Glu Thr Ala Pro Val Tyr Thr Met Thr Pro Asp Leu
           195                 200                 205
   Thr Leu Asn Trp Gly Arg Ile Ser Asn Val Leu Pro Glu Tyr Arg Gly
       210                 215                 220
   Glu Ala Gly Val Arg Val Gly Arg Ile Ser Phe Asn Asn Ile Ser Ala
   225                 230                 235                 240
   Ile Leu Gly Thr Val Ala Val Ile Leu Asn Cys His His Gln Gly Ala
                   245                 250                 255
   Arg Ser Val Arg Ala Glu Ser Gln Pro Glu Cys Gln Ile Thr Gly Asp
               260                 265                 270
   Arg Pro Val Ile Lys Ile Asn Asn Thr Leu Trp Glu Ser Asn Thr Ala
           275                 280                 285
   Ala Ala Phe Leu Asn Arg Lys Ser Gln Pro Leu Tyr Thr Thr Gly Glu
       290                 295                 300
<210> 5
   <211> 210
   <212> DNA
   <213> Escherichia coli
<400> 5
   gcggcggatt gtgctaaagg taaaattgag ttttccaagt ataatgagga taataccttt      60
   actgtgaagg tgtcaggaag agaatactgg acgaacagat ggaatttgca gccattgtta     120
   caaagtgctc agctgacagg gatgactgta acaatcatat ctaatacctg cagttcaggc     180
   tcaggctttg cccaggtgaa gtttaactga                                      210
<210> 6
   <211> 69
   <212> PRT
   <213> Escherichia coli
<400> 6
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn
   65
<210> 7
   <211> 312
   <212> DNA
   <213> Vibrio cholerae
<400> 7
   accccccaga acatcaccga cctctgcgcc gagagccaca acacccaaat ctacaccctc     60
   aacgacaaga ttttcagcta caccgagagc ctcgccggca agagggagat ggccatcatc    120
   accttcaaga acggcgccat cttccaggtc gaggtccccg gcagccagca catcgacagc    180
   cagaagaagg ccatcgagag gatgaaggac accctcagga tcgcctacct caccgaggcc    240
   aaggtcgaga agctctgcgt ctggaacaac aagacccccc acgccatcgc cgccatcagc    300
   atggccaact ga                                                        312
<210> 8
   <211> 103
   <212> PRT
   <213> Vibrio cholerae
<400> 8
   Thr Pro Gln Asn Ile Thr Asp Leu Cys Ala Glu Ser His Asn Thr Gln
   1               5                   10                  15
   Ile Tyr Thr Leu Asn Asp Lys Ile Phe Ser Tyr Thr Glu Ser Leu Ala
               20                  25                  30
   Gly Lys Arg Glu Met Ala Ile Ile Thr Phe Lys Asn Gly Ala Ile Phe
           35                  40                  45
   Gln Val Glu Val Pro Gly Ser Gln His Ile Asp Ser Gln Lys Lys Ala
       50                  55                  60
   Ile Glu Arg Met Lys Asp Thr Leu Arg Ile Ala Tyr Leu Thr Glu Ala
   65                  70                  75                  80
   Lys Val Glu Lys Leu Cys Val Trp Asn Asn Lys Thr Pro His Ala Ile
                   85                  90                  95
   Ala Ala Ile Ser Met Ala Asn
               100
<210> 9
   <211> 453
   <212> DNA
   <213> Artificial
<220>
   <223> an artificially synthesized DNA construct
<400> 9
   gcggcggatt gtgctaaagg taaaattgag ttttccaagt ataatgagga taataccttt      60
   actgtgaagg tgtcaggaag agaatactgg acgaacagat ggaatttgca gccattgtta     120
   caaagtgctc agctgacagg gatgactgta acaatcatat ctaatacctg cagttcaggc     180
   tcaggctttg cccaggtgaa gtttaacaga tcccctggtt ctggtcctgg ttctcctaga     240
   tccgcggcgg attgtgctaa aggtaaaatt gagttttcca agtataatga ggataatacc     300
   tttactgtga aggtgtcagg aagagaatac tggacgaaca gatggaattt gcagccattg     360
   ttacaaagtg ctcagctgac agggatgact gtaacaatca tatctaatac ctgcagttca     420
   ggctcaggct ttgcccaggt gaagtttaac tga                                  453
<210> 10
   <211> 150
   <212> PRT
   <213> Artificial
<220>
   <223> an artificially sinthesized protein
<400> 10
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn Arg Ser Pro Gly Ser Gly Pro Gly Ser Pro Arg
   65                  70                  75                  80
   Ser Ala Ala Asp Cys Ala Lys Gly Lys Ile Glu Phe Ser Lys Tyr Asn
                   85                  90                  95
   Glu Asp Asn Thr Phe Thr Val Lys Val Ser Gly Arg Glu Tyr Trp Thr
               100                 105                 110
   Asn Arg Trp Asn Leu Gln Pro Leu Leu Gln Ser Ala Gln Leu Thr Gly
           115                 120                 125
   Met Thr Val Thr Ile Ile Ser Asn Thr Cys Ser Ser Gly Ser Gly Phe
       130                 135                 140
   Ala Gln Val Lys Phe Asn
                       145                 150
<210> 11
   <211> 657
   <212> DNA
   <213> Artificial
<220>
   <223> an artificially sinthesized DNA construct
<400> 11
   accccccaga acatcaccga cctctgcgcc gagagccaca acacccaaat ctacaccctc      60
   aacgacaaga ttttcagcta caccgagagc ctcgccggca agagggagat ggccatcatc     120
   accttcaaga acggcgccat cttccaggtc gaggtccccg gcagccagca catcgacagc     180
   cagaagaagg ccatcgagag gatgaaggac accctcagga tcgcctacct caccgaggcc     240
   aaggtcgaga agctctgcgt ctggaacaac aagacccccc acgccatcgc cgccatcagc     300
   atggccaaca gatcccctgg ttctggtcct ggttctccta gatccacccc ccagaacatc     360
   accgacctct gcgccgagag ccacaacacc caaatctaca ccctcaacga caagattttc     420
   agctacaccg agagcctcgc cggcaagagg gagatggcca tcatcacctt caagaacggc     480
   gccatcttcc aggtcgaggt ccccggcagc cagcacatcg acagccagaa gaaggccatc     540
   gagaggatga aggacaccct caggatcgcc tacctcaccg aggccaaggt cgagaagctc     600
   tgcgtctgga acaacaagac cccccacgcc atcgccgcca tcagcatggc caactga        657
<210> 12
   <211> 218
   <212> PRT
   <213> Artificial
<220>
   <223> an artificially sinthesized protein
<400> 12
   Thr Pro Gln Asn Ile Thr Asp Leu Cys Ala Glu Ser His Asn Thr Gln
   1               5                   10                  15
   Ile Tyr Thr Leu Asn Asp Lys Ile Phe Ser Tyr Thr Glu Ser Leu Ala
               20                  25                  30
   Gly Lys Arg Glu Met Ala Ile Ile Thr Phe Lys Asn Gly Ala Ile Phe
           35                  40                  45
   Gln Val Glu Val Pro Gly Ser Gln His Ile Asp Ser Gln Lys Lys Ala
       50                  55                  60
   Ile Glu Arg Met Lys Asp Thr Leu Arg Ile Ala Tyr Leu Thr Glu Ala
   65                  70                  75                  80
   Lys Val Glu Lys Leu Cys Val Trp Asn Asn Lys Thr Pro His Ala Ile
                   85                  90                  95
   Ala Ala Ile Ser Met Ala Asn Arg Ser Pro Gly Ser Gly Pro Gly Ser
               100                 105                 110
   Pro Arg Ser Thr Pro Gln Asn Ile Thr Asp Leu Cys Ala Glu Ser His
           115                 120                 125
   Asn Thr Gln Ile Tyr Thr Leu Asn Asp Lys Ile Phe Ser Tyr Thr Glu
       130                 135                 140
   Ser Leu Ala Gly Lys Arg Glu Met Ala Ile Ile Thr Phe Lys Asn Gly
   145                 150                 155                 160
   Ala Ile Phe Gln Val Glu Val Pro Gly Ser Gln His Ile Asp Ser Gln
                   165                 170                 175
   Lys Lys Ala Ile Glu Arg Met Lys Asp Thr Leu Arg Ile Ala Tyr Leu
               180                 185                 190
   Thr Glu Ala Lys Val Glu Lys Leu Cys Val Trp Asn Asn Lys Thr Pro
           195                 200                 205
   His Ala Ile Ala Ala Ile Ser Met Ala Asn
       210                 215
<210> 13
   <211> 489
   <212> DNA
   <213> Artificial
<220>
   <223> an artificially sinthesized DNA construct
<400> 13
   gcggcggatt gtgctaaagg taaaattgag ttttccaagt ataatgagga taataccttt      60
   actgtgaagg tgtcaggaag agaatactgg acgaacagat ggaatttgca gccattgtta     120
   caaagtgctc agctgacagg gatgactgta acaatcatat ctaatacctg cagttcaggc     180
   tcaggctttg cccaggtgaa gtttaacaga tcccctggtt ctggtcctgg ttctcctaga     240
   tccgcggcgg attgtgctaa aggtaaaatt gagttttcca agtataatga ggataatacc     300
   tttactgtga aggtgtcagg aagagaatac tggacgaaca gatggaattt gcagccattg     360
   ttacaaagtg ctcagctgac agggatgact gtaacaatca tatctaatac ctgcagttca     420
   ggctcaggct ttgcccaggt gaagtttaac agatcccctg gttctggtcc tggttctcct     480
   agatcttga                                                             489
<210> 14
   <211> 162
   <212> PRT
   <213> Artificial
<220>
   <223> an artificially sinthesized protein
<400> 14
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn Arg Ser Pro Gly Ser Gly Pro Gly Ser Pro Arg
   65                  70                  75                  80
   Ser Ala Ala Asp Cys Ala Lys Gly Lys Ile Glu Phe Ser Lys Tyr Asn
                   85                  90                  95
   Glu Asp Asn Thr Phe Thr Val Lys Val Ser Gly Arg Glu Tyr Trp Thr
               100                 105                 110
   Asn Arg Trp Asn Leu Gln Pro Leu Leu Gln Ser Ala Gln Leu Thr Gly
           115                 120                 125
   Met Thr Val Thr Ile Ile Ser Asn Thr Cys Ser Ser Gly Ser Gly Phe
       130                 135                 140
   Ala Gln Val Lys Phe Asn Arg Ser Pro Gly Ser Gly Pro Gly Ser Pro
   145                 150                 155                 160
   Arg Ser
<210> 15
   <211> 693
   <212> DNA
   <213> Artificial
<220>
   <223> an artificially sinthesized DNA construct
<400> 15
   accccccaga acatcaccga cctctgcgcc gagagccaca acacccaaat ctacaccctc     60
   aacgacaaga ttttcagcta caccgagagc ctcgccggca agagggagat ggccatcatc    120
   accttcaaga acggcgccat cttccaggtc gaggtccccg gcagccagca catcgacagc    180
   cagaagaagg ccatcgagag gatgaaggac accctcagga tcgcctacct caccgaggcc    240
   aaggtcgaga agctctgcgt ctggaacaac aagacccccc acgccatcgc cgccatcagc    300
   atggccaaca gatcccctgg ttctggtcct ggttctccta gatccacccc ccagaacatc    360
   accgacctct gcgccgagag ccacaacacc caaatctaca ccctcaacga caagattttc    420
   agctacaccg agagcctcgc cggcaagagg gagatggcca tcatcacctt caagaacggc    480
   gccatcttcc aggtcgaggt ccccggcagc cagcacatcg acagccagaa gaaggccatc    540
   gagaggatga aggacaccct caggatcgcc tacctcaccg aggccaaggt cgagaagctc    600
   tgcgtctgga acaacaagac cccccacgcc atcgccgcca tcagcatggc caacagatcc    660
   cctggttctg gtcctggttc tcctagatct tga                                 693
<210> 16
   <211> 230
   <212> PRT
   <213> Artificial
<220>
   <223> an artificially sinthesized protein
<400> 16
   Thr Pro Gln Asn Ile Thr Asp Leu Cys Ala Glu Ser His Asn Thr Gln
   1               5                   10                  15
   Ile Tyr Thr Leu Asn Asp Lys Ile Phe Ser Tyr Thr Glu Ser Leu Ala
               20                  25                  30
   Gly Lys Arg Glu Met Ala Ile Ile Thr Phe Lys Asn Gly Ala Ile Phe
           35                  40                  45
   Gln Val Glu Val Pro Gly Ser Gln His Ile Asp Ser Gln Lys Lys Ala
       50                  55                  60
   Ile Glu Arg Met Lys Asp Thr Leu Arg Ile Ala Tyr Leu Thr Glu Ala
   65                  70                  75                  80
   Lys Val Glu Lys Leu Cys Val Trp Asn Asn Lys Thr Pro His Ala Ile
                   85                  90                  95
   Ala Ala Ile Ser Met Ala Asn Arg Ser Pro Gly Ser Gly Pro Gly Ser
               100                 105                 110
   Pro Arg Ser Thr Pro Gln Asn Ile Thr Asp Leu Cys Ala Glu Ser His
           115                 120                 125
   Asn Thr Gln Ile Tyr Thr Leu Asn Asp Lys Ile Phe Ser Tyr Thr Glu
       130                 135                 140
   Ser Leu Ala Gly Lys Arg Glu Met Ala Ile Ile Thr Phe Lys Asn Gly
   145                 150                 155                 160
   Ala Ile Phe Gln Val Glu Val Pro Gly Ser Gln His Ile Asp Ser Gln
                   165                 170                 175
   Lys Lys Ala Ile Glu Arg Met Lys Asp Thr Leu Arg Ile Ala Tyr Leu
               180                 185                 190
   Thr Glu Ala Lys Val Glu Lys Leu Cys Val Trp Asn Asn Lys Thr Pro
           195                 200                 205
   His Ala Ile Ala Ala Ile Ser Met Ala Asn Arg Ser Pro Gly Ser Gly
       210                 215                 220
   Pro Gly Ser Pro Arg Ser
   225                 230
<210> 17
   <211> 72
   <212> DNA
   <213> Nicotiana tabacum
<400> 17
   atggggagaa tgtcaatacc catgatgggt tttgtggtgt tatgtctatg ggcagtggta      60
   gcagaaggat cc                                                          72
<210> 18
   <211> 24
   <212> PRT
   <213> Nicotiana tabacum
<400> 18
   Met Gly Arg Met Ser Ile Pro Met Met Gly Phe Val Val Leu Cys Leu
   1               5                   10                  15
   Trp Ala Val Val Ala Glu Gly Ser
               20
<210> 19
   <211> 4
   <212> PRT
   <213> Artificial
<220>
   <223> an artificially sinthesized peptide
<400> 19
   Lys Asp Glu Leu
   1
<210> 20
   <211> 4
   <212> PRT
   <213> Artificial
<220>
   <223> an artificially sinthesized peptide
<400> 20
   His Asp Glu Leu
   1
<210> 21
   <211> 4
   <212> PRT
   <213> Artificial
<220>
   <223> an artificially sinthesized peptide
<400> 21
   Lys Asp Glu Phe
   1
<210> 22
   <211> 4
   <212> PRT
   <213> Artificial
<220>
   <223> an artificially sinthesized peptide
<400> 22
   His Asp Glu Phe
   1
<210> 23
   <211> 91
   <212> DNA
   <213> Nicotiana tabacum
<400> 23
   tatttaactc agtattcaga aacaacaaaa gttcttctct acataaaatt ttcctatttt    60
   agtgatcagt gaaggaaatc aagaaaaata a                                   91
<210> 24
   <211> 547
   <212> DNA
   <213> Artificial
<220>
   <223> an artificially sinthesized DNA construct
<400> 24
   tatttaactc agtattcaga aacaacaaaa gttcttctct acataaaatt ttcctatttt    60
   agtgatcagt gaaggaaatc aagaaaaata aatggcggcg gattgtgcta aaggtaaaat   120
   tgagttttcc aagtataatg aggataatac ctttactgtg aaggtgtcag gaagagaata   180
   ctggacgaac agatggaatt tgcagccatt gttacaaagt gctcagctga cagggatgac   240
   tgtaacaatc atatctaata cctgcagttc aggctcaggc tttgcccagg tgaagtttaa   300
   cagatcccct ggttctggtc ctggttctcc tagatccgcg gcggattgtg ctaaaggtaa   360
   aattgagttt tccaagtata atgaggataa tacctttact gtgaaggtgt caggaagaga   420
   atactggacg aacagatgga atttgcagcc attgttacaa agtgctcagc tgacagggat   480
   gactgtaaca atcatatcta atacctgcag ttcaggctca ggctttgccc aggtgaagtt   540
   taactga                                                              547
<210> 25
   <211> 751
   <212> DNA
   <213> Artificial
<220>
   <223> an artificially sinthesized DNA construct
<400> 25
   tatttaactc agtattcaga aacaacaaaa gttcttctct acataaaatt ttcctatttt    60
   agtgatcagt gaaggaaatc aagaaaaata aatgaccccc cagaacatca ccgacctctg   120
   cgccgagagc cacaacaccc aaatctacac cctcaacgac aagattttca gctacaccga   180
   gagcctcgcc ggcaagaggg agatggccat catcaccttc aagaacggcg ccatcttcca   240
   ggtcgaggtc cccggcagcc agcacatcga cagccagaag aaggccatcg agaggatgaa   300
   ggacaccctc aggatcgcct acctcaccga ggccaaggtc gagaagctct gcgtctggaa   360
   caacaagacc ccccacgcca tcgccgccat cagcatggcc aacagatccc ctggttctgg   420
   tcctggttct cctagatcca ccccccagaa catcaccgac ctctgcgccg agagccacaa   480
   cacccaaatc tacaccctca acgacaagat tttcagctac accgagagcc tcgccggcaa   540
   gagggagatg gccatcatca ccttcaagaa cggcgccatc ttccaggtcg aggtccccgg   600
   cagccagcac atcgacagcc agaagaaggc catcgagagg atgaaggaca ccctcaggat   660
   cgcctacctc accgaggcca aggtcgagaa gctctgcgtc tggaacaaca agacccccca   720
   cgccatcgcc gccatcagca tggccaactg a                                  751
<210> 26
   <211> 637
   <212> DNA
   <213> Artificial
<220>
   <223> an artificially sintesized DNA construct
<400> 26
   tatttaactc agtattcaga aacaacaaaa gttcttctct acataaaatt ttcctatttt    60
   agtgatcagt gaaggaaatc aagaaaaata aatggggaga atgtcaatac ccatgatggg   120
   ttttgtggtg ttatgtctat gggcagtggt agcagaagga tccgcggcgg attgtgctaa   180
   aggtaaaatt gagttttcca agtataatga ggataatacc tttactgtga aggtgtcagg   240
   aagagaatac tggacgaaca gatggaattt gcagccattg ttacaaagtg ctcagctgac   300
   agggatgact gtaacaatca tatctaatac ctgcagttca ggctcaggct ttgcccaggt   360
   gaagtttaac agatcccctg gttctggtcc tggttctcct agatccgcgg cggattgtgc   420
   taaaggtaaa attgagtttt ccaagtataa tgaggataat acctttactg tgaaggtgtc   480
   aggaagagaa tactggacga acagatggaa tttgcagcca ttgttacaaa gtgctcagct   540
   gacagggatg actgtaacaa tcatatctaa tacctgcagt tcaggctcag gctttgccca   600
   ggtgaagttt aacagatctg aacatgatga attgtga                            637
<210> 27
   <211> 841
   <212> DNA
   <213> Artificial
<220>
   <223> an artificially sinthesized DNA construct
<400> 27
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccacccccc agaacatcac    180
   cgacctctgc gccgagagcc acaacaccca aatctacacc ctcaacgaca agattttcag    240
   ctacaccgag agcctcgccg gcaagaggga gatggccatc atcaccttca agaacggcgc    300
   catcttccag gtcgaggtcc ccggcagcca gcacatcgac agccagaaga aggccatcga    360
   gaggatgaag gacaccctca ggatcgccta cctcaccgag gccaaggtcg agaagctctg    420
   cgtctggaac aacaagaccc cccacgccat cgccgccatc agcatggcca acagatcccc    480
   tggttctggt cctggttctc ctagatccac cccccagaac atcaccgacc tctgcgccga    540
   gagccacaac acccaaatct acaccctcaa cgacaagatt ttcagctaca ccgagagcct    600
   cgccggcaag agggagatgg ccatcatcac cttcaagaac ggcgccatct tccaggtcga    660
   ggtccccggc agccagcaca tcgacagcca gaagaaggcc atcgagagga tgaaggacac    720
   cctcaggatc gcctacctca ccgaggccaa ggtcgagaag ctctgcgtct ggaacaacaa    780
   gaccccccac gccatcgccg ccatcagcat ggccaacaga tctgaacatg atgaattgtg    840
   a                                                                    841
<210> 28
   <211> 667
   <212> DNA
   <213> Artificial
<220>
   <223> an artificially sinthesized DNA construct
<400> 28
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccgcggcgg attgtgctaa    180
   aggtaaaatt gagttttcca agtataatga ggataatacc tttactgtga aggtgtcagg    240
   aagagaatac tggacgaaca gatggaattt gcagccattg ttacaaagtg ctcagctgac    300
   agggatgact gtaacaatca tatctaatac ctgcagttca ggctcaggct ttgcccaggt    360
   gaagtttaac agatcccctg gttctggtcc tggttctcct agatccgcgg cggattgtgc    420
   taaaggtaaa attgagtttt ccaagtataa tgaggataat acctttactg tgaaggtgtc    480
   aggaagagaa tactggacga acagatggaa tttgcagcca ttgttacaaa gtgctcagct    540
   gacagggatg actgtaacaa tcatatctaa tacctgcagt tcaggctcag gctttgccca    600
   ggtgaagttt aacagatccc ctggttctgg tcctggttct cctagatctg aacatgatga    660
   attgtga                                                              667
<210> 29
   <211> 871
   <212> DNA
   <213> Artificial
<220>
   <223> an artificially sinthesized DNA construct
<400> 29
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccacccccc agaacatcac    180
   cgacctctgc gccgagagcc acaacaccca aatctacacc ctcaacgaca agattttcag    240
   ctacaccgag agcctcgccg gcaagaggga gatggccatc atcaccttca agaacggcgc    300
   catcttccag gtcgaggtcc ccggcagcca gcacatcgac agccagaaga aggccatcga    360
   gaggatgaag gacaccctca ggatcgccta cctcaccgag gccaaggtcg agaagctctg    420
   cgtctggaac aacaagaccc cccacgccat cgccgccatc agcatggcca acagatcccc    480
   tggttctggt cctggttctc ctagatccac cccccagaac atcaccgacc tctgcgccga    540
   gagccacaac acccaaatct acaccctcaa cgacaagatt ttcagctaca ccgagagcct    600
   cgccggcaag agggagatgg ccatcatcac cttcaagaac ggcgccatct tccaggtcga    660
   ggtccccggc agccagcaca tcgacagcca gaagaaggcc atcgagagga tgaaggacac    720
   cctcaggatc gcctacctca ccgaggccaa ggtcgagaag ctctgcgtct ggaacaacaa    780
   gacccccacc gccatcgccg ccatcagcat ggccaacaga tcccctggtt ctggtcctgg    840
   ttctcctaga tctgaacatg atgaattgtg a                                   871
<210> 30
   <211> 38
   <212> DNA
   <213> Artificial
<220>
   <223> an artificially sinthesized primer
<400> 30
   tatctagagc caccatggga tccgcggcgg attgtgct 38
<210> 31
   <211> 30
   <212> DNA
   <213> Artificial
<220>
   <223> an artificially sinthesized primer
<400> 31
   ttcaagatct gttaaacttc acctgggcaa 30
<210> 32
   <211> 23
   <212> DNA
   <213> Artificial
<220>
   <223> an artificially sinthesized primer
<400> 32
   gtcgacggta cccccgggga gct 23
<210> 33
   <211> 23
   <212> DNA
   <213> Artificial
<220>
   <223> an artificially sinthesized primer
<400> 33
   ccccgggggt accgtcgaca gct 23
<210> 34
   <211> 41
   <212> DNA
   <213> Artificial
<220>
   <223> an artificially sinthesized primer
<400> 34
   aatctagagt ctatttaact cagtattcag aaacaacaaa a 41
<210> 35
   <211> 30
   <212> DNA
   <213> Artificial
<220>
   <223> an artificially sinthesized primer
<400> 35
   aaatgcatta tttttcttga tttccttcac 30
<210> 36
   <211> 30
   <212> DNA
   <213> Artificial
<220>
   <223> an artificially sinthesized primer
<400> 36
   aaatgcatgg ggagaatgtc aatacccatg 30
<210> 37
   <211> 30
   <212> DNA
   <213> Artificial
<220>
   <223> an artificially sinthesized primer
<400> 37
   tataggatcc cattattttt cttgatttcc 30
<210> 38
   <211> 7
   <212> PRT
   <213> Artificial
<220>
   <223> an artificially sinthesized peptide
<400> 38
   Gly Ser Glu His Asp Glu Leu
   1               5
<210> 39
   <211> 21
   <212> DNA
   <213> Artificial
<220>
   <223> an artificially sinthesized primer
<400> 39
   gatctgaaca tgatgaattg t 21
<210> 40
   <211> 21
   <212> DNA
   <213> Artificial
<220>
   <223> an artificially sinthesized primer
<400> 40
   gatcacaatt catcatgttc a 21
<210> 41
   <211> 33
   <212> DNA
   <213> Artificial
<220>
   <223> an artificially sinthesized primer
<400> 41
   gatcttatcc ttatgattat cctgattatg ctg 33
<210> 42
   <211> 33
   <212> DNA
   <213> Artificial
<220>
   <223> an artificially sinthesized primer
<400> 42
   gatccagcat aatcaggata atcataagga taa 33
<210> 43
   <211> 30
   <212> DNA
   <213> Artificial
<220>
   <223> an artificially sinthesized primer
<400> 43
   gatcccctgg ttctggtcct ggttctccta 30
<210> 44
   <211> 30
   <212> DNA
   <213> Artificial
<220>
   <223> an artificially sinthesized primer
<400> 44
   gatctaggag aaccaggacc agaaccaggg 30
<210> 45
   <211> 40
   <212> DNA
   <213> Artificial
<220>
   <223> an artificially sinthesized primer
<400> 45
   ttggatccac cccccagaac atcaccgacc tctgcgccga 40
<210> 46
   <211> 39
   <212> DNA
   <213> Artificial
<220>
   <223> an artificially sinthesized primer
<400> 46
   cgttgagggt gtagatttgg gtgttgtggc tctcggcgc 39
<210> 47
   <211> 40
   <212> DNA
   <213> Artificial
<220>
   <223> an artificially sinthesized primer
<400> 47
   ccctcaacga caagattttc agctacaccg agagcctcgc 40
<210> 48
   <211> 40
   <212> DNA
   <213> Artificial
<220>
   <223> an artificially sinthesized primer
<400> 48
   cttgaaggtg atgatggcca tctccctctt gccggcgagg 40
<210> 49
   <211> 32
   <212> DNA
   <213> Artificial
<220>
   <223> an artificially sinthesized primer
<400> 49
   caccttcaag aacggcgcca tcttccaggt cg 32
<210> 50
   <211> 45
   <212> DNA
   <213> Artificial
<220>
   <223> an artificially sinthesized primer
<400> 50
   cttctggctg tcgatgtgct ggctgccggg gacctcgacc tggaa 45
<210> 51
   <211> 45
   <212> DNA
   <213> Artificial
<220>
   <223> an artificially sinthesized primer
<400> 51
   agccagaaga aggccatcga gaggatgaag gacaccctca ggatc 45
<210> 52
   <211> 43
   <212> DNA
   <213> Artificial
<220>
   <223> an artificially sinthesized primer
<400> 52
   gcagagcttc tcgaccttgg cctcggtgag gtaggcgatc ctg 43
<210> 53
   <211> 30
   <212> DNA
   <213> Artificial
<220>
   <223> an artificially sinthesized primer
<400> 53
   aagctctgcg tctggaacaa caagaccccc 30
<210> 54
   <211> 45
   <212> DNA
   <213> Artificial
<220>
   <223> an artificially sinthesized primer
<400> 54
   aaagatctgt tggccatgct gatggcggcg atggcgtggg gggtc 45
<210> 55
   <211> 31
   <212> DNA
   <213> Artificial
<220>
   <223> an artificially primer
<400> 55
   tttggatcca gcaagggcga ggagctgttc a 31
<210> 56
   <211> 33
   <212> DNA
   <213> Artificial
<220>
   <223> an artificially sinthesized primer
<400> 56
   tttagatctc ttgtacagct cgtccatgcc gag 33
<210> 57
   <211> 30
   <212> DNA
   <213> Artificial
<220>
   <223> an artificially sinthesized primer
<400> 57
   aaaggatccg cctcctccga ggacgtcatc 30
<210> 58
   <211> 30
   <212> DNA
   <213> Artificial
<220>
   <223> an artificially sinthesized primer
<400> 58
   aaaagatctg gcgccggtgg agtggcggcc 30
<210> 59
   <211> 30
   <212> DNA
   <213> Artificial
<220>
   <223> an artificially sinthesized primer
<400> 59
   gatcaaaatg gggttgtcat tcggaaagtt 30
<210> 60
   <211> 30
   <212> DNA
   <213> Artificial
<220>
   <223> an artificially sinthesized primer
<400> 60
   attccatcta tgccttgctt gcgatgttgt 30
<210> 61
   <211> 24
   <212> DNA
   <213> Artificial
<220>
   <223> an artificially sinthesized primer
<400> 61
   ctagacaaga tccgtccatt gtgg 24
<210> 62
   <211> 24
   <212> DNA
   <213> Artificial
<220>
   <223> an artificially sinthesized primer
<400> 62
   acccccaaca tcccacacgg tgaa 24
<210> 63
   <211> 7
   <212> PRT
   <213> Artificial
<220>
   <223> an artificially sinthesized peptide
<400> 63
   Arg Ser Pro Gly Ser Arg Ser
   1 5
<210> 64
   <211> 12
   <212> PRT
   <213> Artificial
<220>
   <223> an artificially sinthesized peptide
<400> 64
   Arg Ser Gly Ser Gly Ser Gly Ser Gly Ser Arg Ser
   1 5 10
<210> 65
   <211> 15
   <212> DNA
   <213> Artificial
<220>
   <223> an artificially sinthesized primer
<400> 65
   gatcccctgg ttcca 15
<210> 66
   <211> 15
   <212> DNA
   <213> Artificial
<220>
   <223> an artificially sinthesized primer
<400> 66
   gatctggaac caggg 15
<210> 67
   <211> 30
   <212> DNA
   <213> Artificial
<220>
   <223> an artificially sinthesized primer
<400> 67
   gatccggttc tggttctggt tctggttcca 30
<210> 68
   <211> 30
   <212> DNA
   <213> Artificial
<220>
   <223> an artificially sinthesized primer
<400> 68
   gatctggaac cagaaccaga accagaaccg 30
<210> 69
   <211> 25
   <212> DNA
   <213> Artificial
<220>
   <223> an artificially sinthesized primer
<400> 69
   gtgatcagtg aaggaaatca agaaa 25
<210> 70
   <211> 23
   <212> DNA
   <213> Artificial
<220>
   <223> an artificially sinthesized primer
<400> 70
   cataacacca caaaacccat cat 23
<210> 71
   <211> 22
   <212> DNA
   <213> Artificial
<220>
   <223> an artificially sinthesized primer
<400> 71
   ccaagccaaa gaagatcaag ca 22
<210> 72
   <211> 24
   <212> DNA
   <213> Artificial
<220>
   <223> an artificially sinthesized primer
<400> 72
   ccctgaatca tcgaccttgt agaa 24
<210> 73
   <211> 700
   <212> DNA
   <213> Artificial
<220>
   <223> an artificially sinthesized DNA construct
<400> 73
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccgcggcgg attgtgctaa    180
   aggtaaaatt gagttttcca agtataatga ggataatacc tttactgtga aggtgtcagg    240
   aagagaatac tggacgaaca gatggaattt gcagccattg ttacaaagtg ctcagctgac    300
   agggatgact gtaacaatca tatctaatac ctgcagttca ggctcaggct ttgcccaggt    360
   gaagtttaac agatcccctg gttctggtcc tggttctcct agatccgcgg cggattgtgc    420
   taaaggtaaa attgagtttt ccaagtataa tgaggataat acctttactg tgaaggtgtc    480
   aggaagagaa tactggacga acagatggaa tttgcagcca ttgttacaaa gtgctcagct    540
   gacagggatg actgtaacaa tcatatctaa tacctgcagt tcaggctcag gctttgccca    600
   ggtgaagttt aacagatccc ctggttctgg tcctggttct cctagatctt atccttatga    660
   ttatcctgat tatgctggat ctgaacatga tgaattgtga                          700
<210> 74
   <211> 904
   <212> DNA
   <213> Artificial
<220>
   <223> an artificially sinthesized DNA construct
<400> 74
   tatttaactc agtattcaga aacaacaaaa gttcttctct acataaaatt ttcctatttt    60
   agtgatcagt gaaggaaatc aagaaaaata aatggggaga atgtcaatac ccatgatggg   120
   ttttgtggtg ttatgtctat gggcagtggt agcagaagga tccacccccc agaacatcac   180
   cgacctctgc gccgagagcc acaacaccca aatctacacc ctcaacgaca agattttcag   240
   ctacaccgag agcctcgccg gcaagaggga gatggccatc atcaccttca agaacggcgc   300
   catcttccag gtcgaggtcc ccggcagcca gcacatcgac agccagaaga aggccatcga   360
   gaggatgaag gacaccctca ggatcgccta cctcaccgag gccaaggtcg agaagctctg   420
   cgtctggaac aacaagaccc cccacgccat cgccgccatc agcatggcca acagatcccc   480
   tggttctggt cctggttctc ctagatccac cccccagaac atcaccgacc tctgcgccga   540
   gagccacaac acccaaatct acaccctcaa cgacaagatt ttcagctaca ccgagagcct   600
   cgccggcaag agggagatgg ccatcatcac cttcaagaac ggcgccatct tccaggtcga   660
   ggtccccggc agccagcaca tcgacagcca gaagaaggcc atcgagagga tgaaggacac   720
   cctcaggatc gcctacctca ccgaggccaa ggtcgagaag ctctgcgtct ggaacaacaa   780
   gaccccccac gccatcgccg ccatcagcat ggccaacaga tcccctggtt ctggtcctgg   840
   ttctcctaga tcttatcctt atgattatcc tgattatgct ggatctgaac atgatgaatt   900
   gtga                                                                904
<210> 75
   <211> 21
   <212> DNA
   <213> Nicotiana tabacum
<400> 75
   gatttgttgg ttgatactat g 21
<210> 76
   <211> 7
   <212> PRT
   <213> Nicotiana tabacum
<400> 76
   Asp Leu Leu Val Asp Thr Met
   1               5
<210> 77
   <211> 45
   <212> DNA
   <213> Armoracia rusticana
<400> 77
   ctactccatg atatggtgga ggtcgttgac tttgttagct ctatg 45
<210> 78
   <211> 15
   <212> PRT
   <213> Armoracia rusticana
<400> 78
   Leu Leu His Asp Met Val Glu Val Val Asp Phe Val Ser Ser Met
   1               5                   10                  15
<210> 79
   <211> 75
   <212> PRT
   <213> Lactuca sativa
<400> 79
   Met Ala Ser Ile Ser Ser Ser Ala Ile Ala Thr Val Asn Arg Thr Thr
   1               5                   10                  15
   Ser Thr Gln Ala Ser Leu Ala Ala Pro Phe Thr Gly Leu Lys Ser Asn
               20                  25                  30
   Val Ala Phe Pro Val Thr Lys Lys Ala Asn Asn Asp Phe Ser Ser Leu
           35                  40                  45
   Pro Ser Asn Gly Gly Arg Val Gln Cys Met Lys Val Trp Pro Pro Ile
       50                  55                  60
   Gly Leu Lys Lys Tyr Glu Thr Leu Ser Tyr Leu
   65                  70                  75
<210> 80
   <211> 225
   <212> DNA
   <213> Lactuca sativa
<400> 80
   atggcctcca tctcctcctc agccatcgcc accgtcaacc ggaccacctc cacccaagct      60
   agcttggcag ctccattcac cggcctcaag tctaacgtag ctttcccagt taccaagaag     120
   gctaacaatg acttttcatc cctacccagc aacggtggaa gagtacaatg catgaaggtg     180
   tggccaccaa ttgggttgaa gaagtacgag actctttcat accta                     225
<210> 81
   <211> 51
   <212> DNA
   <213> Artificial
<220>
   <223> an artificially synthesized DNA
<400> 81
   agatcccctg gttctggtcc tggttctcct agatcccctg gttccagatc t 51
<210> 82
   <211> 17
   <212> PRT
   <213> Artificial
<220>
   <223> an artificially synthesized peptide
<400> 82
   Arg Ser Pro Gly Ser Gly Pro Gly Ser Pro Arg Ser Pro Gly Ser Arg
   1               5                   10                  15
   Ser
<210> 83
   <211> 66
   <212> DNA
   <213> Artificial Sequence
<220>
   <223> an artificially synthesized DNA
<400> 83
   agatcccctg gttctggtcc tggttctcct agatcccctg gttctggtcc tggttctcct      60
   agatct                                                                 66
<210> 84
   <211> 22
   <212> PRT
   <213> Artificial
<220>
   <223> an artificially synthesized peptide
<400> 84
   Arg Ser Pro Gly Ser Gly Pro Gly Ser Pro Arg Ser Pro Gly Ser Gly
   1               5                   10                  15
   Pro Gly Ser Pro Arg Ser
               20
<210> 85
   <211> 696
   <212> DNA
   <213> Artificial Sequence
<220>
   <223> an artificially sinthesized DNA construct
<400> 85
   gcggcggatt gtgctaaagg taaaattgag ttttccaagt ataatgagga taataccttt      60
   actgtgaagg tgtcaggaag agaatactgg acgaacagat ggaatttgca gccattgtta     120
   caaagtgctc agctgacagg gatgactgta acaatcatat ctaatacctg cagttcaggc     180
   tcaggctttg cccaggtgaa gtttaacaga tcccctggtt ctggtcctgg ttctcctaga     240
   tccgcggcgg attgtgctaa aggtaaaatt gagttttcca agtataatga ggataatacc     300
   tttactgtga aggtgtcagg aagagaatac tggacgaaca gatggaattt gcagccattg     360
   ttacaaagtg ctcagctgac agggatgact gtaacaatca tatctaatac ctgcagttca     420
   ggctcaggct ttgcccaggt gaagtttaac agatcccctg gttctggtcc tggttctcct     480
   agatccgcgg cggattgtgc taaaggtaaa attgagtttt ccaagtataa tgaggataat     540
   acctttactg tgaaggtgtc aggaagagaa tactggacga acagatggaa tttgcagcca     600
   ttgttacaaa gtgctcagct gacagggatg actgtaacaa tcatatctaa tacctgcagt     660
   tcaggctcag gctttgccca ggtgaagttt aactga                               696
<210> 86
   <211> 231
   <212> PRT
   <213> Artificial
<220>
   <223> an artificially sinthesized protein
<400> 86
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn Arg Ser Pro Gly Ser Gly Pro Gly Ser Pro Arg
   65                  70                  75                  80
   Ser Ala Ala Asp Cys Ala Lys Gly Lys Ile Glu Phe Ser Lys Tyr Asn
                   85                  90                  95
   Glu Asp Asn Thr Phe Thr Val Lys Val Ser Gly Arg Glu Tyr Trp Thr
               100                 105                 110
   Asn Arg Trp Asn Leu Gln Pro Leu Leu Gln Ser Ala Gln Leu Thr Gly
           115                 120                 125
   Met Thr Val Thr Ile Ile Ser Asn Thr Cys Ser Ser Gly Ser Gly Phe
       130                 135                 140
   Ala Gln Val Lys Phe Asn Arg Ser Pro Gly Ser Gly Pro Gly Ser Pro
   145                 150                 155                 160
   Arg Ser Ala Ala Asp Cys Ala Lys Gly Lys Ile Glu Phe Ser Lys Tyr
                   165                 170                 175
   Asn Glu Asp Asn Thr Phe Thr Val Lys Val Ser Gly Arg Glu Tyr Trp
               180                 185                 190
   Thr Asn Arg Trp Asn Leu Gln Pro Leu Leu Gln Ser Ala Gln Leu Thr
           195                 200                 205
   Gly Met Thr Val Thr Ile Ile Ser Asn Thr Cys Ser Ser Gly Ser Gly
       210                 215                 220
   Phe Ala Gln Val Lys Phe Asn
   225                 230
<210> 87
   <211> 732
   <212> DNA
   <213> Artificial
<220>
   <223> an artificially sinthesized DNA construct
<400> 87
   gcggcggatt gtgctaaagg taaaattgag ttttccaagt ataatgagga taataccttt    60
   actgtgaagg tgtcaggaag agaatactgg acgaacagat ggaatttgca gccattgtta   120
   caaagtgctc agctgacagg gatgactgta acaatcatat ctaatacctg cagttcaggc   180
   tcaggctttg cccaggtgaa gtttaacaga tcccctggtt ctggtcctgg ttctcctaga   240
   tccgcggcgg attgtgctaa aggtaaaatt gagttttcca agtataatga ggataatacc   300
   tttactgtga aggtgtcagg aagagaatac tggacgaaca gatggaattt gcagccattg   360
   ttacaaagtg ctcagctgac agggatgact gtaacaatca tatctaatac ctgcagttca   420
   ggctcaggct ttgcccaggt gaagtttaac agatcccctg gttctggtcc tggttctcct   480
   agatccgcgg cggattgtgc taaaggtaaa attgagtttt ccaagtataa tgaggataat   540
   acctttactg tgaaggtgtc aggaagagaa tactggacga acagatggaa tttgcagcca   600
   ttgttacaaa gtgctcagct gacagggatg actgtaacaa tcatatctaa tacctgcagt   660
   tcaggctcag gctttgccca ggtgaagttt aacagatccc ctggttctgg tcctggttct   720
   cctagatcct ga                                                       732
<210> 88
   <211> 243
   <212> PRT
   <213> Artificial
<220>
   <223> an artificially sinthesized protein
<400> 88
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn Arg Ser Pro Gly Ser Gly Pro Gly Ser Pro Arg
   65                  70                  75                  80
   Ser Ala Ala Asp Cys Ala Lys Gly Lys Ile Glu Phe Ser Lys Tyr Asn
                   85                  90                  95
   Glu Asp Asn Thr Phe Thr Val Lys Val Ser Gly Arg Glu Tyr Trp Thr
               100                 105                 110
   Asn Arg Trp Asn Leu Gln Pro Leu Leu Gln Ser Ala Gln Leu Thr Gly
           115                 120                 125
   Met Thr Val Thr Ile Ile Ser Asn Thr Cys Ser Ser Gly Ser Gly Phe
       130                 135                 140
   Ala Gln Val Lys Phe Asn Arg Ser Pro Gly Ser Gly Pro Gly Ser Pro
   145                 150                 155                 160
   Arg Ser Ala Ala Asp Cys Ala Lys Gly Lys Ile Glu Phe Ser Lys Tyr
                   165                 170                 175
   Asn Glu Asp Asn Thr Phe Thr Val Lys Val Ser Gly Arg Glu Tyr Trp
               180                 185                 190
   Thr Asn Arg Trp Asn Leu Gln Pro Leu Leu Gln Ser Ala Gln Leu Thr
           195                 200                 205
   Gly Met Thr Val Thr Ile Ile Ser Asn Thr Cys Ser Ser Gly Ser Gly
       210                 215                 220
   Phe Ala Gln Val Lys Phe Asn Arg Ser Pro Gly Ser Gly Pro Gly Ser
   225                 230                 235                 240
   Pro Arg Ser
<210> 89
   <211> 504
   <212> DNA
   <213> Artificial
<220>
   <223> an artificially sinthesized DNA construct
<400> 89
   gcggcggatt gtgctaaagg taaaattgag ttttccaagt ataatgagga taataccttt        60
   actgtgaagg tgtcaggaag agaatactgg acgaacagat ggaatttgca gccattgtta       120
   caaagtgctc agctgacagg gatgactgta acaatcatat ctaatacctg cagttcaggc       180
   tcaggctttg cccaggtgaa gtttaacaga tcccctggtt ctggtcctgg ttctcctaga       240
   tcccctggtt ccagatctgc ggcggattgt gctaaaggta aaattgagtt ttccaagtat       300
   aatgaggata atacctttac tgtgaaggtg tcaggaagag aatactggac gaacagatgg       360
   aatttgcagc cattgttaca aagtgctcag ctgacaggga tgactgtaac aatcatatct       420
   aatacctgca gttcaggctc aggctttgcc caggtgaagt ttaacagatc ccctggttct       480
   ggtcctggtt ctcctagatc ttga                                              504
<210> 90
   <211> 167
   <212> PRT
   <213> Artificial
<220>
   <223> an artificially sinthesized protein
<400> 90
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn Arg Ser Pro Gly Ser Gly Pro Gly Ser Pro Arg
   65                  70                  75                  80
   Ser Pro Gly Ser Arg Ser Ala Ala Asp Cys Ala Lys Gly Lys Ile Glu
                   85                  90                  95
   Phe Ser Lys Tyr Asn Glu Asp Asn Thr Phe Thr Val Lys Val Ser Gly
               100                 105                 110
   Arg Glu Tyr Trp Thr Asn Arg Trp Asn Leu Gln Pro Leu Leu Gln Ser
           115                 120                 125
   Ala Gln Leu Thr Gly Met Thr Val Thr Ile Ile Ser Asn Thr Cys Ser
       130                 135                 140
   Ser Gly Ser Gly Phe Ala Gln Val Lys Phe Asn Arg Ser Pro Gly Ser
   145                 150                 155                 160
   Gly Pro Gly Ser Pro Arg Ser
                   165
<210> 91
   <211> 519
   <212> DNA
   <213> Artificial
<220>
   <223> an artificially sinthesized DNA construct
<400> 91
   gcggcggatt gtgctaaagg taaaattgag ttttccaagt ataatgagga taataccttt      60
   actgtgaagg tgtcaggaag agaatactgg acgaacagat ggaatttgca gccattgtta     120
   caaagtgctc agctgacagg gatgactgta acaatcatat ctaatacctg cagttcaggc     180
   tcaggctttg cccaggtgaa gtttaacaga tcccctggtt ctggtcctgg ttctcctaga     240
   tcccctggtt ctggtcctgg ttctcctaga tctgcggcgg attgtgctaa aggtaaaatt     300
   gagttttcca agtataatga ggataatacc tttactgtga aggtgtcagg aagagaatac     360
   tggacgaaca gatggaattt gcagccattg ttacaaagtg ctcagctgac agggatgact     420
   gtaacaatca tatctaatac ctgcagttca ggctcaggct ttgcccaggt gaagtttaac     480
   agatcccctg gttctggtcc tggttctcct agatcttga                            519
<210> 92
   <211> 172
   <212> PRT
   <213> Artificial
<220>
   <223> an artificially sinthesized protein
<400> 92
   Ala Ala Asp Cys Ala Lys Gly Lys Ile Glu Phe Ser Lys Tyr Asn Glu
   1               5                   10                  15
   Asp Asn Thr Phe Thr Val Lys Val Ser Gly Arg Glu Tyr Trp Thr Asn
               20                  25                  30
   Arg Trp Asn Leu Gln Pro Leu Leu Gln Ser Ala Gln Leu Thr Gly Met
           35                  40                  45
   Thr Val Thr Ile Ile Ser Asn Thr Cys Ser Ser Gly Ser Gly Phe Ala
       50                  55                  60
   Gln Val Lys Phe Asn Arg Ser Pro Gly Ser Gly Pro Gly Ser Pro Arg
   65                  70                  75                  80
   Ser Pro Gly Ser Gly Pro Gly Ser Pro Arg Ser Ala Ala Asp Cys Ala
                   85                  90                  95
   Lys Gly Lys Ile Glu Phe Ser Lys Tyr Asn Glu Asp Asn Thr Phe Thr
               100                 105                 110
   Val Lys Val Ser Gly Arg Glu Tyr Trp Thr Asn Arg Trp Asn Leu Gln
           115                 120                 125
   Pro Leu Leu Gln Ser Ala Gln Leu Thr Gly Met Thr Val Thr Ile Ile
       130                 135                 140
   Ser Asn Thr Cys Ser Ser Gly Ser Gly Phe Ala Gln Val Lys Phe Asn
   145                 150                 155                 160
   Arg Ser Pro Gly Ser Gly Pro Gly Ser Pro Arg Ser
                   165                 170
<210> 93
   <211> 1002
   <212> DNA
   <213> Artificial
<220>
   <223> an artificially sinthesized DNA construct
<400> 93
   accccccaga acatcaccga cctctgcgcc gagagccaca acacccaaat ctacaccctc      60
   aacgacaaga ttttcagcta caccgagagc ctcgccggca agagggagat ggccatcatc     120
   accttcaaga acggcgccat cttccaggtc gaggtccccg gcagccagca catcgacagc     180
   cagaagaagg ccatcgagag gatgaaggac accctcagga tcgcctacct caccgaggcc     240
   aaggtcgaga agctctgcgt ctggaacaac aagacccccc acgccatcgc cgccatcagc      300
   atggccaaca gatcccctgg ttctggtcct ggttctccta gatccacccc ccagaacatc     360
   accgacctct gcgccgagag ccacaacacc caaatctaca ccctcaacga caagattttc     420
   agctacaccg agagcctcgc cggcaagagg gagatggcca tcatcacctt caagaacggc     480
   gccatcttcc aggtcgaggt ccccggcagc cagcacatcg acagccagaa gaaggccatc     540
   gagaggatga aggacaccct caggatcgcc tacctcaccg aggccaaggt cgagaagctc     600
   tgcgtctgga acaacaagac cccccacgcc atcgccgcca tcagcatggc caacagatcc     660
   cctggttctg gtcctggttc tcctagatcc accccccaga acatcaccga cctctgcgcc     720
   gagagccaca acacccaaat ctacaccctc aacgacaaga ttttcagcta caccgagagc     780
   ctcgccggca agagggagat ggccatcatc accttcaaga acggcgccat cttccaggtc     840
   gaggtccccg gcagccagca catcgacagc cagaagaagg ccatcgagag gatgaaggac     900
   accctcagga tcgcctacct caccgaggcc aaggtcgaga agctctgcgt ctggaacaac     960
   aagacccccc acgccatcgc cgccatcagc atggccaact ga                        1002
<210> 94
   <211> 333
   <212> PRT
   <213> Artificial
<220>
   <223> an artificially sinthesized protein
<400> 94
   Thr Pro Gln Asn Ile Thr Asp Leu Cys Ala Glu Ser His Asn Thr Gln
   1               5                   10                  15
   Ile Tyr Thr Leu Asn Asp Lys Ile Phe Ser Tyr Thr Glu Ser Leu Ala
               20                  25                  30
   Gly Lys Arg Glu Met Ala Ile Ile Thr Phe Lys Asn Gly Ala Ile Phe
           35                  40                  45
   Gln Val Glu Val Pro Gly Ser Gln His Ile Asp Ser Gln Lys Lys Ala
       50                  55                  60
   Ile Glu Arg Met Lys Asp Thr Leu Arg Ile Ala Tyr Leu Thr Glu Ala
   65                  70                  75                  80
   Lys Val Glu Lys Leu Cys Val Trp Asn Asn Lys Thr Pro His Ala Ile
                   85                  90                  95
   Ala Ala Ile Ser Met Ala Asn Arg Ser Pro Gly Ser Gly Pro Gly Ser
               100                 105                 110
   Pro Arg Ser Thr Pro Gln Asn Ile Thr Asp Leu Cys Ala Glu Ser His
           115                 120                 125
   Asn Thr Gln Ile Tyr Thr Leu Asn Asp Lys Ile Phe Ser Tyr Thr Glu
       130                 135                 140
   Ser Leu Ala Gly Lys Arg Glu Met Ala Ile Ile Thr Phe Lys Asn Gly
   145                 150                 155                 160
   Ala Ile Phe Gln Val Glu Val Pro Gly Ser Gln His Ile Asp Ser Gln
                   165                 170                 175
   Lys Lys Ala Ile Glu Arg Met Lys Asp Thr Leu Arg Ile Ala Tyr Leu
               180                 185                 190
   Thr Glu Ala Lys Val Glu Lys Leu Cys Val Trp Asn Asn Lys Thr Pro
           195                 200                 205
   His Ala Ile Ala Ala Ile Ser Met Ala Asn Arg Ser Pro Gly Ser Gly
       210                 215                 220
   Pro Gly Ser Pro Arg Ser Thr Pro Gln Asn Ile Thr Asp Leu Cys Ala
   225                 230                 235                 240
   Glu Ser His Asn Thr Gln Ile Tyr Thr Leu Asn Asp Lys Ile Phe Ser
                   245                 250                 255
   Tyr Thr Glu Ser Leu Ala Gly Lys Arg Glu Met Ala Ile Ile Thr Phe
               260                 265                 270
   Lys Asn Gly Ala Ile Phe Gln Val Glu Val Pro Gly Ser Gln His Ile
           275                 280                 285
   Asp Ser Gln Lys Lys Ala Ile Glu Arg Met Lys Asp Thr Leu Arg Ile
       290                 295                 300
   Ala Tyr Leu Thr Glu Ala Lys Val Glu Lys Leu Cys Val Trp Asn Asn
   305                 310                 315                 320
   Lys Thr Pro His Ala Ile Ala Ala Ile Ser Met Ala Asn
                   325                 330
<210> 95
   <211> 1038
   <212> DNA
   <213> Artificial
<220>
   <223> an artificially sinthesized DNA construct
<400> 95
   accccccaga acatcaccga cctctgcgcc gagagccaca acacccaaat ctacaccctc     60
   aacgacaaga ttttcagcta caccgagagc ctcgccggca agagggagat ggccatcatc     120
   accttcaaga acggcgccat cttccaggtc gaggtccccg gcagccagca catcgacagc     180
   cagaagaagg ccatcgagag gatgaaggac accctcagga tcgcctacct caccgaggcc     240
   aaggtcgaga agctctgcgt ctggaacaac aagacccccc acgccatcgc cgccatcagc     300
   atggccaaca gatcccctgg ttctggtcct ggttctccta gatccacccc ccagaacatc     360
   accgacctct gcgccgagag ccacaacacc caaatctaca ccctcaacga caagattttc     420
   agctacaccg agagcctcgc cggcaagagg gagatggcca tcatcacctt caagaacggc     480
   gccatcttcc aggtcgaggt ccccggcagc cagcacatcg acagccagaa gaaggccatc     540
   gagaggatga aggacaccct caggatcgcc tacctcaccg aggccaaggt cgagaagctc     600
   tgcgtctgga acaacaagac cccccacgcc atcgccgcca tcagcatggc caacagatcc     660
   cctggttctg gtcctggttc tcctagatct acccccagaa acatcaccga cctctgcgcc     720
   gagagccaca acacccaaat ctacaccctc aacgacaaga ttttcagcta caccgagagc     780
   ctcgccggca agagggagat ggccatcatc accttcaaga acggcgccat cttccaggtc     840
   gaggtccccg gcagccagca catcgacagc cagaagaagg ccatcgagag gatgaaggac     900
   accctcagga tcgcctacct caccgaggcc aaggtcgaga agctctgcgt ctggaacaac     960
   aagacccccc acgccatcgc cgccatcagc atggccaaca gatcccctgg ttctggtcct    1020
   ggttctccta gatcttga                                                  1038
<210> 96
   <211> 345
   <212> PRT
   <213> Artificial
<220>
   <223> an artificially sinthesized protein
<400> 96
   Thr Pro Gln Asn Ile Thr Asp Leu Cys Ala Glu Ser His Asn Thr Gln
   1               5                   10                  15
   Ile Tyr Thr Leu Asn Asp Lys Ile Phe Ser Tyr Thr Glu Ser Leu Ala
               20                  25                  30
   Gly Lys Arg Glu Met Ala Ile Ile Thr Phe Lys Asn Gly Ala Ile Phe
           35                  40                  45
   Gln Val Glu Val Pro Gly Ser Gln His Ile Asp Ser Gln Lys Lys Ala
       50                  55                  60
   Ile Glu Arg Met Lys Asp Thr Leu Arg Ile Ala Tyr Leu Thr Glu Ala
   65                  70                  75                  80
   Lys Val Glu Lys Leu Cys Val Trp Asn Asn Lys Thr Pro His Ala Ile
                   85                  90                  95
   Ala Ala Ile Ser Met Ala Asn Arg Ser Pro Gly Ser Gly Pro Gly Ser
               100                 105                 110
   Pro Arg Ser Thr Pro Gln Asn Ile Thr Asp Leu Cys Ala Glu Ser His
           115                 120                 125
   Asn Thr Gln Ile Tyr Thr Leu Asn Asp Lys Ile Phe Ser Tyr Thr Glu
       130                 135                 140
   Ser Leu Ala Gly Lys Arg Glu Met Ala Ile Ile Thr Phe Lys Asn Gly
   145                 150                 155                 160
   Ala Ile Phe Gln Val Glu Val Pro Gly Ser Gln His Ile Asp Ser Gln
                   165                 170                 175
   Lys Lys Ala Ile Glu Arg Met Lys Asp Thr Leu Arg Ile Ala Tyr Leu
               180                 185                 190
   Thr Glu Ala Lys Val Glu Lys Leu Cys Val Trp Asn Asn Lys Thr Pro
           195                 200                 205
   His Ala Ile Ala Ala Ile Ser Met Ala Asn Arg Ser Pro Gly Ser Gly
       210                 215                 220
   Pro Gly Ser Pro Arg Ser Thr Pro Gln Asn Ile Thr Asp Leu Cys Ala
   225                 230                 235                 240
   Glu Ser His Asn Thr Gln Ile Tyr Thr Leu Asn Asp Lys Ile Phe Ser
                   245                 250                 255
   Tyr Thr Glu Ser Leu Ala Gly Lys Arg Glu Met Ala Ile Ile Thr Phe
               260                 265                 270
   Lys Asn Gly Ala Ile Phe Gln Val Glu Val Pro Gly Ser Gln His Ile
           275                 280                 285
   Asp Ser Gln Lys Lys Ala Ile Glu Arg Met Lys Asp Thr Leu Arg Ile
       290                 295                 300
   Ala Tyr Leu Thr Glu Ala Lys Val Glu Lys Leu Cys Val Trp Asn Asn
   305                 310                 315                 320
   Lys Thr Pro His Ala Ile Ala Ala Ile Ser Met Ala Asn Arg Ser Pro
                   325                 330                 335
   Gly Ser Gly Pro Gly Ser Pro Arg Ser
               340                 345
<210> 97
   <211> 708
   <212> DNA
   <213> Artificial
<220>
   <223> an artificially sinthesized DNA construct
<400> 97
   accccccaga acatcaccga cctctgcgcc gagagccaca acacccaaat ctacaccctc    60
   aacgacaaga ttttcagcta caccgagagc ctcgccggca agagggagat ggccatcatc   120
   accttcaaga acggcgccat cttccaggtc gaggtccccg gcagccagca catcgacagc   180
   cagaagaagg ccatcgagag gatgaaggac accctcagga tcgcctacct caccgaggcc   240
   aaggtcgaga agctctgcgt ctggaacaac aagacccccc acgccatcgc cgccatcagc    300
   atggccaaca gatcccctgg ttctggtcct ggttctccta gatcccctgg ttccagatct   360
   accccccaga acatcaccga cctctgcgcc gagagccaca acacccaaat ctacaccctc    420
   aacgacaaga ttttcagcta caccgagagc ctcgccggca agagggagat ggccatcatc   480
   accttcaaga acggcgccat cttccaggtc gaggtccccg gcagccagca catcgacagc   540
   cagaagaagg ccatcgagag gatgaaggac accctcagga tcgcctacct caccgaggcc   600
   aaggtcgaga agctctgcgt ctggaacaac aagacccccc acgccatcgc cgccatcagc    660
   atggccaaca gatcccctgg ttctggtcct ggttctccta gatcttga                708
<210> 18
   <211> 235
   <212> PRT
   <213> Artificial
<220>
   <223> an artificially sinthesized protein
<400> 98
<210> 99
   <211> 723
   <212> DNA
   <213> Artificial
<220>
   <223> an artificially sinthesized DNA construct
<400> 99
   accccccaga acatcaccga cctctgcgcc gagagccaca acacccaaat ctacaccctc      60
   aacgacaaga ttttcagcta caccgagagc ctcgccggca agagggagat ggccatcatc     120
   accttcaaga acggcgccat cttccaggtc gaggtccccg gcagccagca catcgacagc     180
   cagaagaagg ccatcgagag gatgaaggac accctcagga tcgcctacct caccgaggcc     240
   aaggtcgaga agctctgcgt ctggaacaac aagacccccc acgccatcgc cgccatcagc     300
   atggccaaca gatcccctgg ttctggtcct ggttctccta gatcccctgg ttctggtcct     360
   ggttctccta gatctacccc ccagaacatc accgacctct gcgccgagag ccacaacacc     420
   caaatctaca ccctcaacga caagattttc agctacaccg agagcctcgc cggcaagagg     480
   gagatggcca tcatcacctt caagaacggc gccatcttcc aggtcgaggt ccccggcagc     540
   cagcacatcg acagccagaa gaaggccatc gagaggatga aggacaccct caggatcgcc     600
   tacctcaccg aggccaaggt cgagaagctc tgcgtctgga acaacaagac cccccacgcc     660
   atcgccgcca tcagcatggc caacagatcc cctggttctg gtcctggttc tcctagatct     720
   tga                                                                   723
<210> 100
   <211> 240
   <212> PRT
   <213> Artificial
<220>
   <223> an artificially sinthesized protein
<400> 100
   Thr Pro Gln Asn Ile Thr Asp Leu Cys Ala Glu Ser His Asn Thr Gln
   1               5                   10                  15
   Ile Tyr Thr Leu Asn Asp Lys Ile Phe Ser Tyr Thr Glu Ser Leu Ala
               20                  25                  30
   Gly Lys Arg Glu Met Ala Ile Ile Thr Phe Lys Asn Gly Ala Ile Phe
           35                  40                  45
   Gln Val Glu Val Pro Gly Ser Gln His Ile Asp Ser Gln Lys Lys Ala
       50                  55                  60
   Ile Glu Arg Met Lys Asp Thr Leu Arg Ile Ala Tyr Leu Thr Glu Ala
   65                  70                  75                  80
   Lys Val Glu Lys Leu Cys Val Trp Asn Asn Lys Thr Pro His Ala Ile
                   85                  90                  95
   Ala Ala Ile Ser Met Ala Asn Arg Ser Pro Gly Ser Gly Pro Gly Ser
               100                 105                 110
   Pro Arg Ser Pro Gly Ser Gly Pro Gly Ser Pro Arg Ser Thr Pro Gln
           115                 120                 125
   Asn Ile Thr Asp Leu Cys Ala Glu Ser His Asn Thr Gln Ile Tyr Thr
       130                 135                 140
   Leu Asn Asp Lys Ile Phe Ser Tyr Thr Glu Ser Leu Ala Gly Lys Arg
   145                 150                 155                 160
   Glu Met Ala Ile Ile Thr Phe Lys Asn Gly Ala Ile Phe Gln Val Glu
                   165                 170                 175
   Val Pro Gly Ser Gln His Ile Asp Ser Gln Lys Lys Ala Ile Glu Arg
               180                 185                 190
   Met Lys Asp Thr Leu Arg Ile Ala Tyr Leu Thr Glu Ala Lys Val Glu
           195                 200                 205
   Lys Leu Cys Val Trp Asn Asn Lys Thr Pro His Ala Ile Ala Ala Ile
       210                 215                 220
   Ser Met Ala Asn Arg Ser Pro Gly Ser Gly Pro Gly Ser Pro Arg Ser
   225                 230                 235                 240
<210> 101
   <211> 583
   <212> DNA
   <213> Artificial
<220>
   <223> an artificially sinthesized DNA construct
<400> 101
   tatttaactc agtattcaga aacaacaaaa gttcttctct acataaaatt ttcctatttt      60
   agtgatcagt gaaggaaatc aagaaaaata aatggcggcg gattgtgcta aaggtaaaat     120
   tgagttttcc aagtataatg aggataatac ctttactgtg aaggtgtcag gaagagaata     180
   ctggacgaac agatggaatt tgcagccatt gttacaaagt gctcagctga cagggatgac     240
   tgtaacaatc atatctaata cctgcagttc aggctcaggc tttgcccagg tgaagtttaa     300
   cagatcccct ggttctggtc ctggttctcc tagatccgcg gcggattgtg ctaaaggtaa     360
   aattgagttt tccaagtata atgaggataa tacctttact gtgaaggtgt caggaagaga     420
   atactggacg aacagatgga atttgcagcc attgttacaa agtgctcagc tgacagggat     480
   gactgtaaca atcatatcta atacctgcag ttcaggctca ggctttgccc aggtgaagtt     540
   taacagatcc cctggttctg gtcctggttc tcctagatcc tga                       583
<210> 102
   <211> 682
   <212> DNA
   <213> Artificial
<220>
   <223> an artificially sinthesized DNA construct
<400> 102
   tatttaactc agtattcaga aacaacaaaa gttcttctct acataaaatt ttcctatttt      60
   agtgatcagt gaaggaaatc aagaaaaata aatggggaga atgtcaatac ccatgatggg     120
   ttttgtggtg ttatgtctat gggcagtggt agcagaagga tccgcggcgg attgtgctaa     180
   aggtaaaatt gagttttcca agtataatga ggataatacc tttactgtga aggtgtcagg     240
   aagagaatac tggacgaaca gatggaattt gcagccattg ttacaaagtg ctcagctgac     300
   agggatgact gtaacaatca tatctaatac ctgcagttca ggctcaggct ttgcccaggt     360
   gaagtttaac agatcccctg gttctggtcc tggttctcct agatcccctg gttccagatc   420
   tgcggcggat tgtgctaaag gtaaaattga gttttccaag tataatgagg ataatacctt     480
   tactgtgaag gtgtcaggaa gagaatactg gacgaacaga tggaatttgc agccattgtt     540
   acaaagtgct cagctgacag ggatgactgt aacaatcata tctaatacct gcagttcagg     600
   ctcaggcttt gcccaggtga agtttaacag atcccctggt tctggtcctg gttctcctag     660
   atctgaacat gatgaattgt ga                                              682
<210> 103
   <211> 697
   <212> DNA
   <213> Artificial
<220>
   <223> an artificially sinthesized DNA construct
<400> 103
   tatttaactc agtattcaga aacaacaaaa gttcttctct acataaaatt ttcctatttt      60
   agtgatcagt gaaggaaatc aagaaaaata aatggggaga atgtcaatac ccatgatggg     120
   ttttgtggtg ttatgtctat gggcagtggt agcagaagga tccgcggcgg attgtgctaa     180
   aggtaaaatt gagttttcca agtataatga ggataatacc tttactgtga aggtgtcagg     240
   aagagaatac tggacgaaca gatggaattt gcagccattg ttacaaagtg ctcagctgac     300
   agggatgact gtaacaatca tatctaatac ctgcagttca ggctcaggct ttgcccaggt     360
   gaagtttaac agatcccctg gttctggtcc tggttctcct agatcccctg gttctggtcc   420
   tggttctcct agatctgcgg cggattgtgc taaaggtaaa attgagtttt ccaagtataa     480
   tgaggataat acctttactg tgaaggtgtc aggaagagaa tactggacga acagatggaa     540
   tttgcagcca ttgttacaaa gtgctcagct gacagggatg actgtaacaa tcatatctaa     600
   tacctgcagt tcaggctcag gctttgccca ggtgaagttt aacagatccc ctggttctgg     660
   tcctggttct cctagatctg aacatgatga attgtga                              697
<210> 104
   <211> 805
   <212> DNA
   <213> Artificial
<220>
   <223> an artificially sinthesized DNA construct
<400> 104
   tatttaactc agtattcaga aacaacaaaa gttcttctct acataaaatt ttcctatttt      60
   agtgatcagt gaaggaaatc aagaaaaata aatggcctcc atctcctcct cagccatcgc     120
   caccgtcaac cggaccacct ccacccaagc tagcttggca gctccattca ccggcctcaa     180
   gtctaacgta gctttcccag ttaccaagaa ggctaacaat gacttttcat ccctacccag     240
   caacggtgga agagtacaat gcatgaaggt gtggccacca attgggttga agaagtacga     300
   gactctttca tacctagcgg cggattgtgc taaaggtaaa attgagtttt ccaagtataa     360
   tgaggataat acctttactg tgaaggtgtc aggaagagaa tactggacga acagatggaa     420
   tttgcagcca ttgttacaaa gtgctcagct gacagggatg actgtaacaa tcatatctaa     480
   tacctgcagt tcaggctcag gctttgccca ggtgaagttt aacagatccc ctggttctgg     540
   tcctggttct cctagatccg cggcggattg tgctaaaggt aaaattgagt tttccaagta     600
   taatgaggat aataccttta ctgtgaaggt gtcaggaaga gaatactgga cgaacagatg    660
   gaatttgcag ccattgttac aaagtgctca gctgacaggg atgactgtaa caatcatatc     720
   taatacctgc agttcaggct caggctttgc ccaggtgaag tttaacagat cccctggttc     780
   tggtcctggt tctcctagat cctga                                           805
<210> 105
   <211> 826
   <212> DNA
   <213> Artificial
<220>
   <223> an artificially sinthesized DNA construct
<400> 105
   tatttaactc agtattcaga aacaacaaaa gttcttctct acataaaatt ttcctatttt     60
   agtgatcagt gaaggaaatc aagaaaaata aatggcggcg gattgtgcta aaggtaaaat    120
   tgagttttcc aagtataatg aggataatac ctttactgtg aaggtgtcag gaagagaata    180
   ctggacgaac agatggaatt tgcagccatt gttacaaagt gctcagctga cagggatgac    240
   tgtaacaatc atatctaata cctgcagttc aggctcaggc tttgcccagg tgaagtttaa    300
   cagatcccct ggttctggtc ctggttctcc tagatccgcg gcggattgtg ctaaaggtaa    360
   aattgagttt tccaagtata atgaggataa tacctttact gtgaaggtgt caggaagaga    420
   atactggacg aacagatgga atttgcagcc attgttacaa agtgctcagc tgacagggat    480
   gactgtaaca atcatatcta atacctgcag ttcaggctca ggctttgccc aggtgaagtt    540
   taacagatcc cctggttctg gtcctggttc tcctagatcc gcggcggatt gtgctaaagg    600
   taaaattgag ttttccaagt ataatgagga taataccttt actgtgaagg tgtcaggaag    660
   agaatactgg acgaacagat ggaatttgca gccattgtta caaagtgctc agctgacagg    720
   gatgactgta acaatcatat ctaatacctg cagttcaggc tcaggctttg cccaggtgaa    780
   gtttaacaga tcccctggtt ctggtcctgg ttctcctaga tcctga                   826
<210> 106
   <211> 910
   <212> DNA
   <213> Artificial
<220>
   <223> an artificially sinthesized DNA construct
<400> 106
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccgcggcgg attgtgctaa    180
   aggtaaaatt gagttttcca agtataatga ggataatacc tttactgtga aggtgtcagg    240
   aagagaatac tggacgaaca gatggaattt gcagccattg ttacaaagtg ctcagctgac    300
   agggatgact gtaacaatca tatctaatac ctgcagttca ggctcaggct ttgcccaggt    360
   gaagtttaac agatcccctg gttctggtcc tggttctcct agatccgcgg cggattgtgc    420
   taaaggtaaa attgagtttt ccaagtataa tgaggataat acctttactg tgaaggtgtc    480
   aggaagagaa tactggacga acagatggaa tttgcagcca ttgttacaaa gtgctcagct    540
   gacagggatg actgtaacaa tcatatctaa tacctgcagt tcaggctcag gctttgccca    600
   ggtgaagttt aacagatccc ctggttctgg tcctggttct cctagatccg cggcggattg     660
   tgctaaaggt aaaattgagt tttccaagta taatgaggat aataccttta ctgtgaaggt     720
   gtcaggaaga gaatactgga cgaacagatg gaatttgcag ccattgttac aaagtgctca    780
   gctgacaggg atgactgtaa caatcatatc taatacctgc agttcaggct caggctttgc    840
   ccaggtgaag tttaacagat cccctggttc tggtcctggt tctcctagat ccgaacatga    900
   tgaattgtga                                                           910
<210> 107
   <211> 1048
   <212> DNA
   <213> Artificial
<220>
   <223> an artificially sinthesized DNA construct
<400> 107
   tatttaactc agtattcaga aacaacaaaa gttcttctct acataaaatt ttcctatttt      60
   agtgatcagt gaaggaaatc aagaaaaata aatggcctcc atctcctcct cagccatcgc     120
   caccgtcaac cggaccacct ccacccaagc tagcttggca gctccattca ccggcctcaa     180
   gtctaacgta gctttcccag ttaccaagaa ggctaacaat gacttttcat ccctacccag     240
   caacggtgga agagtacaat gcatgaaggt gtggccacca attgggttga agaagtacga     300
   gactctttca tacctagcgg cggattgtgc taaaggtaaa attgagtttt ccaagtataa     360
   tgaggataat acctttactg tgaaggtgtc aggaagagaa tactggacga acagatggaa     420
   tttgcagcca ttgttacaaa gtgctcagct gacagggatg actgtaacaa tcatatctaa     480
   tacctgcagt tcaggctcag gctttgccca ggtgaagttt aacagatccc ctggttctgg     540
   tcctggttct cctagatccg cggcggattg tgctaaaggt aaaattgagt tttccaagta     600
   taatgaggat aataccttta ctgtgaaggt gtcaggaaga gaatactgga cgaacagatg    660
   gaatttgcag ccattgttac aaagtgctca gctgacaggg atgactgtaa caatcatatc     720
   taatacctgc agttcaggct caggctttgc ccaggtgaag tttaacagat cccctggttc     780
   tggtcctggt tctcctagat ccgcggcgga ttgtgctaaa ggtaaaattg agttttccaa     840
   gtataatgag gataatacct ttactgtgaa ggtgtcagga agagaatact ggacgaacag     900
   atggaatttg cagccattgt tacaaagtgc tcagctgaca gggatgactg taacaatcat     960
   atctaatacc tgcagttcag gctcaggctt tgcccaggtg aagtttaaca gatcccctgg    1020
   ttctggtcct ggttctccta gatcctga                                       1048
<210> 108
   <211> 787
   <212> DNA
   <213> Artificial
<220>
   <223> an artificially synthesized DNA construct
<400> 108
   tatttaactc agtattcaga aacaacaaaa gttcttctct acataaaatt ttcctatttt      60
   agtgatcagt gaaggaaatc aagaaaaata aatgaccccc cagaacatca ccgacctctg     120
   cgccgagagc cacaacaccc aaatctacac cctcaacgac aagattttca gctacaccga     180
   gagcctcgcc ggcaagaggg agatggccat catcaccttc aagaacggcg ccatcttcca     240
   ggtcgaggtc cccggcagcc agcacatcga cagccagaag aaggccatcg agaggatgaa     300
   ggacaccctc aggatcgcct acctcaccga ggccaaggtc gagaagctct gcgtctggaa     360
   caacaagacc ccccacgcca tcgccgccat cagcatggcc aacagatccc ctggttctgg     420
   tcctggttct cctagatcca ccccccagaa catcaccgac ctctgcgccg agagccacaa     480
   cacccaaatc tacaccctca acgacaagat tttcagctac accgagagcc tcgccggcaa     540
   gagggagatg gccatcatca ccttcaagaa cggcgccatc ttccaggtcg aggtccccgg     600
   cagccagcac atcgacagcc agaagaaggc catcgagagg atgaaggaca ccctcaggat     660
   cgcctacctc accgaggcca aggtcgagaa gctctgcgtc tggaacaaca agacccccca     720
   cgccatcgcc gccatcagca tggccaacag atcccctggt tctggtcctg gttctcctag     780
   atcctga                                                               787
<210> 109
   <211> 886
   <212> DNA
   <213> Artificial
<220>
   <223> an artificially synthesized DNA construct
<400> 109
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccacccccc agaacatcac    180
   cgacctctgc gccgagagcc acaacaccca aatctacacc ctcaacgaca agattttcag    240
   ctacaccgag agcctcgccg gcaagaggga gatggccatc atcaccttca agaacggcgc    300
   catcttccag gtcgaggtcc ccggcagcca gcacatcgac agccagaaga aggccatcga    360
   gaggatgaag gacaccctca ggatcgccta cctcaccgag gccaaggtcg agaagctctg    420
   cgtctggaac aacaagaccc cccacgccat cgccgccatc agcatggcca acagatcccc    480
   tggttctggt cctggttctc ctagatcccc tggttccaga tctacccccc agaacatcac    540
   cgacctctgc gccgagagcc acaacaccca aatctacacc ctcaacgaca agattttcag    600
   ctacaccgag agcctcgccg gcaagaggga gatggccatc atcaccttca agaacggcgc    660
   catcttccag gtcgaggtcc ccggcagcca gcacatcgac agccagaaga aggccatcga    720
   gaggatgaag gacaccctca ggatcgccta cctcaccgag gccaaggtcg agaagctctg    780
   cgtctggaac aacaagaccc cccacgccat cgccgccatc agcatggcca acagatcccc    840
   tggttctggt cctggttctc ctagatctga acatgatgaa ttgtga                   886
<210> 110
   <211> 901
   <212> DNA
   <213> Artificial
<220>
   <223> an artificially synthesized DNA construct
<400> 110
   tatttaactc agtattcaga aacaacaaaa gttcttctct acataaaatt ttcctatttt     60
   agtgatcagt gaaggaaatc aagaaaaata aatggggaga atgtcaatac ccatgatggg    120
   ttttgtggtg ttatgtctat gggcagtggt agcagaagga tccacccccc agaacatcac    180
   cgacctctgc gccgagagcc acaacaccca aatctacacc ctcaacgaca agattttcag    240
   ctacaccgag agcctcgccg gcaagaggga gatggccatc atcaccttca agaacggcgc    300
   catcttccag gtcgaggtcc ccggcagcca gcacatcgac agccagaaga aggccatcga    360
   gaggatgaag gacaccctca ggatcgccta cctcaccgag gccaaggtcg agaagctctg    420
   cgtctggaac aacaagaccc cccacgccat cgccgccatc agcatggcca acagatcccc    480
   tggttctggt cctggttctc ctagatcccc tggttctggt cctggttctc ctagatctac    540
   cccccagaac atcaccgacc tctgcgccga gagccacaac acccaaatct acaccctcaa    600
   cgacaagatt ttcagctaca ccgagagcct cgccggcaag agggagatgg ccatcatcac    660
   cttcaagaac ggcgccatct tccaggtcga ggtccccggc agccagcaca tcgacagcca    720
   gaagaaggcc atcgagagga tgaaggacac cctcaggatc gcctacctca ccgaggccaa    780
   ggtcgagaag ctctgcgtct ggaacaacaa gaccccccac gccatcgccg ccatcagcat    840
   ggccaacaga tcccctggtt ctggtcctgg ttctcctaga tctgaacatg atgaattgtg    900
   a                                                                    901
<210> 111
   <211> 1009
   <212> DNA
   <213> Artificial
<220>
   <223> an artificially synthesized DNA construct
<400> 111
   tatttaactc agtattcaga aacaacaaaa gttcttctct acataaaatt ttcctatttt     60
   agtgatcagt gaaggaaatc aagaaaaata aatggcctcc atctcctcct cagccatcgc    120
   caccgtcaac cggaccacct ccacccaagc tagcttggca gctccattca ccggcctcaa    180
   gtctaacgta gctttcccag ttaccaagaa ggctaacaat gacttttcat ccctacccag    240
   caacggtgga agagtacaat gcatgaaggt gtggccacca attgggttga agaagtacga    300
   gactctttca tacctaaccc cccagaacat caccgacctc tgcgccgaga gccacaacac    360
   ccaaatctac accctcaacg acaagatttt cagctacacc gagagcctcg ccggcaagag    420
   ggagatggcc atcatcacct tcaagaacgg cgccatcttc caggtcgagg tccccggcag    480
   ccagcacatc gacagccaga agaaggccat cgagaggatg aaggacaccc tcaggatcgc    540
   ctacctcacc gaggccaagg tcgagaagct ctgcgtctgg aacaacaaga ccccccacgc     600
   catcgccgcc atcagcatgg ccaacagatc ccctggttct ggtcctggtt ctcctagatc    660
   caccccccag aacatcaccg acctctgcgc cgagagccac aacacccaaa tctacaccct    720
   caacgacaag attttcagct acaccgagag cctcgccggc aagagggaga tggccatcat    780
   caccttcaag aacggcgcca tcttccaggt cgaggtcccc ggcagccagc acatcgacag    840
   ccagaagaag gccatcgaga ggatgaagga caccctcagg atcgcctacc tcaccgaggc    900
   caaggtcgag aagctctgcg tctggaacaa caagaccccc cacgccatcg ccgccatcag    960
   catggccaac agatcccctg gttctggtcc tggttctcct agatcctga               1009
<210> 112
   <211> 30
   <212> DNA
   <213> Artificial
<220>
   <223> an artificially sinthesized primer
<400> 112
   tataggatcc cattattttt cttgatttcc 30
<210> 113
   <211> 30
   <212> DNA
   <213> Artificial
<220>
   <223> an artificially sinthesized primer
<400> 113
   aaatgcatgg ccctccatctc ctcctcagcc 30
<210> 114
   <211> 30
   <212> DNA
   <213> Artificial
<220>
   <223> an artificially sinthesized primer
<400> 114
   tttggatcct aggtatgaaa gagtctcgta 30